# EUROPEAN PATENT APPLICATION

(11) **EP 4 461 816 A1**
(43) Date of publication of application: **13.11.2024**
(21) Application number: 24175200.5
(22) Date of filing: 10.05.2024
(51) Int. Cl.: C12Q 1/6806

(54) **BORONIC ACID COMPOSITIONS AND METHODS FOR TETHERING RIBONUCLEIC ACIDS IN BIOLOGICAL SAMPLES**

(30) Priority: 10.05.2023 US 202363465491 P; 23.06.2023 US 202363510069 P
(71) Applicant: 10X Genomics, Inc., Pleasanton, CA 94588 (US)
(72) Inventor: COSTA, Justin, Pleasanton, 94588-3260 (US); CHRISTOPHERSON, Cheyenne, Pleasanton, 94588-3260 (US)
(74) Representative: J A Kemp LLP

(57) **Abstract**

The present disclosure relates in some aspects to methods and boronic acid compositions for immobilizing RNA analytes in biological samples, and more specifically fragmented RNAs. RNA analytes may be tethered covalently or non-covalently to exogenous or endogenous molecules in a biological sample, for example, cross-linked directly to a polymerized three-dimensional matrix.

## Description

### CROSS-REFERENCE TO RELATED APPLICATIONS

This application claims priority to U.S. Provisional Patent Application No. 63/465, 491, filed May 10, 2023, and U.S. Provisional Patent Application No. 63/510,069, filed June 23, 2023, which are incorporated by reference in their entireties for all purposes.

### FIELD

The present disclosure relates in some aspects to methods for analyzing biological samples, and more specifically, methods for analyzing RNA.

### BACKGROUND

Despite improvements in transcriptomic analysis, many nucleic acid analytes present in biological samples can be lost throughout sample preparation using standard protocols and reagents, e.g., permeabilization and de-crosslinking, to enable analysis and imaging, such as by fluorescence in situ hybridization. Although the extent of losses of these uncaptured nucleic acid analytes out of the RNA transcripts for a given sample remain unknown, the un-sequenced RNA analytes nonetheless represent substantial segment of the overall transcriptome that remains omitted by analysis and constitute a significant gap in our knowledge for characterizing disease states of tissue samples.

Existing treatments to capture such analytes typically rely upon hybridization of the target RNA with complementary nucleic acid probes and cross-linking to the probes. However, these methods may still suffer from loss of RNA analytes in sample preparation. In instances where limited sample treatment is performed in order to preserve RNA, such target analytes are preserved intact but may be blocked by proteins, ribosomes, *etc.,* that are also present, and, thus, may require large quantities of probe materials to obtain signal as a result.

Thus, improved methods and techniques for sequencing RNAs, particularly fragmented RNAs, are needed. Provided herein are methods and compositions that address such and other needs.

### SUMMARY

In some embodiments, the methods and kits of the present disclosure allow the anchoring or immobilization of ribonucleic acids that otherwise might be removed or destroyed during sample preparation, particularly fragmented ribonucleic acids, to exogenous or endogenous molecules present in a biological sample prior to sample work-up, thereby enabling downstream analysis of the ribonucleic acid analytes.

In one aspect, provided herein is a method, comprising: (a) contacting a biological sample comprising a ribonucleic acid (RNA) with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3' vicinal diol of the RNA; (b) contacting the biological sample with a matrix forming agent; and (c) forming a three-dimensional polymerized matrix from the matrix-forming agent, thereby embedding the biological sample and immobilizing the RNA in the three-dimensional polymerized matrix.

Boronic acid moieties are typically capable of covalently reacting with at least one 2',3' vicinal diol of RNA. Thus, in any of the embodiments herein, a boronic acid moiety capable of covalently reacting with at least one 2',3' vicinal diol of the RNA may also be referred to simply as a boronic acid moiety.

In any of the embodiments herein, contacting the biological sample with the attachment agent typically forms a covalent bond between the boronic acid moiety and the at least one 2',3' vicinal diol of the RNA.

In any of the embodiments herein, it is to be understood that that the biological sample may be contacted with the matrix forming agent prior to, concurrently with or subsequently to contacting the biological sample with the attachment agent.

In any of the embodiments herein, the attachment moiety can be attached covalently to the matrix-forming agent in the biological sample.

In any of the embodiments herein, the attachment moiety can be or comprise an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety.

In any of the embodiments herein, the attachment moiety can be or comprise a click functional group.

In any of the embodiments herein, the step of contacting the biological sample and attachment agent can further comprise contacting the biological sample with one or more reagents under suitable conditions to facilitate the formation of a covalent bond between the attachment moiety of the attachment agent and an exogenous or endogenous molecule in the biological sample. Typically, when the step of contacting the biological sample and attachment agent further comprises contacting the biological sample with one or more reagents under suitable conditions to facilitate the formation of a covalent bond between the attachment moiety of the attachment agent and an exogenous or endogenous molecule in the biological sample, a covalent bond is formed between the attachment moiety of the attachment agent and the exogenous or endogenous molecule in the biological sample.

The exogenous or endogenous molecule in the biological sample may be present in the biological sample throughout the method of the present disclosure. Alternatively, the method of the present disclosure may comprise introducing the exogenous or endogenous molecule into the biological sample. Thus, the method of the present disclosure is not limited to methods in which the exogenous or endogenous molecule is present in the biological sample prior to contacting the biological sample with the attachment agent. When the method of the present disclosure comprises introducing the exogenous or endogenous molecule into the biological sample, the exogenous or endogenous molecule is typically introduced into the biological sample at any stage prior to immobilizing the ribonucleic acid in the biological sample.

In any of the embodiments herein, the attachment moiety can be attached noncovalently to the matrix-forming agent in the biological sample.

In any of the embodiments herein, the attachment moiety can be or comprise biotin.

In any of the embodiments herein, the attachment moiety can be or comprise a nonaromatic compound.

In any of the embodiments herein, the attachment agent can be or comprise a compound of formula (I) 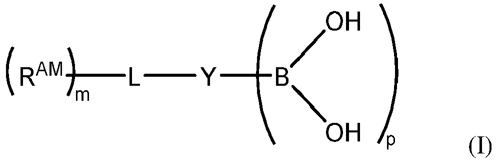 or a salt thereof, wherein
- each R^{AM}: is independently an attachment moiety;
- L: is a bond or linker moiety;
- Y: is a bond, -CH₂CH₂- or -O-;
- m: is an integer from 1 to 4; and
- p: is an integer from 1 to 4.

In any of the embodiments herein, the attachment agent can be multifunctional and can comprise at least two boronic moieties or at least two attachment moieties.

In any of the embodiments herein, the attachment agent can comprise at least two boronic acid moieties.

In any of the embodiments herein, the attachment agent can comprise at least two attachment moieties.

In any of the embodiments herein, the attachment agent can be bifunctional.

In any of the embodiments herein, the attachment agent can be or comprise a compound of formula (I-a) 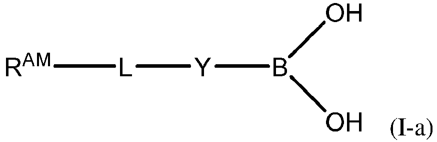 or a salt thereof, wherein
- R^{AM}: is the attachment moiety;
- L: is a bond or linker moiety; and
- Y: is a bond, -CH₂CH₂- or -O-.

In some embodiments, L is a linker moiety. Examples of linker moieties include but are not limited to a nucleic acid (e.g., a nucleic acid between about 4 and about 200 nucleotides in length, optionally wherein the nucleic acid is between about 4 and about 20 nucleotides in length), a small molecule linker, or a peptide linker. In some embodiments, the linker moiety comprises deoxyribonucleic acid (DNA) and/or locked nucleic acid (LNA). In some embodiments, Z is S (e.g., the linker moiety comprises a disulfide bond. In some embodiments, L is a linker moiety according to formula 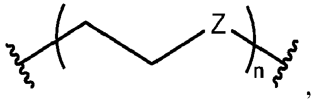 wherein each Z is independently CH₂, O, S, or NH; and n is an integer from 0 to 50. In some embodiments, L is a linker moiety according to formula 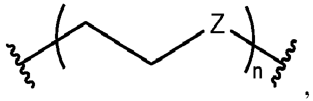 wherein Z is CH₂, O, S, or NH; and n is an integer from 0 to 50.

In any of the embodiments herein, the L can be or comprise an unbranched or branched C₁-C₁₅₀ alkylene optionally interrupted by 1 to 50 heteroatoms independently selected from the group consisting of O, S and NH.

In any of the embodiments herein, L can be or comprise the group 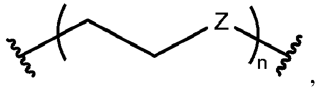 wherein Z is CH₂, O, S, or NH; and n is an integer from 0 to 50.

In any of the embodiments herein, each R^{AM} independently can be or comprise an acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety.

In any of the embodiments herein, the attachment agent can be or comprise a compound of formula (II-a) 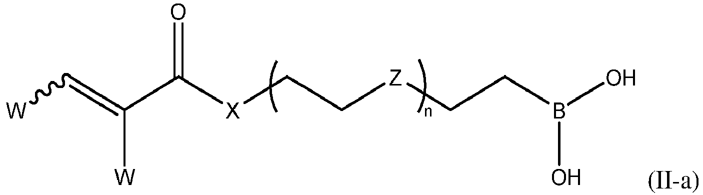 or a salt thereof, wherein
W is H or CH₃;
X is NH or O;
Z is NH, O, or S; and
n is an integer from 0 to 50.

In any of the embodiments herein, the attachment agent can be or comprise a compound of formula 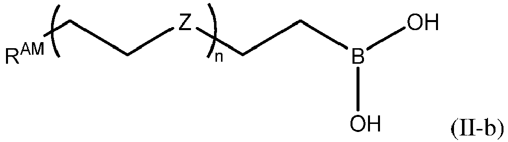 or a salt thereof, wherein
R^{AM} is a biotinyl moiety, dextrin moiety, a click moiety, a thiol moiety, norbornenyl moiety, furanyl moiety, alkyl ester moiety, or maleimidyl moiety;
Z is CH₂, O, S, or NH; and
n is an integer from 0 to 50.

In any of the embodiments herein, n can be 6.

In one aspect, provided herein is a method, comprising: (a) contacting a biological sample comprising a ribonucleic acid (RNA) with a matrix-forming agent, wherein: (i) the RNA comprises a 2'3' vicinal diol, and (ii) the matrix-forming agent comprises a boronic acid moiety capable of covalently reacting with at least one 2',3' vicinal diol of the RNA, and wherein the biological sample and the matrix-forming agent are contacted under conditions suitable to form a covalent bond between the boronic acid moiety and the 2',3;-vicinal diol of the RNA; and (b) forming a three-dimensional polymerized matrix from the matrix-forming agent, thereby embedding the biological sample and immobilizing the RNA in the three-dimensional polymerized matrix.

In any of the embodiments herein, contacting the biological sample with the matrix-forming agent typically forms a covalent bond between the boronic acid moiety of the matrix-forming agent and the at least one 2',3' vicinal diol of the RNA.

In some embodiments wherein the matrix-forming agent comprises a boronic acid moiety, the matrix-forming agent can be prepared by reacting a precursor matrix-forming agent with an attachment agent comprising a boronic acid moiety and an attachment moiety capable of attaching covalently to the precursor matrix-forming agent. It will be understood that a precursor matrix-forming material, as applied herein, refers to a matrix-forming agent that does not comprise a boronic acid moiety. Exemplary precursor matrix-forming materials include but are not limited to acrylamide, bis-acrylamide, polyacrylamide and derivatives thereof, poly(ethylene glycol) and derivatives thereof (e.g. PEG-acrylate (PEG-DA), PEG-RGD), gelatinmethacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly (hydroxy ethyl acrylate), and poly (hydroxy ethyl methacrylate), collagen, hyaluronic acid, chitosan, dextran, agarose, gelatin, alginate, protein polymers, methylcellulose, and the like, and combinations thereof. Reaction of a precursor matrix-forming material with an attachment agent, as described herein, produces a matrix-forming agent comprising a boronic acid. In some embodiments, the attachment agent used to prepare the matrix-forming agent comprising a boronic acid is a compound of formula (I), (I-a), (II-a), or (II-b).

Following formation of the matrix-forming agent comprising a boronic acid moiety, a three dimensional polymerized matrix (e.g., hydrogel) can be formed. In some embodiments, the three-dimensional matrix can be formed by polymerizing the matrix-forming agent comprising a boronic acid moiety with other precursor matrix forming agents. In some embodiments, radical generating compounds (e.g., TMED/ammonium persulfate) can be added to initiate polymerization of the matrix-forming agents. In some embodiments, following polymerization of the matrix-forming agents to generate a three-dimensional polymerized matrix (e.g., hydrogel), the three-dimensional polymerized matrix can be mixed with the biological sample comprising RNA molecules (e.g., fragmented RNA molecules) 2'3' vicinal diol groups. Reaction between the 2'-3' vicinal diol groups with the boronic acid moiety bound to the three-dimensional polymerized matrix polymerized immobilizes the RNA in the three-dimensional polymerized matrix.

In alternative embodiments, the biological sample can be added to the matrix-forming agent comprising a boronic acid moiety prior to polymerization of the matrix-forming agents to form a three-dimensional polymerized matrix. In such embodiments, the 2'3' vicinal diol groups of the RNA molecules in the biological sample react with the boronic acid moiety of the matrix-forming agent comprising the boronic acid moiety. In some embodiments, mixing of the biological sample with matrix-forming agents comprising the boronic acid moiety can be conducted without adding radical generating components, hence reducing polymerization of the matrix-forming material. Following reaction of the boronic acid moiety of the matrix-forming agent with the 2'3' vicinal diol groups of the RNA molecules, radical generating components (e.g., TMED and ammonium persulfate) can be added to initiate polymerization of the matrix-forming materials.

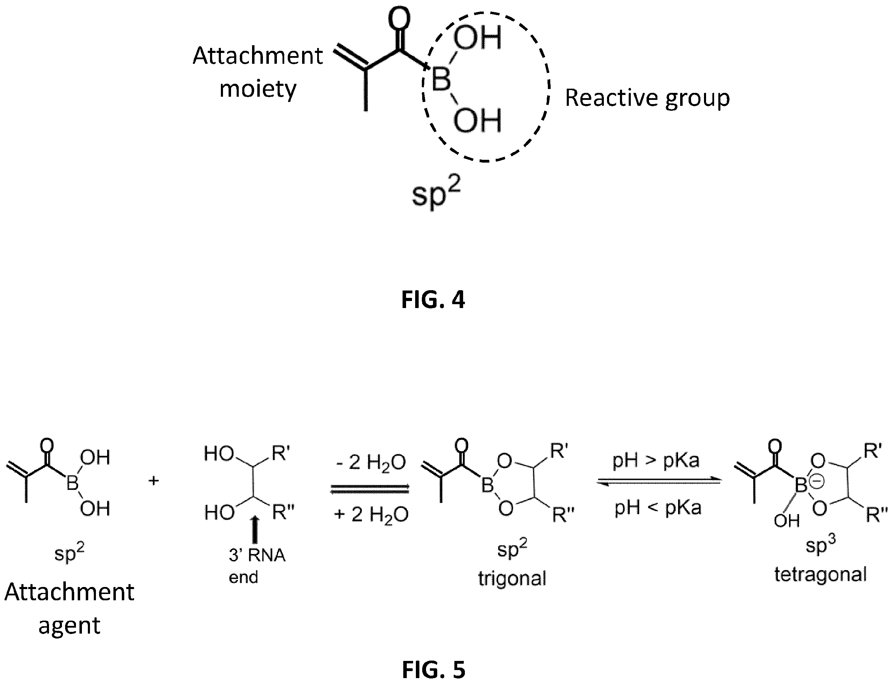

In any of the embodiments herein, the conditions suitable to form a covalent bond between the boronic acid moiety and the 2',3'-vicinal diol of the RNA can be basic conditions. In some embodiments, the basic conditions are mildly basic conditions. For instance, in some embodiments, the reaction between the attachment agent and the RNA in the biological sample can be conducted at a pH of from about 7.2 to a pH of about 9.5. In some embodiments, the reaction between the attachment agent and the RNA in the biological sample can be conducted at a pH of from about 7.5 to a pH of about 8.5. In some embodiments, the reaction between the attachment agent and the RNA in the biological sample can be conducted at a pH of from about 7.5 to a pH of about 8.0. some embodiments, the reaction between the attachment agent and the RNA in the biological sample can be conducted at a pH of from about 8.5 to a pH of about 9.5. In some embodiments, the reaction between the attachment agent and the RNA in the biological sample can be conducted at a pH of about 7.5. In some embodiments, the reaction between the attachment agent and the RNA in the biological sample can be conducted at a pH of about 7.8. In some embodiments, the reaction between the attachment agent and the RNA in the biological sample can be conducted at a pH of about 8.0. In some embodiments, the reaction between the attachment agent and the RNA in the biological sample can be conducted at a pH of about 8.2. In some embodiments, the reaction between the attachment agent and the RNA in the biological sample can be conducted at a pH of about 8.5. In some embodiments, the reaction between the attachment agent and the RNA in the biological sample can be conducted at a pH of about 9. In some embodiments, the reaction between the attachment agent and the RNA in the biological sample can be conducted at a pH of about 9.5 As set forth herein, reactions conducted under basic conditions (e.g., slightly basic conditions) shift the equilibrium towards boronate ester formation, resulting in a greater extent of the RNA molecules bound to the attachment agent (*see* **FIG. 5**).

In other embodiments , the conditions suitable to form a covalent bond between the boronic acid moiety and the 2',3'-vicinal diol of the RNA can be acidic conditions, optionally wherein the acidic conditions are maintained at between pH 5 and pH 7. Under acidic conditions, a sp² trigonal boronate ester is more prevalent at equilibrium than the sp³ tetragonal boronate ester (*see* **FIG. 5**).

A particular advantage of the methods disclosed herein is that formation of the covalent bond between the 2',3'-vicinal diols of the RNA molecules and the boronic acid moiety is reversible. Hence, the position of the equilibrium between the unbound RNA molecules comprising 2',3'-vicinal diols and RNA molecules bound to the attachment agent can be readily controlled. For instance, as set forth above, modifying the pH impacts the equilibrium of the reaction, with the boronate ester being favored at higher (basic) pH and boronic acid and unbound RNA molecules with 2',3'-vicinal diols favored at lower (acidic) pH. In some embodiments, the reaction between the attachment agent and the RNA in the biological sample can be conducted at a basic pH to drive the equilibrium towards boronate ester formation and acidified at a later time period to drive the equilibrium towards boronic acid and unbound RNA molecules with 2',3'-vicinal diols. Accordingly, anchoring of the RNA molecules in the biological sample to an exogenous or endogenous molecule can be effectively revered under acidic conditions.

In some embodiments, liberation of the bound RNA molecules from the three-dimensional polymerized matrix (e.g., hydrogel) can be accomplished by incubating the three-dimensional polymerized matrix in an acidic buffer. For instance, in some embodiments, a hydrogel comprising tethered RNA molecules covalently bonded to the hydrogel via boronate ester moieties can be incubated in 2-(N-morpholino)ethanesulfonic acid (MES) buffer, hence resulting in liberation of the RNA molecules from the hydrogel.

In any of the embodiments herein, the RNA can be or comprise a fragmented RNA.

In any of the embodiments herein, the 2',3'-vicinal diol can be a fragmented 3' end of the RNA.

In any of the embodiments herein, the 2',3'-vicinal diol can be generated from a fragmented RNA having a 2',3'cyclo-phosphate fragmentation at the 3'-terminal end or a 2' hydroxyl and a 3' phosphate fragmentation at the 3'-terminal end.

In any of the embodiments herein, the 2',3'-vicinal diol can be provided by contacting the fragmented RNA with a 3' phosphatase.

In any of the embodiments herein, the 3' phosphatase can be or comprise a T4 polynucleotide kinase.

In any of the embodiments herein, the biological sample can be treated with a degradation agent to induce fragmentation of RNAs, optionally wherein the degradation agent is an RNase or restriction enzyme.

In any of the embodiments herein, the RNA can comprise a 5' cap and the 2',3'-vicinal diol is in the 5' cap.

In any of the embodiments herein, the 5' cap can be or comprise a 7-methylguanosine cap.

In any of the embodiments herein, the method can further comprise clearing the biological sample embedded in the three-dimensional polymerized matrix.

In any of the embodiments herein, the biological sample can be cleared with a detergent, a lipase, and/or a protease.

In any of the embodiments herein, the detergent can comprise a non-ionic surfactant or anionic surfactant, optionally wherein the detergent comprises SDS, tergitol, NP-40, saponin, polyethylene glycolp-(1,1,3,3-tetramethylbutyl)-phenyl ether, or polysorbate 20, or any combinations thereof.

In any of the embodiments herein, the protease can comprise proteinase K, pepsin, or collagenase, trypsin, dispase, thermolysin, or alpha-chymotrypsin, or any combinations thereof, optionally wherein the protease comprises Liberase^{™}.

In any of the embodiments herein, the lipase can comprise a pancreatic, hepatic and/or lysosomal lipase, or any combinations thereof, optionally wherein the lipase comprises sphingomyelinase or esterase, or a combination thereof.

In any of the embodiments herein, the method can further comprise contacting the biological sample with a probe or probe set that binds directly or indirectly to the RNA, optionally wherein the probe or probe set is a detectable probe.

In any of the embodiments herein, the probe or probe set can be or comprise a circular or circularizable probe or probe set, optionally wherein the method can comprise circularizing the circularizable probe or probe set using the RNA or a product thereof as a template, optionally wherein the method can comprise generating an RCA product using the circular or circularizable probe as a template.

In any of the embodiments herein, the method can comprise imaging the biological sample to detect the probe or probe set or the RCA product.

In any of the embodiments herein, the imaging can comprise detecting a signal associated with the probe or probe set or the RCA product, optionally wherein the signal is from a fluorescently labeled probe that directly or indirectly binds to the probe or probe set or the RCA product.. In some embodiments, the method can comprise providing a fluorescently labeled probe that directly or indirectly binds to the probe or probe set or the RCA product, and imaging the biological sample can comprise detecting a signal from the fluorescently labeled probe that directly or indirectly binds to the probe or probe set or the RCA product.

In any of the embodiments herein, the probe or probe set can comprise a barcode region comprising one or more barcode sequences.

In any of the embodiments herein, the method can comprise detecting the barcode sequence or a complement thereof in the probe or probe set or in a product of the probe or probe set.

In one aspect, provided herein is a method of analyzing a fragmented ribonucleic acid (RNA) in a biological sample, the method comprising: (a) contacting the biological sample comprising the fragmented RNA with a 3' phosphatase to provide a fragmented RNA comprising a 2',3'-vicinal diol; (b) contacting the biological sample with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3;-vicinal diol of the RNA; (c) contacting the biological sample with a matrix-forming agent; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a probe or probe set that binds directly or indirectly to the RNA; and (g) detecting the probe or a product thereof at a location in the matrix.

In any of the embodiments herein, the attachment moiety can be attached covalently to the matrix-forming agent in the biological sample.

In any of the embodiments herein, the attachment moiety can be or comprise an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety.

In any of the embodiments herein, the attachment moiety can be or comprise a click functional group.

In any of the embodiments herein, the step of contacting the biological sample and attachment agent can further comprise contacting the biological sample with one or more reagents or under suitable conditions to facilitate the formation of a covalent bond between the attachment moiety of the attachment agent and an exogenous or endogenous molecule in the biological sample.

In any of the embodiments herein, the attachment moiety can be attached noncovalently to the matrix-forming agent in the biological sample.

In any of the embodiments herein, the attachment moiety can be or comprise biotin.

In any of the embodiments herein, the attachment moiety can be or comprise a nonaromatic compound.

In any of the embodiments herein, the attachment agent can be or comprise a compound of formula (I) 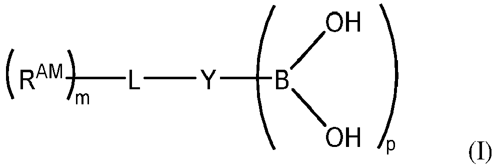 or a salt thereof, wherein
- each R^{AM}: is independently the attachment moiety;
- L: is a bond or linker moiety;
- Y: is a bond, -CH₂CH₂- or -O-;
- m: is an integer from 1 to 4; and
- p: is an integer from 1 to 4.

In any of the embodiments herein, the attachment agent can be multifunctional and can comprise at least two boronic moieties or at least two attachment moieties.

In any of the embodiments herein, the attachment agent can comprise at least two boronic acid moieties.

In any of the embodiments herein, the attachment agent can comprise at least two attachment moieties.

In any of the embodiments herein, the attachment agent can be bifunctional.

In any of the embodiments herein, the attachment agent can be or comprise a compound of formula 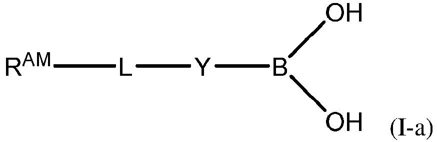 or a salt thereof, wherein
- R^{AM}: is the attachment moiety;
- L: is a bond or linker moiety; and
- Y: is a bond, -CH₂CH₂- or -O-.

In any of the embodiments herein, L can be or comprise an unbranched or branched C₁-C₁₅₀ alkylene optionally interrupted by 1 to 50 heteroatoms independently selected from the group consisting of O, S and NH.

In any of the embodiments herein, L can be or comprise the group 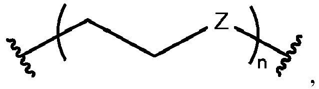 wherein
Z is CH₂, O, S, or NH; and
n is an integer from 0 to 50.

In any of the embodiments herein, each R^{AM} can independently be or comprise an acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety.

In any of the embodiments herein, the attachment agent can be or comprise a compound of formula (II-a) 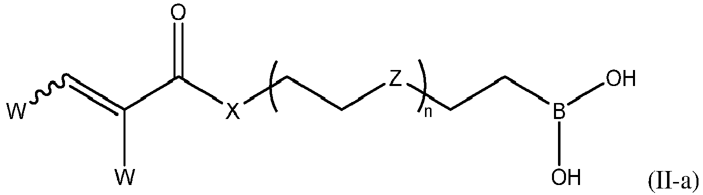 or a salt thereof, wherein
W is H or CH₃;
X is NH or O;
Z is NH, O, or S; and
n is an integer from 0 to 50.

In any of the embodiments herein, the attachment agent can be or comprise a compound of formula (I) 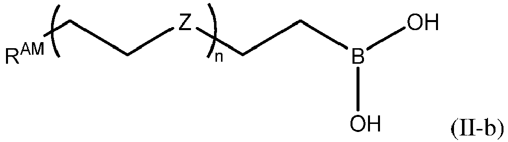 or a salt thereof, wherein
R^{AM} is a biotinyl moiety, dextrin moiety, a click moiety, a thiol moiety, norbornenyl moiety, furanyl moiety, alkyl ester moiety, or maleimidyl moiety;
Z is CH₂, O, S, or NH; and
n is an integer from 0 to 50.

In any of the embodiments herein, n can be 6.

In one aspect, provided herein is a method of analyzing a biological sample comprising a fragmented ribonucleic acid (RNA), the method comprising: (a) contacting the biological sample comprising the fragmented RNA with T4 polynucleotide kinase, wherein the T4 polynucleotide kinase catalyzes formation of a 2',3'-vicinal diol moiety on the fragmented RNA; (b) contacting the biological sample with a deglycosylation agent; (c) contacting the biological sample with a matrix-forming agent, wherein the matrix-forming agent comprises a boronic acid moiety capable of covalently reacting with at least one 2',3' vicinal diol of the RNA, and wherein the biological sample and the matrix-forming agent are contacted under conditions suitable to form a covalent bond between the boronic acid moiety and the 2',3;-vicinal diol of the RNA; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a probe or probe set that binds directly or indirectly to the RNA; and (g) detecting the probe or a product thereof at a location in the matrix. In some embodiments, the fragmented RNA is liberated from the three-dimensional polymerized matrix prior to step (f).

In one aspect, provided herein is a method of analyzing a biological sample comprising a fragmented ribonucleic acid (RNA), the method comprising: (a) contacting the biological sample comprising the fragmented RNA with T4 polynucleotide kinase, wherein the T4 polynucleotide kinase catalyzes formation of a 2',3'-vicinal diol moiety on the fragmented RNA; (b) contacting the biological sample with a deglycosylation agent; (c) contacting the biological sample with a matrix-forming agent, wherein the matrix-forming agent comprises a boronic acid moiety capable of covalently reacting with at least one 2',3' vicinal diol of the RNA, and wherein the biological sample and the matrix-forming agent are contacted under conditions suitable to form a covalent bond between the boronic acid moiety and the 2',3;-vicinal diol of the RNA; (d) forming a three-dimensional polymerized matrix from the matrix-forming agent, thereby embedding the biological sample in the three-dimensional polymerized matrix and anchoring the fragmented RNA to the three-dimensional polymerized matrix; (e) contacting the biological sample with a detergent and a protease; (f) contacting the biological sample with a probe or probe set that binds directly or indirectly to the RNA; and (g) detecting the probe or a product thereof at a location in the matrix. In some instances, the detergent and protease are provided in a buffer of at least pH 8.5. In some instances, the detergent and protease are contacted with the sample for no more than 4 minutes at least 50°C. In some instances, the detergent and protease are provided in a buffer of at least about pH 7.5 (e.g., at least about any of pH 8.0, 8.5, or 9.0). In some instances, the detergent and protease are contacted with the sample for no more than about 10 minutes (e.g., no more than about any of 5 minutes, 4 minutes, 3 minutes, or 2 minutes) at least about 30°C (e.g., at least about any of 35°C, 40°C, 45°C, 50°C, or 55°C). In some embodiments, the fragmented RNA is liberated from the three-dimensional polymerized matrix prior to step (f).

In any of the embodiments herein, the fragmented RNA in step (a) can comprise a 2',3'cyclo-phosphate fragmentation at the 3'-terminal end or a 2' hydroxyl and a 3' phosphate fragmentation at the 3'-terminal end, and wherein the 3' phosphatase can catalyze the formation of the 2',3'-vicinal diol.

In any of the embodiments herein, the 3' phosphatase can be or comprise T4 polynucleotide kinase.

In any of the embodiments herein, the fragmented RNA of step (a) can be generated by treating the biological sample with a degradation agent to induce fragmentation of RNAs, optionally wherein the degradation agent is an RNase or restriction enzyme.

In any of the embodiments herein, the fragmented RNA can further comprise an additional vicinal diol moiety provided by a 5' cap, optionally wherein the 5' cap is a 7-methylguanosine cap.

In any of the embodiments herein, the biological sample can be cleared with a detergent, a lipase, and/or a protease.

In any of the embodiments herein, the detergent can comprise a non-ionic surfactant or anionic surfactant, optionally wherein the detergent comprises SDS, tergitol, NP-40, saponin, polyethylene glycol *p*-(1,1,3,3-tetramethylbutyl)-phenyl ether, or polysorbate 20, or any combinations thereof.

In any of the embodiments herein, the protease can be or comprise proteinase K, pepsin, or collagenase, trypsin, dispase, thermolysin, or alpha-chymotrypsin, or any combinations thereof, optionally wherein the protease comprises Liberase^{™}.

In any of the embodiments herein, the lipase can be or comprise a pancreatic, hepatic and/or lysosomal lipase, or any combinations thereof, optionally wherein the lipase comprises sphingomyelinase or esterase, or a combination thereof.

In any of the embodiments herein, the probe or probe set can be or comprise a circular or circularizable probe or probe set capable of binding the fragmented RNA.

In any of the embodiments herein, the method can further comprise: generating a rolling circle amplification (RCA) product of the circular or circularizable probe or probe set; and detecting a signal associated with a fluorescently labeled probe that directly or indirectly binds to the rolling circle amplification product.

In one aspect, provided herein is a method of analyzing a fragmented ribonucleic acid (RNA) in a biological sample, the method comprising: (a) contacting the biological sample comprising the fragmented RNA with a 3' phosphatase to provide a fragmented RNA comprising a 2',3'-vicinal diol; (b) contacting the biological sample with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3;-vicinal diol of the RNA; (c) contacting the biological sample with a matrix-forming agent; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a circular or circularizable probe or probe set, wherein the circular or circularizable probe binds the RNA; (g) generating a rolling circle amplification (RCA) product of the circular or circularizable probe or probe set; and (h) detecting a signal associated with a fluorescently labeled probe that directly or indirectly binds to the rolling circle amplification product.

In any of the embodiments herein, the attachment moiety can be attached covalently to the matrix-forming agent in the biological sample.

In any of the embodiments herein, the attachment moiety can be or comprise an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety.

In any of the embodiments herein, the attachment moiety can be or comprise a click functional group.

In any of the embodiments herein, the step of contacting the biological sample and attachment agent can further comprise contacting the biological sample with one or more reagents or under suitable conditions to facilitate the formation of a covalent bond between the attachment moiety of the attachment agent and an exogenous or endogenous molecule in the biological sample.

In any of the embodiments herein, the attachment moiety can be attached noncovalently to the matrix-forming agent in the biological sample.

In any of the embodiments herein, the attachment moiety can be or comprise biotin.

In any of the embodiments herein, the attachment moiety can be or comprise a nonaromatic compound.

In any of the embodiments herein, the attachment agent can be or comprise a compound of formula (I) 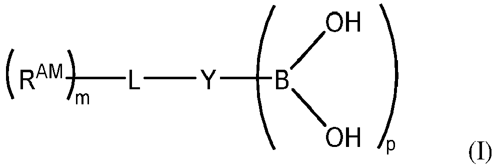 or a salt thereof, wherein
- each R^{AM}: is independently the attachment moiety;
- L: is a bond or linker moiety;
- Y: is a bond, -CH₂CH₂- or -O-;
- m: is an integer from 1 to 4; and
- p: is an integer from 1 to 4.

In any of the embodiments herein, the attachment agent can be multifunctional and can comprise at least two boronic moieties or at least two attachment moieties.

In any of the embodiments herein, the attachment agent can comprise at least two boronic acid moieties.

In any of the embodiments herein, the attachment agent can comprise at least two attachment moieties.

In any of the embodiments herein, the attachment agent can be bifunctional.

In any of the embodiments herein, the attachment agent can be or comprise a compound of formula 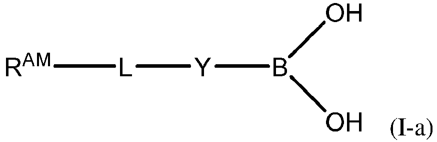 or a salt thereof, wherein
R^{AM} is the attachment moiety;
L is a bond or linker moiety; and
Y is a bond, -CH₂CH₂- or -O-.

In any of the embodiments herein, L can be or comprise an unbranched or branched C₁-C₁₅₀ alkylene optionally interrupted by 1 to 50 heteroatoms independently selected from the group consisting of O, S and NH.

In any of the embodiments herein, L can be or comprise the group 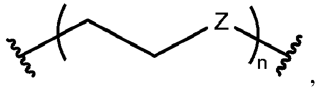 wherein
Z is CH₂, O, S, or NH; and
n is an integer from 0 to 50.

In any of the embodiments herein, each R^{AM} can independently be or comprise an acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety.

In any of the embodiments herein, the attachment agent can be or comprise a compound of formula (II-a) 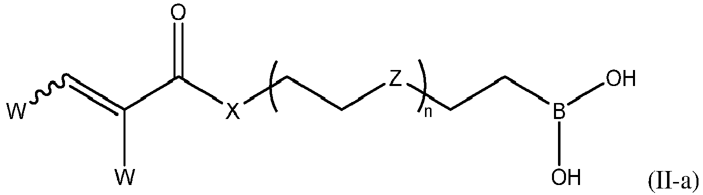 or a salt thereof, wherein
each W is independently H or CH₃;
X is NH or O;
Z is CH₂, O, S, or NH; and
n is an integer from 0 to 50.

In any of the embodiments herein, the attachment agent can be or comprise a compound of formula (I) 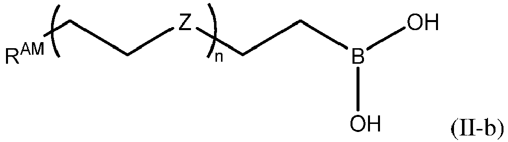 or a salt thereof, wherein
R^{AM} is a biotinyl moiety, dextrin moiety, a click moiety, a thiol moiety, norbornenyl moiety, furanyl moiety, alkyl ester moiety, or maleimidyl moiety;
Z is CH₂, O, S, or NH; and
n is an integer from 0 to 50.

In any of the embodiments herein, n can be 6.

In one aspect, provided herein is a method of analyzing a biological sample comprising a fragmented ribonucleic acid (RNA), the method comprising: (a) contacting the biological sample comprising the fragmented RNA with T4 polynucleotide kinase, wherein the T4 polynucleotide kinase catalyzes formation of a 2',3'-vicinal diol moiety on the fragmented RNA; (b) contacting the biological sample with a deglycosylation agent; (c) contacting the biological sample with a matrix-forming agent, wherein the matrix-forming agent comprises a boronic acid moiety capable of covalently reacting with at least one 2',3' vicinal diol of the RNA; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a circular or circularizable probe or probe set, wherein the circular or circularizable probe binds the RNA; (g) generating a rolling circle amplification (RCA) product of the circular or circularizable probe or probe set; and (h) detecting a signal associated with a fluorescently labeled probe that directly or indirectly binds to the rolling circle amplification product.

In any of the embodiments herein, the biological sample can be cleared with a detergent, a lipase, and/or a protease.

In any of the embodiments herein, the detergent can be or comprise a non-ionic surfactant or anionic surfactant, optionally wherein the detergent comprises SDS, tergitol, NP-40, saponin, polyethylene glycol *p*-(1,1,3,3-tetramethylbutyl)-phenyl ether, or polysorbate 20, or any combinations thereof.

In any of the embodiments herein, the protease can be or comprise proteinase K, pepsin, or collagenase, trypsin, dispase, thermolysin, or alpha-chymotrypsin, or any combinations thereof, optionally wherein the protease comprises Liberase^{™}.

In any of the embodiments herein, the lipase can be or comprise a pancreatic, hepatic and/or lysosomal lipase, or any combinations thereof, optionally wherein the lipase comprises sphingomyelinase or esterase, or a combination thereof.

In some embodiments, the biological sample can be cleared by contacting the biological sample with a detergent and a protease. In some instances, the detergent and protease are provided in a buffer of at least about pH 7.5 (e.g., at least about any of pH 8.0, 8.5, or 9.0). In some instances, the detergent and protease are contacted with the sample for no more than about 10 minutes (e.g., no more than about any of 5 minutes, 4 minutes, 3 minutes, or 2 minutes) at least about 30°C (e.g., at least about any of 35°C, 40°C, 45°C, 50°C, or 55°C).

In any of the embodiments herein, the biological sample can be or comprise cells or cellular components.

In any of the embodiments herein, the biological sample can be or comprise a tissue sample.

In any of the embodiments herein, the tissue sample can be or comprise a tissue slice between about 1 µm and about 50 µm in thickness.

In any of the embodiments herein, the biological sample can be fixed.

In any of the embodiments herein, the biological sample can be a formalin-fixed, paraffin-embedded (FFPE) sample, a fresh tissue sample, or a frozen tissue sample.

In any of the embodiments herein, the method can further comprise staining, permeabilizing, cross-linking, expanding, and/or de-cross-linking the biological sample embedded in the three-dimensional polymerized matrix.

In any of the embodiments herein, the biological sample and fragmented RNA can be treated with a ribonuclease inhibitor.

In any of the embodiments herein, the step of contacting the biological sample with the 3' phosphatase can be performed in the absence of ammonium ions, phosphate ions, or metal chelators.

In any of the embodiments herein, the step of contacting the biological sample with the 3' phosphatase can be performed in the absence of sodium chloride or potassium chloride buffer having a concentration of greater than 50 mM.

In any of the embodiments herein, the matrix-forming agent can be or comprise polyacrylamide, cellulose, alginate, polyamide, cross-linked agarose, cross-linked dextran or cross-linked polyethylene glycol.

In any of the embodiments herein, the three-dimensional polymerized matrix can be formed by subjecting the matrix-forming agent to polymerization (or to further polymerization, in the case of matrix-forming agents that are polymers such as polyethylene glycol)..

In any of the embodiments herein, the polymerization can be initiated by adding a polymerization-inducing catalyst, UV light or functional cross-linkers.

In any of the embodiments herein, the fragmented RNA is can be fragmented mRNA.

In any of the embodiments herein, the method further comprises treating the biological sample with a detergent and a protease after forming the three-dimensional polymerized matrix. In some embodiments, the detergent comprises SDS and the protease comprises proteinase K. In some embodiments, the detergent and the protease are provided in a buffer of at least pH 8.0. In some embodiments, the biological sample is treated with the detergent and the protease at least 45°C for no more than 4 minutes. In some embodiments, the biological sample is treated with the detergent and the protease at about 50°C for about 3 minutes. In some embodiments, the biological sample is treated with 1% SDS and 200 µg/mL proteinase K provided in a PBS buffer of at least pH 8.5 at about 50°C for about 3 minutes.

In one aspect, provided herein is a kit for analyzing fragmented ribonucleic acid (RNA) in a biological sample, comprising: (a) an attachment agent comprising a boronic acid moiety capable of covalently reacting with at least one 2',3' vicinal diol of the RNA and an attachment moiety capable of attaching covalently or noncovalently to an exogenous or endogenous molecule in the biological sample; (b) a matrix-forming agent; (c) a polymerization-inducing catalyst or functional cross linker; and (d) instructions for use.

In one aspect, provided herein is a kit for analyzing fragmented ribonucleic acid (RNA) in a biological sample, comprising: (a) a matrix-forming agent, wherein the matrix forming agent comprises a boronic acid moiety capable of covalently reacting with at least one 2',3' vicinal diol of the RNA; (b) a polymerization-inducing catalyst or functional cross linker; and (c) instructions for use.

In any of the embodiments herein, the kit can further comprise a deglycosylation agent. In any of the embodiments herein, the kit can further comprise a 3' phosphatase. In any of the embodiments herein, the 3' phosphatase can be T4 polynucleotide kinase. In any of the embodiments herein, the kit can further comprise a clearing agent. In any of the embodiments herein, the clearing agent can be or comprise a detergent, a lipase, a protease, and/or a deglycosylase. In some embodiments, the clearing agent comprises a detergent and a protease. In some embodiments, the clearing agent further comprises a buffer of at least about pH 7.5 (e.g., at least about any of pH 8.0, 8.5, or 9.0).

### BRIEF DESCRIPTION OF THE DRAWINGS

The drawings illustrate certain features and advantages of this disclosure. These embodiments are not intended to limit the scope of the appended claims in any manner.
**FIGS. 1A-1C** depict generalized schemes for immobilizing fragmented RNAs to an endogenous or exogenous molecule in a biological sample.
**FIGS. 2A and 2B** illustrate two exemplary mechanisms for RNA fragmentation in biological samples.
**FIG. 3** depicts an exemplary 5' methylguanosine cap containing two vicinal diols.
**FIG. 4** depicts an example of an attachment agent comprising a boronic acid moiety and an attachment moiety.
**FIG. 5** depicts a generalized scheme for immobilizing RNAs in a biological sample using an exemplary attachment agent comprising a boronic acid moiety.
**FIG. 6** is an example workflow of analysis of a biological sample (*e.g.,* a cell or tissue sample) using an opto-fluidic instrument, according to various embodiments.

### DETAILED DESCRIPTION

All publications, comprising patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. Overview

Provided herein are methods and kits for analyzing ribonucleic acids (RNA), particularly highly degraded or fragmented RNA, in a biological sample (e.g., tissue). The methods and kits provided herein can be applied to various applications such as in situ methods. In situ analysis of the identity and spatial localization of RNA requires positional stability of the RNA. However, the preparation of many samples for in situ analysis undergo several harsh processing steps (e.g., formalin-fixed, paraffin-embedded (FFPE) tissues). These steps include baking and deparaffinization, decrosslinking, and permeabilization. The vast majority of RNA (e.g., mRNA) can be lost during and after the decrosslinking step.

Hydrogel-based approaches to emerging in situ technologies offer certain advantages including reduced background autofluorescence and improved diffusional parameters, as well as enhanced tissue adhesion to the hydrogel and temporally spaced orthogonal chemistries that can be leveraged to couple tissue to the hydrogel. However, most hydrogel based approaches have notable limitations. For example, pre-embedding a biological sample with hydrogel monomers prior to digestion of cellular components (e.g., proteins and lipids) and amplification is inherently more time consuming and limited in value for samples containing degraded or fragmented RNA. The methods and kits disclosed herein are intended to overcome many of these shortcomings through use of a brief, enzymatic reaction that converts all 3' ends of fragmented RNA molecules into vicinal diols. At this point, reactive groups can be covalently attached to the 3' end of the converted RNA fragments for incorporation into the hydrogel. Because the 3' end of the majority of fragmented RNA can be converted into vicinal diols and unfragmented RNA naturally contains vicinal diols at the 3' end, the present disclosure provides a substrate that can be broadly leveraged for hydrogel-based in situ applications. The methods involving enzymatic and chemical reactions provided herein can enable pre-embedding approaches to samples that include degraded RNA, preserving their spatial orientation and opening access to thousands of RNA fragments/cell that have been unable to study until now. In some aspects, the tethering methods provided herein are agnostic to the size of RNA fragments (any 3' end can be tethered) and can be used with compromised biological samples. For example, RNA fragments of about 10 to 100 nucleotides, about 10 to 80 nucleotides, about 10 to 60 nucleotides, about 10 to 40 nucleotides, about 10 to 20 nucleotides, about 20 to 100 nucleotides, about 20 to 80 nucleotides, about 20 to 60 nucleotides, about 20 to 40 nucleotides, or about 20 to 30 nucleotides are analyzed using the methods provided herein. In some embodiments, RNA fragments of about 20 to 50 nucleotides are analyzed using the methods provided herein. In some embodiments, 3' end tethering of fragmented RNA can increase sensitivity and reduce the need for using multiple probes per analyte.

### II. Methods for Analyzing RNA

Provided herein are methods for analyzing RNA in a biological sample (e.g., a tissue sample). The methods provided herein include a series of enzymatic and non-enzymatic reactions that can be utilized to immobilize or tether any fragmented RNAs to an endogenous molecule in the biological sample or an exogenous molecule delivered to the biological sample, such as a matrix-forming agent.

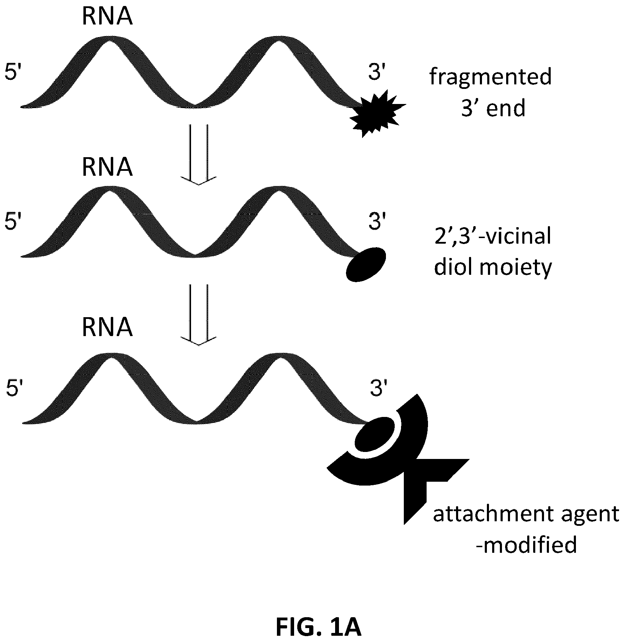

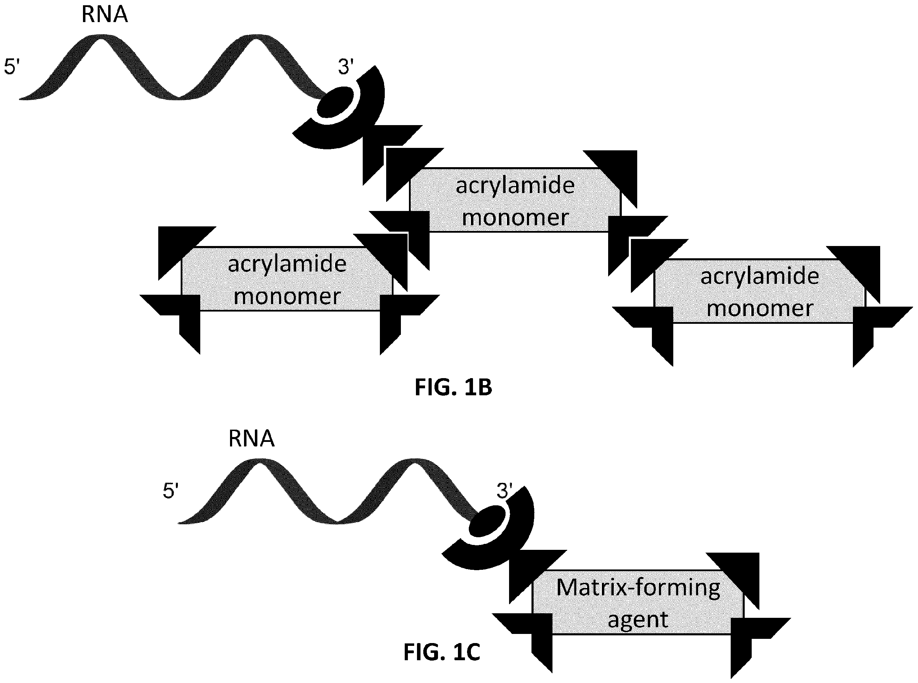

More specifically, the methods and kits as provided herein enable conversion of fragmented 3' ends of RNA to functional groups capable of being immobilized in the biological sample. With reference to **FIGS. 1A-1C****,** examples of modes of immobilizing RNAs in a biological sample according to the methods of the present disclosure are shown. In FIG. 1A, a RNA having a fragmented terminal 3' end is converted to 2',3' vicinal diol. The 2',3' vicinal diol can bind covalently to an attachment agent having a boronic acid moiety, which is capable of reacting with the vicinal diol to form a boronate ester. The attachment agent can then bind covalently or non-covalently to a molecule in the biological sample. As shown in **FIG. 1B****,** the attachment agent may be selected for bonding to an exogenous molecule, such as a matrix-forming agent (e.g., acrylamide monomers). Examples of matrix-forming agents include but are not limited to acrylamide, bisacrylamide, cellulose, alginate, polyamide, agarose, dextran, and polyethylene glycol.

In one aspect, provided herein is a method, comprising: (a) contacting a biological sample comprising a ribonucleic acid (RNA) with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3' vicinal diol of the RNA; (b) contacting the biological sample with a matrix forming agent; and (c) forming a three-dimensional polymerized matrix from the matrix-forming agent, thereby embedding the biological sample and immobilizing the RNA in the three-dimensional polymerized matrix. In some embodiments, after embedding the biological sample in the three-dimensional polymerized matrix and anchoring the fragmented RNA to the three-dimensional polymerized matrix and prior to contacting the biological sample with a probe or probe set that binds directly or indirectly to the RNA, the method comprises treating the biological sample embedded in the three-dimensional polymerized matrix to remove proteins from the tethered RNA (e.g., to remove ribosomes from the RNA). In some embodiments, treating the biological sample embedded in the three-dimensional polymerized matrix to remove proteins from the tethered RNA comprises using a detergent (e.g., SDS) and a protease (e.g., proteinase K).

In some embodiments, the step of contacting the biological sample and the and attachment agent further comprises contacting the biological sample with one or more reagents under suitable conditions to facilitate the formation of a covalent bond between the attachment moiety of the attachment agent and an exogenous or endogenous molecule in the biological sample.

In another aspect, provided herein is a method of analyzing a fragmented ribonucleic acid (RNA) in a biological sample, the method comprising: (a) contacting the biological sample comprising the fragmented RNA with a 3' phosphatase to provide a fragmented RNA comprising a 2',3'-vicinal diol; (b) contacting the biological sample with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3;-vicinal diol of the RNA; (c) contacting the biological sample with a matrix-forming agent; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a probe or probe set that binds directly or indirectly to the RNA; and (g) detecting the probe or a product thereof at a location in the matrix.

In yet another aspect, provided herein is a method of analyzing a fragmented ribonucleic acid (RNA) in a biological sample, the method comprising: (a) contacting the biological sample comprising the fragmented RNA with a 3' phosphatase to provide a fragmented RNA comprising a 2',3'-vicinal diol; (b) contacting the biological sample with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3;-vicinal diol of the RNA; (c) contacting the biological sample with a matrix-forming agent; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a circular or circularizable probe or probe set, wherein the circular or circularizable probe binds the RNA; (g) generating a rolling circle amplification (RCA) product of the circular or circularizable probe or probe set; and (h) detecting a signal associated with a fluorescently labeled probe that directly or indirectly binds to the rolling circle amplification product.

The methods and kits as provided herein may also enable immobilization of fragmented RNA analytes directly to three-dimensional polymerized matrices. For example, with reference to **FIG. 1C****,** a matrix-forming agent may be modified with a boronic acid moiety to bind directly and covalently with a 2',3'-vicinal diol on a RNA. The matrix-forming agent may subsequently be subjected to polymerization conditions in order to provide a three-dimensional polymerized matrix. Following polymerization, the RNA is directly tethered to the three-dimensional polymerized matrix by way of the covalent bonds of the boronate ester.

In one aspect, provided herein is a method, comprising: (a) contacting a biological sample comprising a ribonucleic acid (RNA) with a matrix-forming agent, wherein: (i) the RNA comprises a 2'3' vicinal diol, and (ii) the matrix-forming agent comprises a boronic acid moiety capable of covalently reacting with at least one 2',3' vicinal diol of the RNA, and wherein the biological sample and the matrix-forming agent are contacted under conditions suitable to form a covalent bond between the boronic acid moiety and the 2',3;-vicinal diol of the RNA; and (b) forming a three-dimensional polymerized matrix from the matrix-forming agent, thereby embedding the biological sample and immobilizing the RNA in the three-dimensional polymerized matrix. In accordance with this method, the matrix-forming agent is covalently bonded to the attachment agent comprising a boronic acid moiety prior to contacting the biological sample comprising the RNA molecules comprising 2',3'-vicinal diols (*see* **FIG. 1C**). Accordingly, the attachment moiety is tethered to the matrix-forming agent prior to contacting with the biological sample.

Tethering of the boronic acid moiety to the matrix-forming agent can be carried out under suitable conditions to ensure covalent bond formation between the attachment moiety of the attachment agent and a complementary functional group on the matrix-forming agent. In some embodiments, the matrix-forming agent is covalently bonded to the attachment moiety at a basic pH (e.g., at a pH of from about 8 to about 10). In one such embodiments, the reaction is carried out in Thermopol buffer (20mM Tris-HCL, 10mM (NH4)SO4, 10mM KCL, 2mM MgSO4, 0.1% TritonX-100, pH8.8). In some embodiments, following the formation of the bond between the matrix-forming agent and the attachment moiety, the matrix-forming agent can be polymerized to form a three-dimensional polymerized matrix (e.g., hydrogel). The three-dimensional polymerized matrix (e.g., hydrogel) comprising the covalently bound attachment agent can next be contacted with the biological sample. The boronic acid moiety of the bound attachment agent reacts with the 2'3'-diols of the RNA molecules in the biological sample, thereby immobilizing the RNA in the three-dimensional matrix. In other embodiments, contacting of the biological sample and polymerization of the matrix-forming agent are carried out simultaneously. In still other embodiments, contacting of the biological sample and the matrix-forming agent with bound attachment agent is carried out under conditions that will not initially result in polymerization of the matrix-forming agent. For instance, in embodiments where acrylamide is the matrix-forming agent, the matrix-forming agent with bound attachment can be contacted with the biological sample in the absence of tetramethylethylenediamine (TMED) and ammonium persulfate in order to abrogate polymerization of the acrylamide. Following reaction between the boronic acid moieties bound to the acrylamide and the RNA molecules in the biological sample, radical generating compounds (e.g., TMED/ammonium persulfate) can be added to initiate polymerization of the acrylamide. This method has some advantages over the aforementioned methods, as it does not require the RNA molecules to diffuse through the three-dimensional matrix to find a pendant boronic acid moiety. The method allows the various components of the gel to arrange themselves around the RNA molecules in the biological sample prior to gel formation. Additionally, the method can be carried out in a single solution.

In another aspect, provided herein is a method of analyzing a biological sample comprising a fragmented ribonucleic acid (RNA), the method comprising: (a) contacting the biological sample comprising the fragmented RNA with T4 polynucleotide kinase, wherein the T4 polynucleotide kinase catalyzes formation of a 2',3'-vicinal diol moiety on the fragmented RNA; (b) contacting the biological sample with a deglycosylation agent; (c) contacting the biological sample with a matrix-forming agent, wherein the matrix-forming agent comprises a boronic acid moiety capable of covalently reacting with at least one 2',3' vicinal diol of the RNA, and wherein the biological sample and the matrix-forming agent are contacted under conditions suitable to form a covalent bond between the boronic acid moiety and the 2',3;-vicinal diol of the RNA; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a probe or probe set that binds directly or indirectly to the RNA; and (g) detecting the probe or a product thereof at a location in the matrix.

In still yet another aspect, provided herein is a method of analyzing a biological sample comprising a fragmented ribonucleic acid (RNA), the method comprising: (a) contacting the biological sample comprising the fragmented RNA with T4 polynucleotide kinase, wherein the T4 polynucleotide kinase catalyzes formation of a 2',3'-vicinal diol moiety on the fragmented RNA; (b) contacting the biological sample with a deglycosylation agent; (c) contacting the biological sample with a matrix-forming agent, wherein the matrix-forming agent comprises a boronic acid moiety capable of covalently reacting with at least one 2',3' vicinal diol of the RNA; (d) forming a three-dimensional polymerized matrix from the matrix-forming agent, thereby embedding the biological sample in the three-dimensional polymerized matrix and anchoring the fragmented RNA to the three-dimensional polymerized matrix; (e) clearing the biological sample; (f) contacting the biological sample with a circular or circularizable probe or probe set, wherein the circular or circularizable probe binds the RNA; (g) generating a rolling circle amplification (RCA) product of the circular or circularizable probe or probe set; and (h) detecting a signal associated with a fluorescently labeled probe that directly or indirectly binds to the rolling circle amplification product.

### A. Ribonucleic Acid(s)

In some embodiments, the RNA analyzed by a method provided herein comprises various types of coding and non-coding RNA. Examples of the different types of RNA analytes include messenger RNA (mRNA), including a nascent RNA, a pre-mRNA, a primary-transcript RNA, and a processed RNA, such as a capped mRNA (e.g., with a 5' 7-methyl guanosine cap), a polyadenylated mRNA (poly-A tail at the 3' end), and a spliced mRNA in which one or more introns have been removed. Also included in the analytes disclosed herein are non-capped mRNA, a non-polyadenylated mRNA, and a non-spliced mRNA. The RNA analyte can be a transcript of another nucleic acid molecule (e.g., DNA or RNA such as viral RNA) present in a tissue sample. Examples of a non-coding RNAs (ncRNA) that is not translated into a protein include transfer RNAs (tRNAs) and ribosomal RNAs (rRNAs), as well as small non-coding RNAs such as microRNA (miRNA), small interfering RNA (siRNA), Piwiinteracting RNA (piRNA), small nucleolar RNA (snoRNA), small nuclear RNA (snRNA), extracellular RNA (exRNA), small Cajal body-specific RNAs (scaRNAs), and the long ncRNAs such as Xist and HOTAIR. The RNA can be small (e.g., less than 200 nucleic acid bases in length) or large (e.g., RNA greater than 200 nucleic acid bases in length). Examples of small RNAs include 5.8S ribosomal RNA (rRNA), 5S rRNA, tRNA, miRNA, siRNA, snoRNAs, piRNA, tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA).

As detailed above, the methods of the present disclosure are especially suitable for immobilization of fragmented RNA. In some embodiments, any one of the RNA analytes disclosed herein can be fragmented. In some embodiments, the RNA analyzed by the methods provided herein comprises a fragmented RNA. In some embodiments, the RNA is a fragmented RNA. In some embodiments, the fragmented RNA is fragmented mRNA. In some embodiments, the fragmented RNA comprises a 2',3'-vicinal diol. In some embodiments, the 2',3'-vicinal diol is at the fragmented 3' end of the RNA. In some embodiments, the 2',3'-vicinal diol is a fragmented 3' end of the RNA.

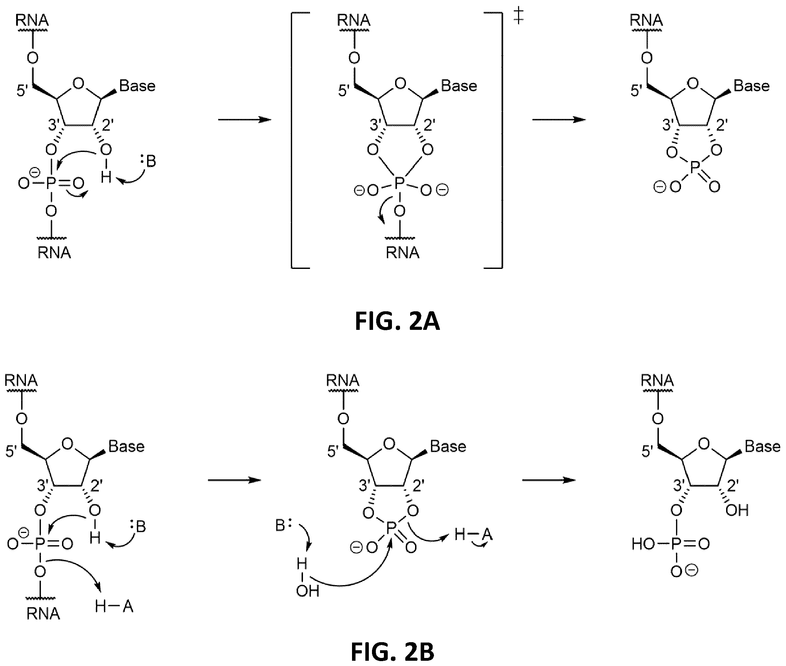

In **FIGS. 2A** and **2B****,** exemplary mechanisms for RNA fragmentation in situ are shown. With reference to **FIG. 2A****,** RNA fragmentation can result in formation of 2',3'-cyclo-phosphate on the fragmented 3' end of the RNA. With reference to **FIG. 2B**, a separate fragmentation mechanism results in an RNA fragment having a 2' hydroxyl group and 3' phosphate group on the terminal 3' end of the RNA. The two mechanisms illustrated in **FIGS. 2A** and **2B** are two of the most common fragmentation patterns observed for RNA in biological samples. As provided herein, the methods of the present disclosure utilize enzymatic reactions, driven by 3'-phosphatases, to convert these most commonly occurring RNA fragments into a 2',3'-vicinal diol.

In some embodiments, the 2',3'-vicinal diol is generated from a fragmented RNA having a 2',3'cyclo-phosphate fragmentation at the 3'-terminal end or a 2' hydroxyl and a 3' phosphate fragmentation at the 3'-terminal end. In some embodiments, the 2',3'-vicinal diol is generated from a fragmented RNA having a 2',3'cyclo-phosphate fragmentation at the 3'-terminal end. In some embodiments, the 2',3'-vicinal diol is generated from a fragmented RNA having a 2' hydroxyl and a 3' phosphate fragmentation at the 3'-terminal end. In some embodiments, the 2',3'-vicinal diol is provided by contacting a fragmented RNA with a 3' phosphatase. For instance, the methods provided herein may comprise contacting the fragmented ribonucleic acid with a 3' phosphatase to provide the 2',3'-vicinal diol. In some embodiments, the methods provided herein comprise contacting a fragmented RNA, wherein the fragmented RNA comprises a 2',3'cyclo-phosphate fragmentation at the 3'-terminal end or a 2' hydroxyl and a 3' phosphate fragmentation at the 3'-terminal end, with a 3'-phosphatase to generate a fragmented RNA comprising a 2',3'-vicinal diol.

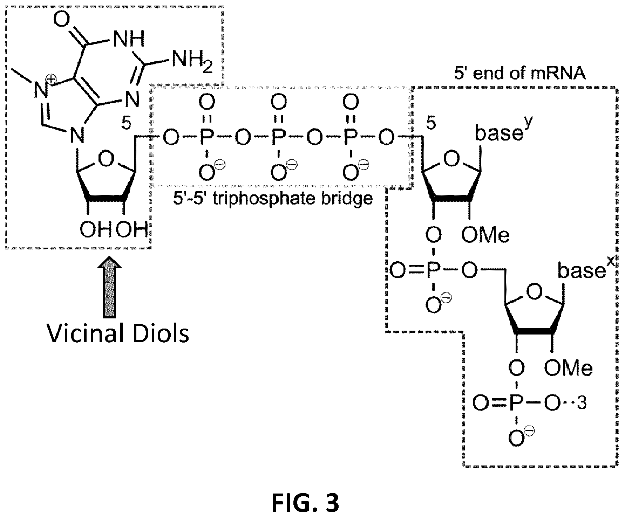

The methods of the present disclosure may also be extended to direct immobilization of any RNA analytes, whether fragmented or not, wherein the RNA analyte possesses a vicinal diol moiety, for example, on a 5' cap or an intact 3' terminal end. An exemplary 5' cap, 7-methylguanosine, is shown in **FIG. 3****.** In some embodiments, the RNA comprising a 2',3'-vicinal diol comprises a 5' cap and the 2',3'-vicinal diol is in the 5' cap. In some embodiments, the 5' cap is a 7-methylguanosine cap.

In some embodiments, the fragmented RNA is generated by treating the biological sample containing the RNA with a degradation agent to induce fragmentation of RNAs. The term "degradation agent" refers to a chemical reagent or enzyme that induces fragmentation of RNA. Examples of degradation agents include a nuclease, like a ribonuclease, or a restriction enzyme. In certain embodiments, the degradation agent is an RNase or restriction enzyme.

Ribonucleases, or RNases, are nucleases that catalyze degradation of RNA. Exemplary RNases may include but are not limited to RNase A, RNase B, RNase C, RNase E, RNase H, RNase HI, RNase HII, RNase II, RNase III, RNase F1, RNase L, RNase M, RNase Ms, RNase N, RNase P, RNase PhyM, RNase R, RNase Sa, RNase St, RNase T1, RNase T2, RNase U2, RNase IV, RNase V, RNase E, RNase E, polynucleotide phosphorylase (PNPase), RNase PH, RNase, RNase BN, RNase D, RNase T, RNase 1, oligoribonuclease, exoribonuclease I, and exoribonuclease II.

A restriction enzyme, restriction endonuclease, REase, ENase or restrictase is an enzyme that cleaves RNA or DNA into fragments at or near specific recognition sites within molecules known as restriction sites. Exemplary restriction enzymes may include but are not limited to EcoRI, EcoRII, BamHI, HindIII, TaqI, NotI, HinFI, Sau3AI, PvuII, SmaI, HaeIII, HgaI, AluI, EcoRV, EcoP15I, KpnI, Pme1, PstI, SacI, SalI, ScaI, SpeI, SphI, StuI, and XbaI.

It should be recognized, however, that in some embodiments, wherein the intentional fragmentation has already been achieved via contact with a degradation agent and/or wherein no further fragmentation is desired, such as for FFPE tissue samples, the methods of the present disclosure may encompass the use of ribonuclease inhibitors. In some embodiments, the RNA is not fragmented RNA. In some embodiments, the sample is a fresh frozen sample such as a fresh frozen tissue sample. In some embodiments, the RNAs in the sample are not fragmented, or the fragmentation or degradation of RNAs in the sample is prevented or reduced by treating the sample with an ribonuclease inhibitor.

The methods as described herein may further comprise contacting or treating the biological sample and/or fragmented RNA with RNase inhibitors to prevent any undesired fragmentation. In some embodiments, the method further comprises the biological sample and fragmented RNA are treated with a ribonuclease inhibitor. If the biological sample is contacted with a degradation agent to induce fragmentation, the method may further comprise treating the biological sample and fragmented RNA with a ribonuclease inhibitor after being contacted with the degradation agent. In some embodiments, the biological sample and fragmented RNA are treated with a ribonuclease inhibitor after the biological sample has been contacted with a degradation agent. In some embodiments, the biological sample is treated with one or more RNase inhibitors. In some embodiments, the one or more RNase inhibitors are the same. In some embodiments, the one or more RNase inhibitors are different.

Exemplary ribonuclease inhibitors may include but are not limited to an anti-RNase antibodies, recombinant enzymes, or non-enzymatic inhibitors.

In some embodiments, the anti-RNase antibody is capable of binding to RNase A, RNase B, RNase C, RNase E, RNase H, RNase HI, RNase HII, RNase II, RNase III, RNase F1, RNase L, RNase M, RNase Ms, RNase N, RNase P, RNase PhyM, RNase R, RNase Sa, RNase St, RNase T1, RNase T2, RNase U2, RNase IV, RNase V, RNase E, RNase E, polynucleotide phosphorylase (PNPase), RNase PH, RNase, RNase BN, RNase D, RNase T, RNase 1, oligoribonuclease, exoribonuclease I, or exoribonuclease II. In some embodiments, the anti-RNase antibody comprises the Roche Protector RNase inhibitor (Millipore Sigma, Cat. # C756R82), In some embodiments, the anti-RNase antibody is Roche Protector RNase inhibitor.

In some embodiments, the recombinant enzyme is capable of degrading RNase A, RNase B, RNase C, RNase E, RNase H, RNase HI, RNase HII, RNase II, RNase III, RNase F1, RNase L, RNase M, RNase Ms, RNase N, RNase P, RNase PhyM, RNase R, RNase Sa, RNase St, RNase T1, RNase T2, RNase U2, RNase IV, RNase V, RNase E, RNase E, polynucleotide phosphorylase (PNPase), RNase PH, RNase, RNase BN, RNase D, RNase T, RNase 1, oligoribonuclease, exoribonuclease I, or exoribonuclease II. In some embodiments, the recombinant enzyme comprises Invitrogen's SUPERase•In^{™} RNase Inhibitor, RNaseOUT^{™} Recombinant Ribonuclease Inhibitor, RNAsecure^{™} RNase Inactivation Reagent, or Ambion^{™} RNase Inhibitor.

In some embodiments, the non-enzymatic ribonuclease inhibitor comprises a ribonucleotide-derived RNase inhibitor or a nonnucleotide RNase inhibitor. In some embodiments, the RNase inhibitor is a vanadium salt. In some embodiments, the ribonucleotide-derived RNase inhibitor comprises one or more ribonucleotide vanadyl complexes (RVC), dinucleotide derivatives of adenosine 5'-pyrophosphate, such as 5'-diphosphoadenosine 3'-phosphate (ppA-3'-p) or 5'-diphosphoadenosine 2'-phosphate (ppA-2'-p), diadenosine derivatives, 3'-N-alkylamino-3'-deoxy-ara-uridines, or ribonucleotide zinc complexes, such as 3'-N-oxyurea-3'-deoxythymidine 5'-phosphate zinc complex. In some embodiments, the nonnucleotide RNase inhibitor comprises 8-amino-5-(4'-hydroxybiphenyl-4-ylazo)naphthalene-2-sulfonate or a catechin, such as epi-gallocatechin-3-gallate.

### B. Phosphatase(s)

As detailed above, the methods of the present disclosure employ enzymatic conversion of the two most commonly occurring RNA fragmentation patterns into a 2',3'-vicinal diol moiety, which may subsequently modified to enable downstream chemistries and immobilization in the biological sample. Exemplary fragmented RNAs comprising a 2',3'cyclo-phosphate fragmentation at the 3'-terminal end or a 2' hydroxyl and a 3' phosphate fragmentation at the 3'-terminal end may be treated with a 3' phosphatase, such as T4 polynucleotide kinase, thereby resulting in the formation of a 2'3'-vicinal diol moiety at the 3'-terminal ribose ring. In some embodiments, the 2',3'-vicinal diol at the 3' end of the fragmented RNA is generated by a 3' phosphatase.

In some embodiments, the 2',3'-vicinal diol is provided by contacting the fragmented RNA with a 3' phosphatase. For instance, the methods provided herein may comprise contacting the fragmented ribonucleic acid with a 3' phosphatase to provide the 2',3'-vicinal diol. In some embodiments, the fragmented RNA has a 2',3'cyclo-phosphate fragmentation at the 3'-terminal end or a 2' hydroxyl and a 3' phosphate fragmentation at the 3'-terminal end, wherein the 3' phosphatase catalyzes the formation of the 2',3'-vicinal diol.

It should be recognized that the 3' phosphatase as referred to herein may also be referred to as a 3' nucleotidase or analogous enzyme in the class of hydrolases capable of catalyzing the reaction to convert a ribonucleotide comprising a 3' terminal phosphate group to the corresponding ribonucleotide and cleaved phosphate. An exemplary listing of 3' phosphatases is provided in the SIB Swiss Institute of Bioinformatics Expasy enzyme nomenclature database (entry: EC 3.1.3.6). In some embodiments, the 3' phosphatase comprises a T4 polynucleotide kinase. In some embodiments, the 3' phosphatase is T4 polynucleotide kinase.

In some embodiments wherein the method comprises contacting a fragmented RNA comprising a 2',3'cyclo-phosphate fragmentation at the 3'-terminal end or a 2' hydroxyl and a 3' phosphate fragmentation at the 3'-terminal end with a 3' phosphatase or analogous enzyme, the step of contacting the biological sample or RNA with the 3' phosphatase is performed under conditions suitable for the 3' phosphatase activity to occur. For example, in some embodiments, the presence of certain buffers and/or reagents utilized for this step may be limited to those that are compatible with 3' phosphatase activity or are limited with respect to the concentrations at which they are included to permit 3' phosphatase activity to occur. In some embodiments, the step of contacting the biological sample with the 3' phosphatase is not performed in the presence of ammonium ions, phosphate ions, or metal chelators. In some embodiments, the step of contacting the biological sample with the 3' phosphatase is not performed in the presence of sodium chloride or potassium chloride buffer having a concentration of greater than 50 mM.

### C. Attachment Agent(s)

As detailed herein, the methods of the present disclosure encompass the preparation of terminal vicinal diols on fragmented RNAs for immobilization of the RNAs in the biological sample. In some embodiments, the terminal vicinal diols are present on nonfragmented RNA in the biological sample. The methods provided herein achieve immobilization of the RNAs through the use of an attachment agent that mediates the interaction between the RNA and the biological sample. In some embodiments, the methods as provided herein comprise contacting the biological sample with one or more attachment agents. In some embodiments, wherein the method comprises contacting the biological sample with two or more attachment agents, the attachment agents may be the same or different.

In some embodiments, the attachment agent is a bi- or multifunctional molecule comprising at least two different functional groups. In some embodiments, the attachment agent is a bifunctional molecule. In some embodiments wherein the attachment agent is a bifunctional molecule, the attachment agent comprises at least two functional groups, the first of which is a boronic acid moiety capable of covalently bonding to the vicinal diols of RNA and the second of which is capable of covalently or non-covalently bonding to an exogenous or endogenous molecule present in the biological sample. As described herein, the functional groups comprise a boronic acid moiety and an attachment moiety. The boronic acid moiety can be any boronic acid moiety or derivative thereof that reacts with and covalently bonds to a vicinal diol. The attachment moiety can be any functional group that interacts with an exogenous or endogenous molecule in a biological sample and, in some embodiments, can comprise an attachment moiety that reacts with and covalently bonds to a complementary reactive group on the exogenous or endogenous molecule.

In other embodiments, the attachment agent is a multifunctional molecule. In some embodiments wherein the attachment agent is a multifunctional molecule, the attachment agent comprises at least three (e.g., at least any of 3, 4, 5, 6, 7, or 8) functional groups, wherein at least two functional groups are boronic acid moieties capable of covalently bonding to the RNA and at least one functional group is capable of covalently or non-covalently bonding to an exogenous or endogenous molecule present in the biological sample, or wherein at least two functional groups are capable of covalently or non-covalently bonding to an exogenous or endogenous molecule present in the biological sample and at least one functional group is a boronic acid moiety capable of covalently bonding to the RNA. In some embodiments wherein the attachment agent is a multifunctional molecule, the attachment agent may comprise two or more functional groups of the same category, e.g., boronic acid moieties capable of covalently bonding to the RNA or capable of covalently or non-covalently bonding to an exogenous or endogenous molecule present in the biological sample. For example, in some embodiments wherein the attachment agent is a multifunctional molecule, the attachment agent may comprise at least two boronic acid moieties capable of covalently bonding to the RNA. In other embodiments wherein the attachment agent is a multifunctional molecule, the attachment agent may comprise at least two functional groups capable of covalently or non-covalently bonding to an exogenous or endogenous molecule present in the biological sample. In certain embodiments wherein the attachment agent is a multifunctional molecule, the attachment agent may comprise at least two boronic acid moieties capable of covalently bonding to the RNA and at least two functional groups capable of covalently or non-covalently bonding to an exogenous or endogenous molecule present in the biological sample. In some embodiments, the attachment agent comprises at least two boronic acid moieties or at least two attachment moieties. In some embodiments, the attachment agent comprises at least two boronic acid moieties. In some embodiments, the attachment agent comprises at least two attachment moieties. As described herein, the two categories of functional groups of the multifunctional molecule comprise at least a boronic acid moiety and an attachment moiety.

It should be recognized that reference to the attachment agent, whether bi- or multifunctional, as defined herein refers to the attachment agent prior to binding with the RNA and the exogenous or endogenous molecule present in the biological sample, unless otherwise noted.

Whether bifunctional or multifunctional, the attachment agents as provided herein each possess at least one boronic acid moiety capable of covalently bonding to the RNA and at least one functional group capable of covalently or non-covalently bonding to an exogenous or endogenous molecule present in the biological sample. It should be recognized that the multifunctional attachment agent will have at least one additional functional group capable of covalently bonding to the RNA (*e.g.,* a boronic acid moiety) or capable of covalently or noncovalently bonding to an exogenous or endogenous molecule present in the biological sample.

### (i) Boronic Acid Moiety

As provided herein, the functional group(s) capable of covalently bonding to the RNA may is boronic acid moiety that reacts with and covalently bonds to the vicinal diols of a RNA. In some embodiments, the attachment agent comprises a boronic acid moiety capable of reacting with at least one 2',3'-vicinal diol of the RNA to form a covalent bond and an attachment moiety capable of attaching covalently or non-covalently to an exogenous or endogenous molecule in the biological sample. Methods of covalently bonding boronic acids to 2',3'-vicinal diols are known in the art. *See* Debiasis et al., Chem Rec. 2022, 22, e202200085 and Lelièvre-Büttner et al., Chem. Eur. J. 2023, 29, e202300196.

An exemplary attachment agent is shown in **FIG. 4**. In **FIG. 5****,** the formation of a covalent bond between an exemplary attachment agent and a 2',3'-vicinal diol on the 3'-terminal end of a RNA is shown. As shown in **FIG. 5****,** the boronic acid moiety of the attachment agent reacts covalently with the 2',3'-vicinal diol of the RNA to form a boronic ester. The covalent bonds of the boronic ester formed by the boronic acid moiety and the RNA is a reversible linkage, the formation and stability of which may be mediated by environmental pH, with slightly basic conditions favoring formation and stability of the boronic ester over the free boronic acid moiety and free vicinal diols of the RNA. For example, when the pH is higher than the pKₐ of the boronic ester, the formation of a sp³ tetragonal boric ester is favored. However, if the pH is lower than the pKₐ, equilibrium favors the sp² trigonal boronic ester, which may reversibly convert back to the original separate attachment agent with a boronic acid moiety and RNA with 2',3'-vicinal diols.

In some embodiments, the concentration of boronic acid moiety added to the biological sample (e.g., an FFPE tissue sample) is from 0.05 millimolar (mM) to about 10mM. In some embodiments, the concentration of boronic acid moiety added to the biological sample is from 1 mM to about 5 mM. In some embodiments, the concentration of boronic acid moiety added to the biological sample is about 0.5 mM. In some embodiments, the concentration of boronic acid moiety added to the biological sample is about 1 mM. In some embodiments, the concentration of boronic acid moiety added to the biological sample is about 1.5 mM. In some embodiments, the concentration of boronic acid moiety added to the biological sample is about 2 mM. In some embodiments, the concentration of boronic acid moiety added to the biological sample is about 2.5 mM. In some embodiments, the concentration of boronic acid moiety added to the biological sample is about 3 mM. In some embodiments, the concentration of boronic acid moiety added to the biological sample is about 3.5 mM. In some embodiments, the concentration of boronic acid moiety added to the biological sample is about 4 mM. In some embodiments, the concentration of boronic acid moiety added to the biological sample is about 4.5 mM. In some embodiments, the concentration of boronic acid moiety added to the biological sample is about 5 mM. In some embodiments, sugar molecules such as mannose can be removed from the biological sample using commercially available kits.

In some embodiments, the biological sample and attachment agent can be contacted under conditions suitable to form a covalent bond between the boronic acid moiety(ies) of the attachment agent and 2',3'-vicinal diols of RNA(s) in the biological sample. In some embodiments, the step of contacting the biological sample and attachment agent under conditions suitable to form a covalent bond can be performed in a solution or a suspension. In some embodiments, the solution or suspension has a basic pH. In some embodiments, the solution or suspension has a pH of from about 7.2 to a pH pf about 10. In some embodiments, the solution or suspension has a pH of from about 7.5 to a pH of about 8.5. In some embodiments, the solution or suspension has a pH of from about 7.5 to a pH of about 8. In some embodiments, the solution or suspension has a pH of from about 8.5 to a pH of about 9.5. In some embodiments, the solution or suspension has a pH of 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9 or 10.

In other embodiments, the step of contacting the biological sample and attachment agent under conditions suitable to form a covalent bond can be performed in a solution or a suspension under acidic conditions. In some embodiments, the solution or suspension has a pH from about 4.5 to about 7, such as pH 4.5, pH 4.6, pH 4.7, pH 4.8, pH 4.9, pH 5.0, pH 5.1, pH 5.2, pH 5.3, pH 5.4, pH 5.5, pH 5.6, pH 5.7, pH 5.8, pH 5.9, pH 6.0, pH 6.1, pH 6.2, pH 6.3, pH 6.4, pH 6.5, pH 6.6, pH 6.7, pH 6.8, pH 6.9, or pH 7. In some embodiments, the solution or suspension has a pH of about 5 or higher.

As set forth above, acidic conditions favor formation of the sp² trigonal boronate ester over the sp³ tetragonal boronate ester at equilibrium. The sp² trigonal boronate ester may reversibly convert back to the original separate attachment agent with a boronic acid moiety and RNA with 2',3'-vicinal diols. Therefore, basic conditions are favorable for greater anchoring of the RNA molecules biological sample to a three-dimensional polymerized matrix (e.g., hydrogel) and acidic conditions are favored to reverse the anchoring, hence regenerating the RNA with 2',3'-vicinal diols.

In some embodiments, the RNA molecules can be freed from the three-dimensional polymerized matrix (e.g., hydrogel) can be accomplished by incubating the three-dimensional polymerized matrix in an acidic buffer. For instance, in some embodiments, a hydrogel comprising tethered RNA molecules covalently bonded to the hydrogel via boronate ester moieties can be incubated in 2-(N-morpholino)ethanesulfonic acid (MES) buffer, hence resulting in liberation of the RNA molecules from the hydrogel. In some embodiments, the pH of the MES buffer is from about 5.4 to about 5.8 (e.g., pH 5.4, pH 5.5, pH 5.6, pH 5.7 or pH 5.8).

### (ii) Attachment Moiety

In some embodiments, the attachment agent comprises an attachment moiety. In some embodiments, the attachment moiety can be any functional group that interacts with an exogenous or endogenous molecule in a biological sample and, in some embodiments, can comprise or be a group capable of reacting with, covalently binding, or non-covalently binding to a complementary reactive group on the exogenous or endogenous molecule. In some embodiments, the attachment moiety is capable of attaching covalently or non-covalently to an exogenous or endogenous molecule in the biological sample.

In some embodiments, the attachment moiety is capable of attaching covalently to an exogenous or endogenous molecule in the biological sample. In some embodiments, the attachment moiety is capable of attaching covalently to an exogenous molecule in the biological sample. In some embodiments, the attachment moiety is capable of attaching covalently to an endogenous molecule in the biological sample. In some embodiments, the attachment moiety is capable of reacting with an exogenous or endogenous molecule in the biological sample to form a covalent bond. In some embodiments, the attachment moiety is capable of reacting with an exogenous molecule in the biological sample to form a covalent bond. In some embodiments, the attachment moiety is capable of reacting with an endogenous molecule in the biological sample to form a covalent bond.

In some embodiments, the attachment moiety(ies) comprises or is an electrophilic group that is capable of interacting with a reactive nucleophilic group present on exogenous or endogenous molecule in the biological sample to provide a covalent bond between the attachment moiety and exogenous or endogenous molecule in the biological sample. In some embodiments, the nucleophilic groups on the exogenous or endogenous molecule in the biological sample having that capability include but are not limited to, sulfhydryl, hydroxyl and amino functional groups. In some embodiments, the attachment moiety(ies) comprises or is a maleimide, haloacetamide, or NHS ester.

In some embodiments, the attachment moiety(ies) comprises or is a nucleophilic group that is capable of interacting with a reactive electrophilic group present on exogenous or endogenous molecule in the biological sample to provide a covalent bond between the attachment moiety and exogenous or endogenous molecule in the biological sample. In some embodiments, the attachment moiety(ies) comprises or is a thiol, phenol, amino, hydrazide, hydroxylamine, hydrazine, thiosemicarbazone, hydrazine carboxylate, or arylhydrazide. In some embodiments, the attachment moiety(ies) comprises or is a biotinyl moiety, dextrin moiety, a click moiety, a thiol moiety, norbornenyl moiety, furanyl moiety, alkyl ester moiety, or maleimidyl moiety.

In some embodiments, the attachment moiety(ies) comprises or is a click functional group. Suitable click functional groups may include functional groups compatible with a nucleophilic addition reaction, a cyclopropane-tetrazine reaction, a strain-promoted azide-alkyne cycloaddition (SPAAC) reaction, an alkyne hydrothiolation reaction, an alkene hydrothiolation reaction, a strain-promoted alkyne-nitrone cycloaddition (SPANC) reaction, an inverse electron-demand Diels-Alder (IED-DA) reaction, a cyanobenzothiazole condensation reaction, an aldehyde/ketone condensation reaction, and Cu(I)-catalyzed azide-alkyne cycloaddition (CuAAC) reaction. In some embodiments, the attachment moiety(ies) can comprise or be any functional group involved in click reactions. In some embodiments, such click reactions may involve (i) azido and cyclooctynyl; (ii) azido and alkynyl; (iii) tetrazine and dienophile; (iv) thiol and alkynyl; (v) cyano and amino thiol; (vi) nitrone and cyclooctynyl; or (vii) cyclooctynyl and nitrone. It should be recognized that in instances in which the attachment moiety comprises or is a click functional group, the exogenous or endogenous molecule to which it is capable of forming a covalent bond comprises the complementary click functional group to that of the attachment moiety. For example, in some embodiments, the attachment moiety comprises or is an azide moiety and the exogenous or endogenous molecule in the biological sample comprises a complementary alkyne moiety.

In some embodiments, the attachment moiety(ies) comprises or is a group capable of reacting with a matrix-forming agent. In some embodiments, the attachment moiety is capable of attaching covalently to the matrix-forming agent in the biological sample. In some embodiments, the attachment moiety is capable of attaching non-covalently to the matrix-forming agent in the biological sample. As detailed herein, matrix-forming agents may include but are not limited to acrylamide, bis-acrylamide, polyacrylamide and derivatives thereof, poly(ethylene glycol) and derivatives thereof (e.g. PEG-acrylate (PEG-DA), PEG-RGD), gelatinmethacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly (hydroxy ethyl acrylate), and poly (hydroxy ethyl methacrylate), collagen, hyaluronic acid, chitosan, dextran, agarose, gelatin, alginate, protein polymers, methylcellulose, and the like, and combinations thereof. In some embodiments, the attachment moiety(ies) comprises or is an alkenyl, allyl or vinyl moiety, an amide moiety, an alcohol moiety, a polyol moiety, a furan moiety, a maleimide moiety, a norbornene moiety, a thiol moiety (e.g., sulfide half), a phenol moiety (e.g., phenol HRP), a urethane moiety, a cyano moiety, an isocyanate moiety, an isothiocyanate moiety, an ether moiety, a dextran moiety, or an alginate moiety. In some embodiments, the attachment moiety(ies) comprises or is an alkenyl, allyl or vinyl moiety (*e.g.,* -C=C- or HC=C- or HC=C-CH₂-), such as in N-(2-aminoethyl)methacrylamide, 2-aminoethyl methacrylate, 2-aminoethyl (*E*)-but-2-enoate, 2-aminoethyl methacrylate or methylacrylamide, or norbornene. Such alkenyl, allyl or vinyl moieties may be suitable for reaction with matrix-forming agents.

In some embodiments, the attachment moiety(ies) comprises or is an acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety. In certain embodiments, the attachment moiety(ies) comprises or is a biotinyl moiety, dextrin moiety, a click moiety, a thiol moiety, norbornenyl moiety, furanyl moiety, alkyl ester moiety, or maleimidyl moiety.

In some embodiments, the formation of a covalent or non-covalent bond between the attachment moiety and the exogenous or endogenous molecule in the biological sample is mediated by an external reagent or stimulus (e.g., phenol HRP, alkyne/azide click, norbornene/sulfide UV, furan/maleimide Diels Alder, allyl ester/sulfide UV). For example, in some embodiments, the formation of a covalent or non-covalent bond between the attachment moiety and the exogenous or endogenous molecule is initiated or induced by an enzyme, a catalyst, chemical reagents (*e.g*., acid, base, reducing agent, oxidant, etc.), heat, and/or light. In some embodiments, the step of contacting the biological sample and attachment agent further comprises contacting the biological sample with one or more reagents or under suitable conditions to facilitate the formation of a covalent bond between the attachment moiety(ies) of the attachment agent and the exogenous or endogenous molecule in the biological sample. For example, in some embodiments wherein the attachment moiety comprises an alkene or a click functional group, the method may further comprise adding reagents to activate the alkene or click functional group, such as a radical initiator or a copper catalyst, respectively. In other embodiments wherein the attachment moiety(ies) comprises an alkenyl, allyl or vinyl moiety, the method may further comprise exposing the biological sample and attachment agent to (ultraviolet) light or heat to facilitate formation of a covalent bond. In some embodiments wherein the attachment moiety(ies) comprises or is a norbornene moiety, furan moiety, maleimide moiety, or other alkenyl, allyl or vinyl moiety, the method may further comprise exposing the sample to light or heat. In yet other embodiments, the method may further comprise adding an enzyme to facilitate formation of a covalent bond. For example, in some embodiments wherein the attachment moiety(ies) comprises or is a phenol moiety, the method may further comprise adding horseradish peroxidase (HRP).

In some embodiments, the attachment moiety is capable of attaching noncovalently to an exogenous or endogenous molecule in the biological sample. In some embodiments, the attachment moiety is capable of attaching non-covalently to an exogenous molecule in the biological sample. In some embodiments, the attachment moiety is capable of attaching non-covalently to an endogenous molecule in the biological sample. In some embodiments, the attachment moiety comprises or is a group capable of binding to an exogenous or endogenous molecule in the biological sample via non-covalent interaction, such as but not limited to hydrogen bonding, van der Waals interaction, and/or pi-stacking.

In some embodiments, the attachment agent is biotinylated. In some embodiments, the attachment moiety is a biotin moiety or a derivative thereof.

### (iii) Formulae

In one aspect, provided herein is a compound of Formula (I) 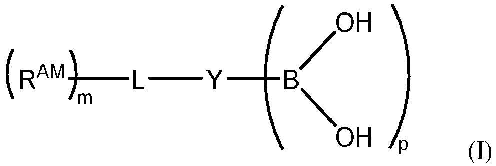 or a salt thereof, wherein
- each R^{AM}: is independently an attachment moiety;
- L: is a bond or linker moiety;
- Y: is a bond, -CH₂CH₂- or -O-;
- m: is an integer from 1 to 4; and
- p: is an integer from 1 to 4.

In some embodiments, the attachment agent as described herein is a compound of Formula (I).

In some embodiments of Formula (I), p is any of 1, 2, 3, or 4. In some embodiments, the attachment agent (e.g., Formula (I)) comprises more than one aldehyde-reactive groups R^{ald} (e.g., p is any of 2, 3, or 4), wherein the R^{ald} groups can be the same group, selected from the embodiments provided herein. In some embodiments, the attachment agent (e.g., Formula (I)) comprises more than one aldehyde-reactive groups R^{ald} (e.g., p is any of 2, 3, or 4), wherein each R^{ald} is independently selected from the embodiments provided herein, provided the more than one aldehyde reactive groups are chemically compatible and have chemically compatible ribonucleic-binding mechanisms or reactions.

In some embodiments, the attachment agent (e.g., Formula (I)) comprises any of 1, 2, 3, or 4 attachment moieties (e.g., R^{AM}). The attachment moieties R^{AM} of Formula (I) are as defined in Section II(C)(ii) above.

In some embodiments, R^{AM} is capable of reacting with an exogenous or endogenous molecule in the biological sample to form a covalent bond. In some embodiments, R^{AM} is capable of reacting with an exogenous molecule in the biological sample to form a covalent bond. In some embodiments, R^{AM} is capable of reacting with an endogenous molecule in the biological sample to form a covalent bond. In some embodiments, R^{AM} is a maleimide, haloacetamide, or NHS ester. In some embodiments, R^{AM} is a thiol, phenol, amino, hydrazide, hydroxylamine, hydrazine, thiosemicarbazone, hydrazine carboxylate, or arylhydrazide. In some embodiments, R^{AM} is a click functional group.

In some embodiments, R^{AM} is capable of reacting with a matrix-forming agent. In some embodiments, R^{AM} is In some embodiments, R^{AM} is an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety. In some embodiments, R^{AM} is a biotin moiety or a derivative thereof. In some embodiments, R^{AM} is an acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety. In certain embodiments, R^{AM} is a biotinyl moiety, dextrin moiety, a click moiety, a thiol moiety, norbornenyl moiety, furanyl moiety, alkyl ester moiety, or maleimidyl moiety.

In some embodiments of Formula (I), m is any of 1, 2, 3, or 4. In some embodiments, the attachment agent (e.g., Formula (I)) comprises more than one attachment moieties R^{AM} (e.g., m is any of 2, 3, or 4), wherein the attachment moieties are the same group, selected from the embodiments provided herein. In some embodiments, the attachment agent (e.g., Formula (I)) comprises more than one attachment moieties R^{AM} (e.g., m is any of 2, 3, or 4), wherein each R^{AM} is independently selected from the embodiments provided herein, provided the more than one reactive groups are chemically compatible and their binding mechanism or reactions to the exogenous or endogenous molecule present in the biological sample are also chemically compatible.

In some embodiments, the compound of formula (I) is a compound of formula (I-a). In some embodiments, the attachment agent is a compound of Formula (I-a), 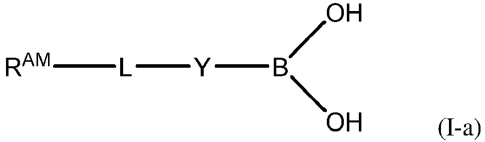 or a salt thereof,
wherein
Y is a bond, -CH₂CH₂- or -O-;
L is a bond or a linker moiety; and
R^{AM} is an attachment moiety.

In some embodiments, the compound of formula (I) is a compound of formula (I-b). In some embodiments, the attachment agent is a compound of Formula (I-b), 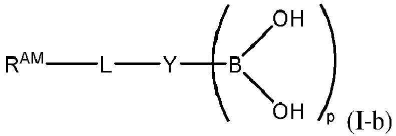 or a salt thereof,
wherein
Y is a bond, -CH₂CH₂- or -O-;
L is a bond or a linker moiety;
R^{AM} is an attachment moiety; and
p is an integer from 1 to 4.

In some embodiments, the compound of formula (I) is a compound of formula (I-c). In some embodiments, the attachment agent is a compound of Formula (I-c), 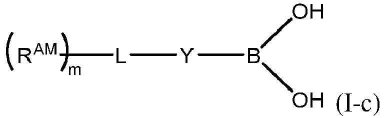 or a salt thereof,
wherein
Y is a bond, -CH₂CH₂- or -O-;
L is a bond or a linker moiety;
each R^{AM} is independently an attachment moiety; and
m is an integer from 1 to 4.

In some embodiments of Formula (I), L is a bond. In some embodiments of Formula (I), L is a linker moiety. In some embodiment, L is an unbranched or branched C₁-C₁₅₀ alkylene, which can be interrupted by 1 to 50 independently selected O, NH, N, S, C₆-C₁₂ arylene, or 5- to 12-membered heteroarylene. In some embodiments, L is an unbranched and uninterrupted C₁-C₁₅₀ alkylene. In some embodiments, L is a branched and uninterrupted C₁-C₁₅₀ alkylene. In some embodiments, L is an unbranched C₁-C₁₅₀ alkylene interrupted by 1 to 50 NH, O, or S. In some embodiments, L is 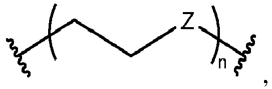 Z is CH₂, O, S; or NH; and n is an integer between 0 and 50. In some embodiments, L is 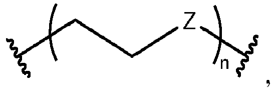 Z is CH₂, O, S; or NH; and n is an integer between 1 and 10. In some embodiment, L is 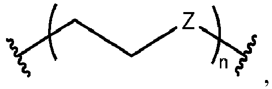 Z is CH₂, O, S; or NH; and n is 6. In some embodiments, L is an unbranched C₁-C₁₅₀ alkylene interrupted by 1 to 50 oxygen. In some embodiments, L comprises a polyethylene glycol portion or is a polyethylene glycol moiety. In some embodiments, L is 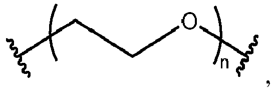 and n is an integer between 0 and 50. In some embodiments, L is 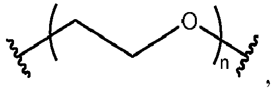 and n is an integer between 1 and 10. In some embodiment, L is 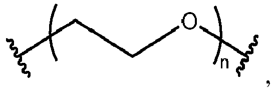 and n is 6. In some embodiments, L comprises an oligoethylene glycol. In some embodiments, L is an oligoethylene glycol moiety. In some embodiments, L is a branched C₁-C₁₅₀ alkylene interrupted by 1 to 50 oxygen. In some embodiments, L is an unbranched C₁-C₁₅₀ alkylene interrupted by 1 to 50 sulfurs. In some embodiments, L comprises or is 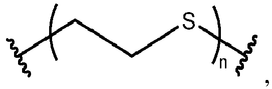 and n is an integer between 0 and 50. In some embodiment, L is 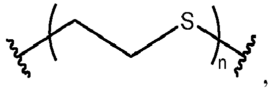 and n is 6. In some embodiments, L is a branched C₁-C₁₅₀ alkylene interrupted by 1 to 50 sulfurs. In some embodiments, L is a branched C₁-C₁₅₀ alkylene interrupted by 1 to 50 -NH-. In some embodiments, L is an unbranched C₁-C₁₅₀ alkylene interrupted by 1 to 50 -NH-. In some embodiments, L comprises or is 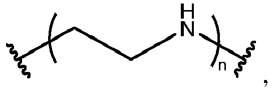 and n is an integer between 0 and 50. In some embodiment, L is 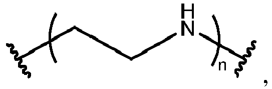 and n is 6. In some embodiments, L is a branched C₁-C₁₅₀ alkylene interrupted by 1 to 50 -NH-, wherein the -NH- is not at a branching point. In some embodiments, L is a branched C₁-C₁₅₀ alkylene interrupted by 1 to 50 -N-, wherein the -N- is at a branching point. In some embodiments, L is an unbranched or branched C₁-C₁₅₀ alkylene interrupted by 1 to 50 independently selected C₆-C₁₂ arylene, for example, any of phenyl or naphthalene. In some embodiments, L is an unbranched or branched C₁-C₁₅₀ alkylene interrupted by 1 to 50 independently selected 5- to 12-membered heteroarylene, for example, any of pyridine, furan, pyrrole, or thiophene.

In some embodiments, Y is -CH₂CH₂-. In some embodiments, Y is O.

In some embodiments, the attachment agent is a compound of Formula (I), 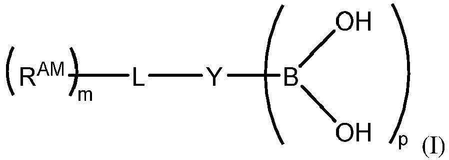 or a salt thereof,
wherein
Y is a bond, -CH₂CH₂- or -O-;
L is a bond or an unbranched or branched C₁-C₁₅₀ alkylene optionally interrupted by 1 to 50 independently selected O, NH, N, S, C₆-C₁₂ arylene, or 5- to 12-membered heteroarylene;
each R^{AM} is independently an attachment moiety capable of attaching covalently or noncovalently to an exogenous or endogenous molecule in the biological sample;
m is an integer from 1 to 4; and
p is an integer from 1 to 4.

In some embodiments, the attachment agent is a molecule of Formula (I), 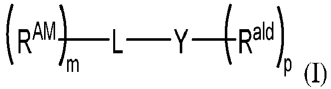 or a salt thereof,
wherein
Y is a bond, -CH₂CH₂- or -O-;
L is an unbranched or branched C₁-C₁₅₀ alkylene optionally interrupted by 1 to 50 heteroatoms independently selected from the group consisting of O, S and NH;
each R^{AM} is independently an attachment moiety that is capable of attaching covalently or non-covalently to an exogenous or endogenous molecule in the biological sample;
m is an integer from 1 to 4; and
p is an integer from 1 to 4.

In some embodiments, the attachment agent is a compound of Formula (I), 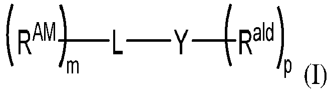 or a salt thereof,
wherein
Y is a bond, -CH₂CH₂- or -O-;
L is a bond, an unbranched and uninterrupted C₁-C₁₅₀ alkylene, 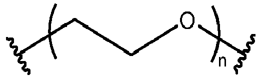 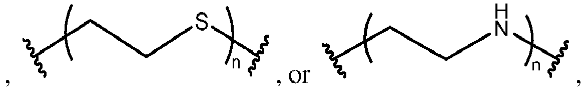 wherein n is an integer from 1 to 50;
each R^{AM} is independently an acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety;
m is an integer from 1 to 4; and
p is an integer from 1 to 4.

In some embodiments, the attachment agent is a bifunctional molecule of Formula (II-a), 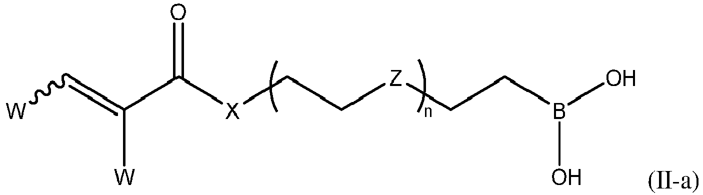 or a salt thereof, wherein
each W is independently H or CH₃;
X is NH or O;
Z is CH₂, O, S; or NH; and
n is an integer from 0 to 50.

In some embodiments, the attachment agent is a bifunctional molecule of Formula (II-b), 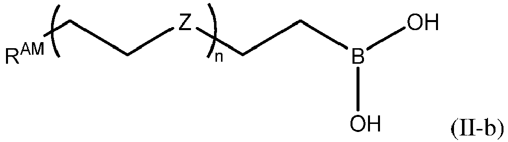 or a salt thereof, wherein
R^{AM} is a biotinyl moiety, dextrin moiety, a click moiety, a thiol moiety, norbornenyl moiety, furanyl moiety, alkyl ester moiety, or maleimidyl moiety;
Z is CH₂, O, S; or NH;; and
n is an integer from 0 to 50.

In some embodiments of any of Formulae (I), (I-a), (II-a), and (II-b), L is 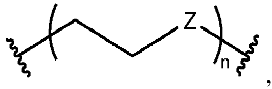 Z is O and n is 6. In some embodiments wherein L is 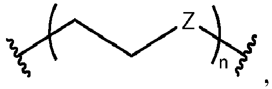 L is a hexaethylene glycol moiety. In some embodiments, the linker L is at least or about 3 nm, at least or about 4 nm, at least or about 5 nm, at least or about 6 nm, at least or about 7 nm, at least or about 8 nm, at least or about 9 nm, or at least or about 10 nm. In some embodiments, the linker L is at least or about 10, at least or about 12, at least or about 14, at least or about 16, at least or about 18, at least or about 20, at least or about 22, at least or about 24, at least or about 26, at least or about 28, or at least or about 30 nucleotides of RNA.

In some embodiments, the attachment moiety is a nonaromatic compound. In other embodiments, the attachment agent can be an aromatic compound. In some embodiments wherein the attachment agent is a nonaromatic compound, the three-dimensional polymerized matrix in which the tissue or biological sample is embedded may have greater translucence (e.g., may be less cloudy or opaque) than a corresponding three-dimensional polymerized matrix in which an aromatic compound was used as an attachment agent. In some embodiments, the attachment moiety is capable of interacting with a matrix-forming agent and is optically inactive.

In some embodiments, a linker between a boronic acid moiety and an acrylamide moiety is hydrophilic or comprises a hydrophilic group. In some embodiments, the linker comprises hexaethylene glycol.

### D. Exogenous or Endogenous Molecule(s)

In some embodiments, the methods of the present disclosure comprise immobilization of a RNA modified with an attachment agent having an attachment moiety capable of forming a covalent or non-covalent bond to an exogenous or endogenous molecule.

In some embodiments, the attachment moiety is capable of attaching covalently to an exogenous or endogenous molecule in the biological sample. In some embodiments, the attachment moiety is capable of attaching covalently to an exogenous molecule in the biological sample. In some embodiments, the attachment moiety is capable of attaching covalently to an endogenous molecule in the biological sample. In some embodiments, the attachment moiety is capable of attaching non-covalently to an exogenous or endogenous molecule in the biological sample. In some embodiments, the attachment moiety is capable of attaching non-covalently to an exogenous molecule in the biological sample. In some embodiments, the attachment moiety is capable of attaching non-covalently to an endogenous molecule in the biological sample.

In some embodiments, the exogenous or endogenous molecule in the biological sample to which the attachment moiety is capable of attaching an exogenous molecule. In some embodiments, the exogenous or endogenous molecule in the biological sample to which the attachment moiety is capable of attaching an endogenous molecule.

It should be recognized that the attachment agent (and attachment moiety thereof) and the exogenous or endogenous molecule are selected with respect to one another, such that the exogenous or endogenous molecule has a functional group capable of ligating or bonding to the attachment moiety of the attachment agent. In some embodiments, the exogenous or endogenous molecule comprises a complementary functional group capable of bonding covalently or non-covalently to the attachment moiety of the attachment agent. For example, in some embodiments wherein the attachment moiety is 2-aminoethyl methacrylate, the exogenous or endogenous molecule is an acrylamide monomer. In some embodiments wherein the attachment moiety is biotin or the attachment agent is biotinylated, the exogenous or endogenous molecule is streptavidin.

In some embodiments, the exogenous molecule is a matrix-forming agent (e.g., any described in Section III), capable of forming a three-dimensional polymerized matrix under suitable reaction conditions. Suitable matrix-forming agents are described herein. For example, in some embodiments, the matrix-forming agent comprises polyacrylamide, cellulose, alginate, polyamide, cross-linked agarose, cross-linked dextran or cross-linked polyethylene glycol. In some embodiments wherein the exogenous molecule is a matrix-forming agent, the method further comprises contacting the biological sample with a matrix-forming agent; and forming a three-dimensional polymerized matrix from the matrix-forming agent, thereby embedding the biological sample in the three-dimensional polymerized matrix and anchoring the RNA to the three-dimensional polymerized matrix. In some embodiments wherein the exogenous molecule is a matrix-forming agent and a three-dimensional polymerized matrix has been formed from the matrix-forming agent, the method may further comprise clearing the biological sample embedded in the three-dimensional polymerized matrix. In some embodiments, biological sample is cleared with a detergent, a lipase, and/or a protease.

As detailed herein, in some embodiments, the step of contacting the biological sample and attachment agent further comprises contacting the biological sample with one or more reagents or under suitable conditions to facilitate the formation of a covalent bond between the attachment moiety of the attachment agent and the exogenous or endogenous molecule in the biological sample. For example, in some embodiments wherein the attachment moiety comprises an alkenyl, the method may further comprise reagents to activate the alkenyl or click functional group, such as a radical initiator for polymerization.

For example, in some embodiments, the three-dimensional polymerized matrix is formed by subjecting the matrix-forming agent to polymerization (or to further polymerization, in the case of matrix-forming agents that are polymers such as polyethylene glycol).. In some embodiments, the polymerization is initiated by adding a polymerization-inducing catalyst, UV light or functional cross-linkers. In some embodiments, the method further comprises staining, permeabilizing, cross-linking, expanding, and/or de-cross-linking the biological sample embedded in the three-dimensional polymerized matrix. In some embodiments, the method further comprises staining, permeabilizing, cross-linking, expanding, and/or de-cross-linking the biological sample embedded in the three-dimensional polymerized matrix after the attachment moiety has been covalently or non-covalently bonded to the matrix-forming agent.

In some embodiments, the exogenous molecule to which the fragmented RNA binds through the attachment moiety is a nucleic acid probe. In some embodiments, the nucleic acid probe and the RNA hybridize to one another, e.g., they can comprise complementary sequences. In some embodiments, the nucleic acid probe and the RNA do not hybridize to one another. In some embodiments, the exogenous molecule to which the fragmented RNA binds through the attachment moiety is not a nucleic acid probe. The binding between the exogenous molecule and the fragmented RNA through the attachment moiety can be covalent (e.g., covalently linked via a linker) or comprise a non-covalent binding pair (e.g., via a streptavidin/biotin binding pair).

In some embodiments, the method further comprises clearing the biological sample embedded in a three-dimensional polymerized matrix. In some instances, the clearing step may comprise the use of a suitable clearing agent, such as an organic solvent, high refractive index aqueous solution, hyperhydrating solution, detergent, digestion enzyme (e.g., protease such as proteinase K) or protein denaturant. In some embodiments, the clearing step comprises contacting the biological sample with a digestion enzyme, optionally wherein the digestion enzyme (e.g., protease) is proteinase K.

In some embodiments, the method may further comprise treating the biological sample embedded in the three-dimensional polymerized matrix to remove proteins from the tethered RNA. In some embodiments, biological sample is treated to remove ribosomes from the RNA. For example, the treatment to remove proteins is performed after RNA is anchored to the three-dimensional polymerized matrix. In some embodiments, the method comprises treating the biological sample embedded in the three-dimensional polymerized matrix with a detergent and a protease. In some cases, the detergent comprises SDS and the protease comprises proteinase K. In some embodiments, the method comprises treating the biological sample embedded in the three-dimensional polymerized matrix with about 50 to about 500 µg/mL proteinase K, about 100 to about 400 µg/mL proteinase K, about 150 to about 300 µg/mL proteinase K, or about 150 to about 250 µg/mL proteinase K. In some embodiments, the method comprises treating the biological sample embedded in the three-dimensional polymerized matrix with about 200 µg/mL proteinase K. In some embodiments, the method comprises treating the biological sample embedded in the three-dimensional polymerized matrix with about 0.5% to about 2% SDS, about 0.5% to about 1% SDS, about 1% to about 2% SDS or about 0.8% to about 1.2% SDS. In some embodiments, the method comprises treating the biological sample embedded in the three-dimensional polymerized matrix with about 1% SDS. In some embodiments, the method may comprise treating the biological sample embedded in the three-dimensional polymerized matrix with a detergent and a protease in a reaction buffer at about pH 8 to pH 9. In some embodiments, the method may comprise treating the biological sample embedded in the three-dimensional polymerized matrix with a detergent and a protease in a reaction buffer at about pH 8.5.

In some embodiments, the method may comprise treating the biological sample embedded in the three-dimensional polymerized matrix with a detergent and a protease in a reaction buffer at about 45°C to 60°C, at about 45°C to 55°C, at about 45°C to 50°C, at about 48°C to 55°C, at about 48°C to 52°C, or at about 50°C to 52°C. In some embodiments, the method may comprise treating the biological sample embedded in the three-dimensional polymerized matrix with a detergent and a protease in a reaction buffer at about 50°C for at least 2 minutes, at least 3 minutes, or at least 4 minutes. In some embodiments, the method may comprise treating the biological sample embedded in the three-dimensional polymerized matrix with a detergent and a protease in a reaction buffer at about 50°C for no more than 5 minutes, no more than 4 minutes, or no more than 3 minutes. In some instances, the biological sample embedded in the three-dimensional polymerized matrix is treated with 1% SDS and 200µg/ml PK in PBS pH 8.5 at 50°C for about 3 minutes.

In some embodiments, treating the biological sample embedded in the three-dimensional polymerized matrix with a detergent and a protease as described herein improves the detection sensitivity for the analyte (e.g., tethered RNA). In some embodiments, treating the biological sample embedded in the three-dimensional polymerized matrix with a detergent and a protease as described herein does not significantly alter the nuclei morphology. In some embodiments, treating the biological sample embedded in the three-dimensional polymerized matrix with a detergent and a protease as described herein does not lead to significant degradation of the histones. For example, at least about 50%, such as at least about any of 60%, 70%, 80%, 90%, 95%, or 99% histones remain undegraded after treating the biological sample embedded in the three-dimensional polymerized matrix with a detergent and a protease as described herein.

### III. Samples, Analytes, and Detection

### A. Samples and Matrix Embedding

A sample disclosed herein can be or be derived from any biological sample. Methods and compositions disclosed herein may be used for analyzing a biological sample, which may be obtained from a subject using any of a variety of techniques including, but not limited to, biopsy, surgery, and laser capture microscopy (LCM), and generally includes cells and/or other biological material from the subject. In addition to the subjects described above, a biological sample can be obtained from a prokaryote such as a bacterium, an archaea, a virus, or a viroid. A biological sample can also be obtained from non-mammalian organisms (e.g., a plant, an insect, an arachnid, a nematode, a fungus, or an amphibian). A biological sample can also be obtained from a eukaryote, such as a tissue sample, a patient derived organoid (PDO) or patient derived xenograft (PDX). A biological sample from an organism may comprise one or more other organisms or components therefrom. For example, a mammalian tissue section may comprise a prion, a viroid, a virus, a bacterium, a fungus, or components from other organisms, in addition to mammalian cells and non-cellular tissue components. Subjects from which biological samples can be obtained can be healthy or asymptomatic individuals, individuals that have or are suspected of having a disease (e.g., a patient with a disease such as cancer) or a predisposition to a disease, and/or individuals in need of therapy or suspected of needing therapy.

The biological sample can include any number of macromolecules, for example, cellular macromolecules and organelles (e.g., mitochondria and nuclei). The biological sample can include nucleic acids (such as DNA or RNA), proteins/polypeptides, carbohydrates, and/or lipids. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample may comprise cells which are deposited on a surface, cells from a portion of a cell block or cells from a portion of a cell pellet.

Biological samples can be derived from a homogeneous culture or population of the subjects or organisms mentioned herein or alternatively from a collection of several different organisms.

Biological samples can include one or more diseased cells. A diseased cell can have altered metabolic properties, gene expression, protein expression, and/or morphologic features. Examples of diseases include inflammatory disorders, metabolic disorders, nervous system disorders, and cancer. Cancer cells can be derived from solid tumors, hematological malignancies, cell lines, or obtained as circulating tumor cells. Biological samples can also include fetal cells and immune cells.

Biological samples can include analytes (e.g., protein, RNA, and/or DNA) embedded in a 3D matrix. In some embodiments, amplicons (e.g., rolling circle amplification products) derived from or associated with analytes (e.g., protein, RNA, and/or DNA) can be embedded in a 3D matrix. In some embodiments, a 3D matrix may comprise a network of natural molecules and/or synthetic molecules that are chemically and/or enzymatically linked, e.g., by crosslinking. In some embodiments, a 3D matrix may comprise a synthetic polymer. In some embodiments, a 3D matrix comprises a hydrogel.

In some embodiments, a substrate herein can be any support that is insoluble in aqueous liquid and which allows for positioning of biological samples, analytes, features, and/or reagents (e.g., probes) on the support. In some embodiments, a biological sample can be attached to a substrate. Attachment of the biological sample can be irreversible or reversible, depending upon the nature of the sample and subsequent steps in the analytical method. In certain embodiments, the sample can be attached to the substrate reversibly by applying a suitable polymer coating to the substrate, and contacting the sample to the polymer coating. The sample can then be detached from the substrate, e.g., using an organic solvent that at least partially dissolves the polymer coating. Hydrogels are examples of polymers that are suitable for this purpose.

In some embodiments, the substrate can be coated or functionalized with one or more substances to facilitate attachment of the sample to the substrate. Suitable substances that can be used to coat or functionalize the substrate include, but are not limited to, lectins, poly-lysine, antibodies, and polysaccharides.

In some embodiments, the biological sample comprises an exogenous or endogenous molecule. In some embodiments, the endogenous molecule comprises a RNA. In some embodiments, the biological sample comprises an analyte. In some embodiments, the analyte comprises a RNA. In some embodiments, the biological sample comprises a RNA.

In some embodiments, the biological sample comprises cells or cellular components. In some embodiments, the biological sample comprises a tissue.

A variety of steps can be performed to prepare or process a biological sample for and/or during an assay. Except where indicated otherwise, the preparative or processing steps described below can generally be combined in any manner and in any order to appropriately prepare or process a particular sample for and/or analysis.

### (i) Preparation

A biological sample can be harvested from a subject (e.g., via surgical biopsy, whole subject sectioning) or grown in vitro on a growth substrate or culture dish as a population of cells, and prepared for analysis as a tissue slice or tissue section. Grown samples may be sufficiently thin for analysis without further processing steps. Alternatively, grown samples, and samples obtained via biopsy or sectioning, can be prepared as thin tissue sections using a mechanical cutting apparatus such as a vibrating blade microtome. As another alternative, in some embodiments, a thin tissue section can be prepared by applying a touch imprint of a biological sample to a suitable substrate material.

The thickness of the tissue section can be a fraction of (e.g., less than 0.9, 0.8, 0.7, 0.6, 0.5, 0.4, 0.3, 0.2, or 0.1) the maximum cross-sectional dimension of a cell. However, tissue sections having a thickness that is larger than the maximum cross-section cell dimension can also be used. For example, cryostat sections can be used, which can be, e.g., 10-20 µm thick.

More generally, the thickness of a tissue section typically depends on the method used to prepare the section and the physical characteristics of the tissue, and therefore sections having a wide variety of different thicknesses can be prepared and used. For example, the thickness of the tissue section can be at least 0.1, 0.2, 0.3, 0.4, 0.5, 0.7, 1.0, 1.5, 2, 3, 4, 5, 6, 7, 8, 9, 10, 12, 13, 14, 15, 20, 30, 40, or 50 µm. Thicker sections can also be used if desired or convenient, e.g., at least 70, 80, 90, or 100 µm or more. Typically, the thickness of a tissue section is between 1-100 µm, 1-50 µm, 1-30 µm, 1-25 µm, 1-20 µm, 1-15 µm, 1-10 µm, 2-8 µm, 3-7 µm, or 4-6 µm, but as mentioned above, sections with thicknesses larger or smaller than these ranges can also be analyzed. In some embodiments, the tissue slice is between about 1-50 µm in thickness.

Multiple sections can also be obtained from a single biological sample. For example, multiple tissue sections can be obtained from a surgical biopsy sample by performing serial sectioning of the biopsy sample using a sectioning blade. Spatial information among the serial sections can be preserved in this manner, and the sections can be analyzed successively to obtain three-dimensional information about the biological sample.

In some embodiments, the biological sample (e.g., a tissue section as described above) can be prepared by deep freezing at a temperature suitable to maintain or preserve the integrity (e.g., the physical characteristics) of the tissue structure. The frozen tissue sample can be sectioned, e.g., thinly sliced, onto a substrate surface using any number of suitable methods. For example, a tissue sample can be prepared using a chilled microtome (e.g., a cryostat) set at a temperature suitable to maintain both the structural integrity of the tissue sample and the chemical properties of the nucleic acids in the sample. Such a temperature can be, e.g., less than -15°C, less than -20°C, or less than -25°C.

In some embodiments, the biological sample is a frozen tissue sample. In other embodiments, the biological sample is a fresh tissue sample. In some embodiments, the sample is a fresh frozen tissue sample (FF). In some embodiments, the sample is a tissue section of a tissue block.

In some embodiments, the biological sample can be prepared using formalin-fixation and paraffin-embedding (FFPE), which are established methods. In some embodiments, FFPE can be performed prior to embedding the sample in a matrix. In some embodiments, cell suspensions and other non-tissue samples can be prepared using formalin-fixation and paraffin-embedding. Following fixation of the sample and embedding in a paraffin or resin block, the sample can be sectioned as described above. Prior to analysis (e.g., prior to matrix-embedding or introduction of a matrix forming material), the paraffin-embedding material can be removed from the tissue section (e.g., deparaffinization) by incubating the tissue section in an appropriate solvent (e.g., xylene) followed by a rinse (e.g., 99.5% ethanol for 2 minutes, 96% ethanol for 2 minutes, and 70% ethanol for 2 minutes).

As an alternative to formalin fixation described above, a biological sample can be fixed in any of a variety of other fixatives to preserve the biological structure of the sample prior to analysis. For example, a sample can be fixed via immersion in ethanol, methanol, acetone, paraformaldehyde (PFA)-Triton, and combinations thereof.

In some embodiments, the methods provided herein comprise one or more post-fixing (also referred to as postfixation) steps. In some embodiments, one or more post-fixing step is performed after contacting a sample with a polynucleotide disclosed herein, e.g., a probe. In some embodiments, one or more post-fixing step is performed after a hybridization complex comprising the nucleic acid molecule and a target is formed in a sample. In some embodiments, one or more post-fixing step is performed prior to a ligation reaction disclosed herein.

In some embodiments, a method disclosed herein comprises de-crosslinking the reversibly cross-linked biological sample. The de-crosslinking does not need to be complete.

In some embodiments, a biological sample can be permeabilized to facilitate transfer of species (such as probes) into the sample. If a sample is not permeabilized sufficiently, the transfer of species (such as probes) into the sample may be too low to enable adequate analysis. Conversely, if the tissue sample is too permeable, the relative spatial relationship of the analytes within the tissue sample can be lost. Hence, a balance between permeabilizing the tissue sample enough to obtain good signal intensity while still maintaining the spatial resolution of the analyte distribution in the sample is desirable.

In general, a biological sample can be permeabilized by exposing the sample to one or more permeabilizing agents. Suitable agents for this purpose include, but are not limited to, organic solvents (e.g., acetone, ethanol, and methanol), cross-linking agents (e.g., paraformaldehyde), detergents (e.g., saponin, Triton X-100^{™} or Tween-20^{™}), and enzymes (e.g., trypsin, proteases). In some embodiments, the biological sample can be incubated with a cellular permeabilizing agent to facilitate permeabilization of the sample. Additional methods for sample permeabilization are described, for example, in Jamur et al., Method Mol. Biol. 588:63-66, 2010, the entire contents of which are incorporated herein by reference. Any suitable method for sample permeabilization can generally be used in connection with the samples described herein.

In some embodiments, the biological sample can be permeabilized by any suitable methods. For example, one or more lysis reagents can be added to the sample. Examples of suitable lysis agents include, but are not limited to, bioactive reagents such as lysis enzymes that are used for lysis of different cell types, e.g., gram positive or negative bacteria, plants, yeast, mammalian, such as lysozymes, achromopeptidase, lysostaphin, labiase, kitalase, lyticase, and a variety of other commercially available lysis enzymes. Other lysis agents can additionally or alternatively be added to the biological sample to facilitate permeabilization. For example, surfactant-based lysis solutions can be used to lyse sample cells. Lysis solutions can include ionic surfactants such as, for example, sarcosyl and sodium dodecyl sulfate (SDS). More generally, chemical lysis agents can include, without limitation, organic solvents, chelating agents, detergents, surfactants, and chaotropic agents.

Additional reagents can be added to a biological sample to perform various functions prior to analysis of the sample. In some embodiments, DNase and RNase inactivating agents or inhibitors such as proteinase K, and/or chelating agents such as EDTA, can be added to the sample. For example, a method disclosed herein may comprise a step for increasing accessibility of a nucleic acid for binding, e.g., a denaturation step to open up DNA in a cell for hybridization by a probe. For example, proteinase K treatment may be used to free up DNA with proteins bound thereto.

### (ii) Embedding

In some embodiments, the biological sample can be embedded in a matrix (e.g., a hydrogel matrix). Any methods and aspects of hydrogel embedding of biological samples are described for example in Chen et al., Science 347(6221):543-548, 2015, the content of which is herein incorporated by reference in its entirety.

In some embodiments, the biological sample comprises a RNA. In some embodiments, embedding a biological sample comprising a RNA anchors the RNA to the matrix. Embedding the sample in this manner typically involves contacting the biological sample with a hydrogel such that the biological sample becomes surrounded by the hydrogel. For example, the sample can be embedded by contacting the sample with a suitable polymer material, and activating the polymer material to form a hydrogel. In some embodiments, the hydrogel is formed such that the hydrogel is internalized within the biological sample. Biological samples can include analytes (e.g., protein, RNA, and/or DNA) embedded in a 3D matrix. In some embodiments, amplicons (e.g., rolling circle amplification products) derived from or associated with analytes (e.g., protein, RNA, and/or DNA) can be embedded in a 3D matrix. In some embodiments, a 3D matrix may comprise a network of natural molecules and/or synthetic molecules that are chemically and/or enzymatically linked, e.g., by crosslinking. In some embodiments, a 3D matrix may comprise a synthetic polymer. In some embodiments, a 3D matrix comprises a hydrogel.

In some aspects, a biological sample can be embedded in any of a variety of other embedding materials to provide structural substrate to the sample prior to sectioning and other handling steps. In some cases, the embedding material can be removed e.g., prior to analysis of tissue sections obtained from the sample. Suitable embedding materials include, but are not limited to, waxes, resins (e.g., methacrylate resins), epoxies, and agar.

Matrix-forming agents include acrylamide, bisacrylamide, cellulose, alginate, polyamide, agarose, dextran, or polyethylene glycol. The matrix-forming agents can form a matrix by three-dimensional polymerization and/or crosslinking of the matrix-forming agents using methods specific for the matrix-forming agents and methods, reagents and conditions. In some embodiments, the three-dimensional polymerized matrix is formed by subjecting the matrix-forming agent to polymerization (or to further polymerization, in the case of matrix-forming agents that are polymers such as polyethylene glycol). In some embodiments, the matrix comprises polyacrylamide, cellulose, alginate, polyamide, cross-linked agarose, cross-linked dextran or cross-linked polyethylene glycol.

According to one aspect, a biological sample comprising a RNA can be contacted with a matrix-forming agent to form a three-dimensional polymerized matrix thereby embedding the biological sample and anchoring the RNA. Forming a three-dimensional polymerized matrix can include adding a polymerization inducing catalyst, UV or functional cross-linker to allow the formation of the three-dimensional polymerized matrix.

In some embodiments, biological samples embedded in the three-dimensional polymerized matrix (e.g., hydrogel) can be cleared using any suitable method (e.g., by contacting the biological sample with a clearing agent). A clearing agent can be any suitable agent for clearing a biological sample. For example, biological samples embedded in the three-dimensional polymerized matrix can be cleared with a detergent, a lipase and/or a protease. In some embodiments, the detergent and lipase removes fatty molecules. In some embodiments, the detergent comprises an ionic detergent or a nonionic detergent. In some embodiments, the detergent comprises a non-ionic surfactant or an anionic surfactant. In some embodiments, the detergent comprises SDS, tergitol, NP-40, saponin, p-(1,1,3,3-tetramethylbutyl)-phenyl ether (also known as Triton X-100^{™}, octyl phenol ethoxylate, polyoxyethylene octyl phenyl ether, 4-octylphenol polyethoxylate, t-octylphenoxypolyethoxyethanol, and octoxynol-9), polysorbate 20 (also known as Tween-20^{™}, polyoxyethylene (20) sorbitan monolaurate, or PEG(20)sorbitan monolaurate), or any combinations thereof. In some embodiments, the lipase comprises a pancreatic, hepatic and/or lysosomal lipase, or any combinations thereof. In some embodiments, the lipase comprises sphingomyelinase or esterase, or a combination thereof. In some embodiments, the protease targets extracellular matrix, fibronectin, collagen and/or elastin. In some embodiments, the protease comprises proteinase K, pepsin, collagenase, trypsin, dispase, thermolysin, or alpha-chymotrypsin, or any combinations thereof. In some embodiments, the protease comprises proteinase K, pepsin, collagenase, trypsin, dispase, thermolysin, or alpha-chymotrypsin, or any combinations thereof. In some embodiments, the protease comprises Liberase^{™}, (Collagenase I, Collagenase II and thermolysin). In other embodiments, electrophoretic tissue clearing methods can be used to remove biological macromolecules from the hydrogel-embedded sample. In some embodiments, the detergent can remove protein (e.g., ribosome) from the tethered RNA. In some embodiments, a hydrogel-embedded sample is stored before or after clearing of hydrogel, in a medium (e.g., a mounting medium, methylcellulose, or other semi-solid mediums).

According to one aspect, the matrix is sufficiently optically transparent or otherwise has optical properties suitable for deep three dimensional imaging for high throughput information readout, such as for detection of probe or probe set (i.e., a detectable probe).

According to one aspect, the matrix is porous thereby allowing the introduction of reagents into the matrix at the site of a RNA molecule comprising a free 3' end. Porosity can result from polymerization and/or crosslinking of molecules used to make the matrix material. The diffusion property within the gel matrix is largely a function of the pore size. The molecular sieve size is chosen to allow for rapid diffusion of enzymes, oligonucleotides, formamide and other buffers used for amplification and sequencing (>50-nm). The molecular sieve size is also chosen so that large DNA or RNA amplicons do not readily diffuse within the matrix (<500-nm). The porosity is controlled by changing the cross-linking density, the chain lengths and the percentage of co-polymerized branching monomers. Additional control over the molecular sieve size and density of the matrix is achieved by adding additional cross-linkers such as functionalized polyethylene glycols. In some embodiments, the reagents introduced into the matrix include any of the reagents provided herein. In some embodiments, the reagents comprise a probe or probe set (e.g., detectable probe), amplification reagents (e.g., polymerase), and/or primers.

In some embodiments, the biological sample is reversibly cross-linked prior to or during an *in situ* assay. In some aspects, the analytes, polynucleotides and/or amplification product (e.g., amplicon) of an analyte or a probe bound thereto can be anchored to a polymer matrix. For example, the polymer matrix can be a hydrogel. In some embodiments, one or more of the polynucleotide probe(s) and/or amplification product (e.g., amplicon) thereof can be modified to contain functional groups that can be used as an anchoring site to attach the polynucleotide probes and/or amplification product to a polymer matrix. In some embodiments, a modified probe comprising oligo dT may be used to bind to mRNA molecules of interest, followed by reversible or irreversible crosslinking of the mRNA molecules.

In some embodiments, the biological sample is immobilized in a hydrogel via cross-linking of the polymer material that forms the hydrogel. Cross-linking can be performed chemically and/or photochemically, or alternatively by any other suitable hydrogel-formation method. A hydrogel may include a macromolecular polymer gel including a network. Within the network, some polymer chains can optionally be cross-linked, although cross-linking does not always occur.

In some embodiments, a hydrogel can include hydrogel subunits, such as, but not limited to, acrylamide, bis-acrylamide, polyacrylamide and derivatives thereof, poly(ethylene glycol) and derivatives thereof (e.g. PEG-acrylate (PEG-DA), PEG-RGD), gelatin-methacryloyl (GelMA), methacrylated hyaluronic acid (MeHA), polyaliphatic polyurethanes, polyether polyurethanes, polyester polyurethanes, polyethylene copolymers, polyamides, polyvinyl alcohols, polypropylene glycol, polytetramethylene oxide, polyvinyl pyrrolidone, polyacrylamide, poly (hydroxy ethyl acrylate), and poly (hydroxy ethyl methacrylate), collagen, hyaluronic acid, chitosan, dextran, agarose, gelatin, alginate, protein polymers, methylcellulose, and the like, and combinations thereof.

In some embodiments, a hydrogel includes a hybrid material, e.g., the hydrogel material includes elements of both synthetic and natural polymers. Examples of suitable hydrogels are described, for example, in U.S. Patent Nos. 6,391,937, 9,512,422, and 9,889,422, and in U.S. Patent Application Publication Nos. 2017/0253918, 2018/0052081 and 2010/0055733, the entire contents of each of which are incorporated herein by reference.

The composition and application of the hydrogel-matrix to a biological sample typically depends on the nature and preparation of the biological sample (e.g., sectioned, non-sectioned, type of fixation). As one example, where the biological sample is a tissue section, the hydrogel-matrix can include a monomer solution and an ammonium persulfate (APS) initiator/tetramethylethylenediamine (TEMED) accelerator solution. As another example, where the biological sample consists of cells (e.g., cultured cells or cells disassociated from a tissue sample), the cells can be incubated with the monomer solution and APS/TEMED solutions. For cells, hydrogel-matrix gels are formed in compartments, including but not limited to devices used to culture, maintain, or transport the cells. For example, hydrogel-matrices can be formed with monomer solution plus APS/TEMED added to the compartment to a depth ranging from about 0.1 µm to about 2 mm.

Additional methods and aspects of hydrogel embedding of biological samples are described for example in Chen et al., Science 347(6221):543-548, 2015, the entire contents of which are incorporated herein by reference.

In some embodiments, the hydrogel can form the substrate. In some embodiments, the substrate includes a hydrogel and one or more second materials. In some embodiments, the hydrogel is placed on top of one or more second materials. For example, the hydrogel can be pre-formed and then placed on top of, underneath, or in any other configuration with one or more second materials. In some embodiments, hydrogel formation occurs after contacting one or more second materials during formation of the substrate. Hydrogel formation can also occur within a structure (e.g., wells, ridges, projections, and/or markings) located on a substrate.

In some embodiments, hydrogel formation on a substrate occurs before, contemporaneously with, or after probes are provided to the sample. For example, hydrogel formation can be performed on the substrate already containing the probes.

In some embodiments, hydrogel formation occurs within a biological sample. In some embodiments, a biological sample (e.g., tissue section) is embedded in a hydrogel. In some embodiments, hydrogel subunits are infused into the biological sample, and polymerization of the hydrogel is initiated by an external or internal stimulus.

In embodiments in which a hydrogel is formed within a biological sample, functionalization chemistry can be used. In some embodiments, functionalization chemistry includes hydrogel-tissue chemistry (HTC). Any hydrogel-tissue backbone (e.g., synthetic or native) suitable for HTC can be used for anchoring biological macromolecules and modulating functionalization. Non-limiting examples of methods using HTC backbone variants include CLARITY, PACT, ExM, SWITCH and ePACT. In some embodiments, hydrogel formation within a biological sample is permanent. For example, biological macromolecules can permanently adhere to the hydrogel allowing multiple rounds of interrogation. In some embodiments, hydrogel formation within a biological sample is reversible. In some embodiments, HTC reagents are added to the hydrogel before, contemporaneously with, and/or after polymerization. In some embodiments, a cell labeling agent is added to the hydrogel before, contemporaneously with, and/or after polymerization. In some embodiments, a cell-penetrating agent is added to the hydrogel before, contemporaneously with, and/or after polymerization.

In some embodiments, additional reagents are added to the hydrogel subunits before, contemporaneously with, and/or after polymerization. For example, additional reagents can include but are not limited to oligonucleotides (e.g., probes), endonucleases to fragment DNA, fragmentation buffer for DNA, DNA polymerase enzymes, dNTPs used to amplify the nucleic acid and to attach the barcode to the amplified fragments. Other enzymes can be used, including without limitation, RNA polymerase, ligase, proteinase K, and DNAse. Additional reagents can also include reverse transcriptase enzymes, including enzymes with terminal transferase activity, primers, and oligonucleotides. In some embodiments, optical labels are added to the hydrogel subunits before, contemporaneously with, and/or after polymerization.

Hydrogels embedded within biological samples can be cleared using any suitable method. For example, electrophoretic tissue clearing methods can be used to remove biological macromolecules from the hydrogel-embedded sample. In some embodiments, a hydrogel-embedded sample is stored before or after clearing of hydrogel, in a medium (e.g., a mounting medium, methylcellulose, or other semi-solid mediums).

In some embodiments, a biological sample embedded in a matrix (e.g., a hydrogel) can be isometrically expanded. Isometric expansion methods that can be used include hydration, a preparative step in expansion microscopy, as described in, e.g., Chen et al., Science 347(6221):543-548, 2015 and U.S. Pat. 10,059,990, which are herein incorporated by reference in their entireties. Isometric expansion of the sample can increase the spatial resolution of the subsequent analysis of the sample. The increased resolution in spatial profiling can be determined by comparison of an isometrically expanded sample with a sample that has not been isometrically expanded. In some embodiments, a biological sample is isometrically expanded to a size at least 2x, 2.1x, 2.2x, 2.3x, 2.4x, 2.5x, 2.6x, 2.7x, 2.8x, 2.9x, 3x, 3.1x, 3.2x, 3.3x, 3.4x, 3.5x, 3.6x, 3.7x, 3.8x, 3.9x, 4x, 4.1x, 4.2x, 4.3x, 4.4x, 4.5x, 4.6x, 4.7x, 4.8x, or 4.9x its non-expanded size. In some embodiments, the sample is isometrically expanded to at least 2x and less than 20x of its non-expanded size.

### (iii) Staining and Immunohistochemistry (IHC)

To facilitate visualization, biological samples can be stained using a wide variety of stains and staining techniques. In some embodiments, for example, a sample can be stained using any number of stains and/or immunohistochemical reagents. One or more staining steps may be performed to prepare or process a biological sample for an assay described herein or may be performed during and/or after an assay. In some embodiments, the sample can be contacted with one or more nucleic acid stains, membrane stains (e.g., cellular or nuclear membrane), cytological stains, or combinations thereof. In some examples, the stain may be specific to proteins, phospholipids, DNA (e.g., dsDNA, ssDNA), RNA, an organelle or compartment of the cell. The sample may be contacted with one or more labeled antibodies (e.g., a primary antibody specific for the analyte of interest and a labeled secondary antibody specific for the primary antibody). In some embodiments, cells in the sample can be segmented using one or more images taken of the stained sample.

In some embodiments, the stain is performed using a lipophilic dye. In some examples, the staining is performed with a lipophilic carbocyanine or aminostyryl dye, or analogs thereof (e.g, DiI, DiO, DiR, DiD). Other cell membrane stains may include FM and RH dyes or immunohistochemical reagents specific for cell membrane proteins. In some examples, the stain may include but not limited to, acridine orange, Bismarck brown, carmine, coomassie blue, cresyl violet, DAPI, eosin, ethidium bromide, acid fuchsine, haematoxylin, Hoechst stains, iodine, methyl green, methylene blue, neutral red, Nile blue, Nile red, osmium tetroxide, ruthenium red, propidium iodide, rhodamine (e.g., rhodamine B), or safranine or derivatives thereof. In some embodiments, the sample may be stained with haematoxylin and eosin (H&E).

The sample can be stained using hematoxylin and eosin (H&E) staining techniques, using Papanicolaou staining techniques, Masson's trichrome staining techniques, silver staining techniques, Sudan staining techniques, and/or using Periodic Acid Schiff (PAS) staining techniques. PAS staining is typically performed after formalin or acetone fixation. In some embodiments, the sample can be stained using Romanowsky stain, including Wright's stain, Jenner's stain, Can-Grunwald stain, Leishman stain, and Giemsa stain.

In some embodiments, biological samples can be destained. Any suitable methods of destaining or discoloring a biological sample may be utilized, and generally depend on the nature of the stain(s) applied to the sample. For example, in some embodiments, one or more immunofluorescent stains are applied to the sample via antibody coupling. Such stains can be removed using techniques such as cleavage of disulfide linkages via treatment with a reducing agent and detergent washing, chaotropic salt treatment, treatment with antigen retrieval solution, and treatment with an acidic glycine buffer. Methods for multiplexed staining and destaining are described, for example, in Bolognesi et al., J. Histochem. Cytochem. 2017; 65(8): 431-444, Lin et al., Nat Commun. 2015; 6:8390, Pirici et al., J. Histochem. Cytochem. 2009; 57:567-75, and Glass et al., J. Histochem. Cytochem. 2009; 57:899-905, the entire contents of each of which are incorporated herein by reference.

In some embodiments, the embedded biological sample is stained.

### B. Analytes

A biological sample may comprise one or a plurality of analytes of interest. Methods for performing multiplexed assays to analyze two or more different analytes in a single biological sample are provided.

The methods disclosed herein can be used to detect and analyze a wide variety of different analytes (e.g., including RNA as described in Section II and in combination with other analytes). In some aspects, an analyte can include any biological substance, structure, moiety, or component to be analyzed. In some aspects, a target disclosed herein may similarly include any analyte of interest. In some examples, a target or analyte can be directly or indirectly detected.

Analytes can be derived from a specific type of cell and/or a specific subcellular region. For example, analytes can be derived from cytosol, from cell nuclei, from mitochondria, from microsomes, and more generally, from any other compartment, organelle, or portion of a cell. Permeabilizing agents that specifically target certain cell compartments and organelles can be used to selectively release analytes from cells for analysis, and/or allow access of one or more reagents (e.g., probes for analyte detection) to the analytes in the cell or cell compartment or organelle.

The analyte may include any biomolecule or chemical compound, including a macromolecule such as a protein or peptide, a lipid or a nucleic acid molecule, or a small molecule, including organic or inorganic molecules. The analyte may be a cell or a microorganism, including a virus, or a fragment or product thereof. An analyte can be any substance or entity for which a specific binding partner (e.g. an affinity binding partner) can be developed. Such a specific binding partner may be a nucleic acid probe (for a nucleic acid analyte).

Analytes of particular interest may include nucleic acid molecules, such as RNA (e.g. mRNA, microRNA, rRNA, snRNA, viral RNA, etc.), and synthetic and/or modified nucleic acid molecules, (e.g. including nucleic acid domains comprising or consisting of synthetic or modified nucleotides such as LNA, PNA, morpholino, etc.), proteinaceous molecules such as peptides, polypeptides, proteins or prions or any molecule which includes a protein or polypeptide component, etc., or fragments thereof, or a lipid or carbohydrate molecule, or any molecule which comprise a lipid or carbohydrate component. The analyte may be a single molecule or a complex that contains two or more molecular subunits, e.g. including but not limited to protein-RNA complexes, which may or may not be covalently bound to one another, and which may be the same or different. Thus in addition to cells or microorganisms, such a complex analyte may also be a protein complex or protein interaction. Such a complex or interaction may thus be a homo- or hetero-multimer. Aggregates of molecules, e.g. proteins may also be target analytes, for example aggregates of the same protein or different proteins. The analyte may also be a complex between proteins or peptides and nucleic acid molecules such as RNA, e.g. interactions between proteins and nucleic acids, e.g. regulatory factors, such as transcription factors, and RNA.

### (i) Endogenous Analytes

In some embodiments, an analyte herein is endogenous to a biological sample and can include nucleic acid analytes and non-nucleic acid analytes. Methods and compositions disclosed herein can be used to analyze nucleic acid analytes (e.g., by immobilizing or tethering any fragmented RNAs to an endogenous molecule in the biological sample or an exogenous molecule delivered to the biological sample as described in Section II and using a nucleic acid probe or probe set that directly or indirectly hybridizes to the immobilized or tethered nucleic acid analyte).

Examples of nucleic acid analytes include RNA analytes such as various types of coding and non-coding RNA. Examples of the different types of RNA analytes include messenger RNA (mRNA), including a nascent RNA, a pre-mRNA, a primary-transcript RNA, and a processed RNA, such as a capped mRNA (e.g., with a 5' 7-methyl guanosine cap), a polyadenylated mRNA (poly-A tail at the 3' end), and a spliced mRNA in which one or more introns have been removed. Also included in the analytes disclosed herein are non-capped mRNA, a non-polyadenylated mRNA, and a non-spliced mRNA. The RNA analyte can be a transcript of another nucleic acid molecule (e.g., DNA or RNA such as viral RNA) present in a tissue sample. Examples of a non-coding RNAs (ncRNA) that is not translated into a protein include transfer RNAs (tRNAs) and ribosomal RNAs (rRNAs), as well as small non-coding RNAs such as microRNA (miRNA), small interfering RNA (siRNA), Piwi-interacting RNA (piRNA), small nucleolar RNA (snoRNA), small nuclear RNA (snRNA), extracellular RNA (exRNA), small Cajal body-specific RNAs (scaRNAs), and the long ncRNAs such as Xist and HOTAIR. The RNA can be small (e.g., less than 200 nucleic acid bases in length) or large (e.g., RNA greater than 200 nucleic acid bases in length). Examples of small RNAs include 5.8S ribosomal RNA (rRNA), 5S rRNA, tRNA, miRNA, siRNA, snoRNAs, piRNA, tRNA-derived small RNA (tsRNA), and small rDNA-derived RNA (srRNA). The RNA can be double-stranded RNA or single-stranded RNA. The RNA can be circular RNA. The RNA can be a bacterial rRNA (e.g., 16s rRNA or 23s rRNA). In some embodiments described herein, an analyte may be a fragmented RNA.

Methods and compositions disclosed herein can be used to analyze any number of analytes. For example, the number of analytes that are analyzed can be at least about 2, at least about 3, at least about 4, at least about 5, at least about 6, at least about 7, at least about 8, at least about 9, at least about 10, at least about 11, at least about 12, at least about 13, at least about 14, at least about 15, at least about 20, at least about 25, at least about 30, at least about 40, at least about 50, at least about 100, at least about 1,000, at least about 10,000, at least about 100,000 or more different analytes present in a region of the sample or within an individual feature of the substrate.

### (ii) Labelling Agents

In some embodiments, provided herein are methods and compositions for analyzing endogenous analytes (e.g., RNA) in a sample and other analytes using one or more labelling agents. In some aspects, the methods for immobilizing or tethering fragmented RNAs to an endogenous molecule in the biological sample or an exogenous molecule delivered to the biological sample as described in Section II is compatible with protein analysis (e.g., using a labelling agent). In some embodiments, an analyte labelling agent may include an agent that interacts with an analyte (e.g., an endogenous analyte in a sample). In some embodiments, the labelling agents can comprise a reporter oligonucleotide that is indicative of the analyte or portion thereof interacting with the labelling agent. For example, the reporter oligonucleotide may comprise a barcode sequence that permits identification of the labelling agent. In some cases, the sample contacted by the labelling agent can be further contacted with a probe (e.g., a single-stranded probe sequence), that hybridizes to a reporter oligonucleotide of the labelling agent, in order to identify the analyte associated with the labelling agent. In some embodiments, the analyte labelling agent comprises an analyte binding moiety and a labelling agent barcode domain comprising one or more barcode sequences, e.g., a barcode sequence that corresponds to the analyte binding moiety and/or the analyte. An analyte binding moiety barcode includes to a barcode that is associated with or otherwise identifies the analyte binding moiety. In some embodiments, by identifying an analyte binding moiety by identifying its associated analyte binding moiety barcode, the analyte to which the analyte binding moiety binds can also be identified. An analyte binding moiety barcode can be a nucleic acid sequence of a given length and/or sequence that is associated with the analyte binding moiety. An analyte binding moiety barcode can generally include any of the variety of aspects of barcodes described herein.

In some embodiments, the method comprises one or more post-fixing (also referred to as post-fixation) steps after contacting the sample with one or more labelling agents.

In some embodiments, an analyte binding moiety may include any molecule or moiety capable of binding to an analyte (e.g., a biological analyte, e.g., a macromolecular constituent). A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have coupled thereto a first reporter oligonucleotide, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat. Pub. 20190367969, which are each incorporated by reference herein in their entirety.

In some embodiments, an analyte binding moiety includes one or more antibodies or antigen binding fragments thereof. The antibodies or antigen binding fragments including the analyte binding moiety can specifically bind to a target analyte. In some embodiments, the analyte is a protein (e.g., a protein on a surface of the biological sample (e.g., a cell) or an intracellular protein). In some embodiments, a plurality of analyte labelling agents comprising a plurality of analyte binding moieties bind a plurality of analytes present in a biological sample. In some embodiments, the plurality of analytes includes a single species of analyte (e.g., a single species of polypeptide). In some embodiments in which the plurality of analytes includes a single species of analyte, the analyte binding moieties of the plurality of analyte labelling agents are the same. In some embodiments in which the plurality of analytes includes a single species of analyte, the analyte binding moieties of the plurality of analyte labelling agents are the different (e.g., members of the plurality of analyte labelling agents can have two or more species of analyte binding moieties, wherein each of the two or more species of analyte binding moieties binds a single species of analyte, e.g., at different binding sites). In some embodiments, the plurality of analytes includes multiple different species of analyte (e.g., multiple different species of polypeptides).

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide.

In some aspects, these reporter oligonucleotides may comprise nucleic acid barcode sequences that permit identification of the labelling agent which the reporter oligonucleotide is coupled to. The selection of oligonucleotides as the reporter may provide advantages of being able to generate significant diversity in terms of sequence, while also being readily attachable to most biomolecules, e.g., antibodies, etc., as well as being readily detected, e.g., using the *in situ* detection techniques described herein.

Attachment (coupling) of the reporter oligonucleotides to the labelling agents may be achieved through any of a variety of direct or indirect, covalent or non-covalent associations or attachments. In some embodiments, the oligonucleotide attached to a labelling agent comprises a sequence that can serve as a primer and can be used as a reporter (e.g., a barcode). In some embodiments, the oligonucleotide attached to a labelling agent comprises both a reporter sequence (e.g., a barcode) and a sequence serving as a primer. For example, oligonucleotides may be covalently attached to a portion of a labelling agent (such a protein, e.g., an antibody or antibody fragment) using chemical conjugation techniques (e.g., Lightning-Link^{®} antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715, the content of which is herein incorporated by reference in its entirety. Likewise, protein and peptide biotinylation techniques have been developed and are readily available. See, e.g., U.S. Pat. No. 6,265,552, which is entirely incorporated herein by reference for all purposes. Furthermore, click reaction chemistry may be used to couple reporter oligonucleotides to labelling agents. Commercially available kits, such as those from Thunderlink and Abcam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the labelling agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide or primer may be attached to the labeling agent through a labile bond (e.g., chemically labile, photolabile, thermally labile, etc.) as generally described for releasing molecules from supports elsewhere herein. In some instances, the reporter oligonucleotides described herein may include one or more functional sequences that can be used in subsequent processing, such as an adapter sequence or a unique molecular identifier (UMI) sequence.

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to a first oligonucleotide that is complementary (e.g., hybridizes) to a sequence of the reporter oligonucleotide.

### C. Detection and Analysis

In some aspects, the provided methods involve analyzing the tethered or immobilized RNA as described in Section II and optionally other analytes, e.g., by detecting or determining, one or more sequences present in probes or probe sets or products thereof (e.g., rolling circle amplification products thereof). In some embodiments, the detecting is performed at one or more locations in a biological sample. In some embodiments, the locations are the locations of tethered or immobilized RNA transcripts in the biological sample. In some embodiments, the locations are the locations at which probes or probe sets hybridize to the RNA transcripts in the biological sample, and are optionally ligated and amplified by rolling circle amplification.

In some embodiments, the method provided herein comprises contacting the biological sample with a probe or probe set that binds directly or indirectly to the RNA. In some embodiments, the probe or probe set is a detectable probe. In some embodiments, the probe or probe set is a circular or circularizable probe or probe set. In some embodiments, the method comprises circularizing the circularizable probe or probe set using the RNA or a product thereof as a template. In some embodiments, the method comprises generating an RCA product using the circular or circularizable probe as a template.

In some embodiments, the circular or circularizable probe or probe set is capable of binding the fragmented RNA. In some embodiments, the method further comprises generating a RCA product of the circular or circularizable probe or probe set; and detecting a signal associated with a fluorescently labeled probe that directly or indirectly binds to the RCA product.

In some embodiments, the probe or probe set comprises a barcode sequence. In some embodiments, the method comprises detecting the barcode sequence or a complement thereof in the probe or probe set or in a product of the probe or probe set. In some embodiments, the probe or probe set comprises an intermediate probe and a detection oligonucleotide. In some embodiments, the detecting comprises a plurality of repeated cycles of hybridization and removal of probes (e.g., detectably labeled probes, or intermediate probes that bind to detectably labeled probes) to the primary probe or probe set hybridized to the target nucleic acid, or to a rolling circle amplification product generated from the probe or probe set hybridized to the target nucleic acid.. In some embodiments, a single probe or probe set is hybridized to each target nucleic acid. In some aspects, number of detected RCPs, size of RCPs and/or signal to background ratio is improved in tethered samples (e.g., as described in Section II) compared to samples where RNA is not tethered and only one probe or probe set is needed to bind each target nucleic acid (e.g., RNA) to generate sufficient signal for detection.

In some embodiments, the method comprises contacting the biological sample with a circular or circularizable probe, wherein the circular or circularizable probe binds the fragmented RNA and generates a rolling circle amplification (RCA) product.

In some embodiments, the method comprises imaging the biological sample to detect a signal associated a probe or probe set (e.g., a circular or circularizable probe or probe set) that binds directly or indirectly to the RNA. In some embodiments, the method comprises imaging the biological sample to detect the RCA product. In some embodiments, imaging comprises detecting a signal associated the probe or probe set or the RCA product, optionally with a fluorescently labeled probe that directly or indirectly binds to the RCA product.

### (a) Hybridization, Ligation, and Amplification

In some embodiments, the method comprises contacting the biological sample with a probe or probe set that hybridizes directly or indirectly to the RNA. For purposes of hybridization, two nucleic acid sequences are "substantially complementary" if at least 60% (e.g., at least 70%, at least 80%, or at least 90%) of their individual bases are complementary to one another. Various probes and probe sets can be hybridized to an endogenous analyte (e.g., RNA) and/or a labelling agent.

In some embodiments, the method comprises generating a ligation product with a probe or probe set that hybridizes directly or indirectly to the RNA.

In some embodiments, provided herein is a probe or probe set capable of DNA-templated ligation, such as from a cDNA molecule. See, e.g., U.S. Pat. 8,551,710, which is hereby incorporated by reference in its entirety. In some embodiments, provided herein is a probe or probe set capable of RNA-templated ligation. See, e.g., U.S. Pat. Pub. 2020/0224244 which is hereby incorporated by reference in its entirety. In some embodiments, the probe set is a SNAIL probe set. See, e.g., U.S. Pat. Pub. 20190055594, which is hereby incorporated by reference in its entirety. In some embodiments, provided herein is a multiplexed proximity ligation assay. See, e.g., U.S. Pat. Pub. 20140194311 which is hereby incorporated by reference in its entirety. In some embodiments, provided herein is a probe or probe set capable of proximity ligation, for instance a proximity ligation assay for RNA (e.g., PLAYR) probe set. See, e.g., U.S. Pat. Pub. 20160108458, which is hereby incorporated by reference in its entirety. In some embodiments, a circular probe can be indirectly hybridized to the target nucleic acid. In some embodiments, the circular construct is formed from a probe set capable of proximity ligation, for instance a proximity ligation *in situ* hybridization (PLISH) probe set. See, e.g., U.S. Pat. Pub. 2020/0224243 which is hereby incorporated by reference in its entirety.

In some embodiments, the ligation involves chemical ligation. In some embodiments, the ligation involves template dependent ligation. In some embodiments, the ligation involves template independent ligation. In some embodiments, the ligation involves enzymatic ligation.

In some embodiments, the enzymatic ligation involves use of a ligase. In some aspects, the ligase used herein comprises an enzyme that is commonly used to join polynucleotides together or to join the ends of a single polynucleotide. An RNA ligase, a DNA ligase, or another variety of ligase can be used to ligate two nucleotide sequences together. Ligases comprise ATP-dependent double-strand polynucleotide ligases, NAD-i-dependent double-strand DNA or RNA ligases and single-strand polynucleotide ligases, for example any of the ligases described in EC 6.5.1.1 (ATP-dependent ligases), EC 6.5.1.2 (NAD+-dependent ligases), EC 6.5.1.3 (RNA ligases). Specific examples of ligases comprise bacterial ligases such as E. coli DNA ligase, Tth DNA ligase, Thermococcus sp. (strain 9° N) DNA ligase (9°N^{™} DNA ligase, New England Biolabs), Taq DNA ligase, Ampligase^{™} (Epicentre Biotechnologies) and phage ligases such as T3 DNA ligase, T4 DNA ligase and T7 DNA ligase and mutants thereof. In some embodiments, the ligase is a T4 RNA ligase. In some embodiments, the ligase is a splintR ligase. In some embodiments, the ligase is a single stranded DNA ligase. In some embodiments, the ligase is a T4 DNA ligase. In some embodiments, the ligase is a ligase that has an DNA-splinted DNA ligase activity. In some embodiments, the ligase is a ligase that has an RNA-splinted DNA ligase activity.

In some embodiments, the ligation herein is a direct ligation. In some embodiments, the ligation herein is an indirect ligation. "Direct ligation" means that the ends of the polynucleotides hybridize immediately adjacently to one another to form a substrate for a ligase enzyme resulting in their ligation to each other (intramolecular ligation). Alternatively, "indirect" means that the ends of the polynucleotides hybridize non-adjacently to one another, e.g., separated by one or more intervening nucleotides or "gaps". In some embodiments, said ends are not ligated directly to each other, but instead occurs either via the intermediacy of one or more intervening (so-called "gap" or "gap-filling" (oligo)nucleotides) or by the extension of the 3' end of a probe to "fill" the "gap" corresponding to said intervening nucleotides (intermolecular ligation). In some cases, the gap of one or more nucleotides between the hybridized ends of the polynucleotides may be "filled" by one or more "gap" (oligo)nucleotide(s) which are complementary to a splint, circularizable probe (e.g., padlock probe), or target nucleic acid. The gap may be a gap of 1 to 60 nucleotides or a gap of 1 to 40 nucleotides or a gap of 3 to 40 nucleotides. In specific embodiments, the gap may be a gap of about 1, 2, 3, 4, 5, 6, 7, 8, 9 or 10 or more nucleotides, of any integer (or range of integers) of nucleotides in between the indicated values. In some embodiments, the gap between said terminal regions may be filled by a gap oligonucleotide or by extending the 3' end of a polynucleotide. In some cases, ligation involves ligating the ends of the probe to at least one gap (oligo)nucleotide, such that the gap (oligo)nucleotide becomes incorporated into the resulting polynucleotide. In some embodiments, the ligation herein is preceded by gap filling. In other embodiments, the ligation herein does not require gap filling.

In some embodiments, the method comprises detecting a product that includes a molecule or a complex generated in a series of reactions, e.g., hybridization, ligation, extension, replication, transcription/reverse transcription, and/or amplification (e.g., rolling circle amplification), in any suitable combination.

In some aspects, the polynucleotides and/or amplification product (e.g., amplicon) can be anchored to a polymer matrix. For example, the polymer matrix can be a hydrogel. In some embodiments, one or more of the polynucleotide probe(s) can be modified to contain functional groups that can be used as an anchoring site to attach the polynucleotide probes and/or amplification product to a polymer matrix. Exemplary modification and polymer matrix that can be employed in accordance with the provided embodiments comprise those described in, for example, WO 2014/163886, WO 2017/079406, US 2016/0024555, US 2018/0251833 and US 2017/0219465. In some examples, the scaffold also contains modifications or functional groups that can react with or incorporate the modifications or functional groups of the probe set or amplification product. In some examples, the scaffold can comprise oligonucleotides, polymers or chemical groups, to provide a matrix and/or support structures.

The amplification products may be immobilized within the matrix generally at the location of the nucleic acid being amplified, thereby creating a localized colony of amplicons. The amplification products may be immobilized within the matrix by steric factors. The amplification products may also be immobilized within the matrix by covalent or noncovalent bonding. In this manner, the amplification products may be considered to be attached to the matrix. By being immobilized to the matrix, such as by covalent bonding or cross-linking, the size and spatial relationship of the original amplicons is maintained. By being immobilized to the matrix, such as by covalent bonding or cross-linking, the amplification products are resistant to movement or unraveling under mechanical stress.

In some aspects, the amplification products are copolymerized and/or covalently attached to the surrounding matrix thereby preserving their spatial relationship and any information inherent thereto. For example, if the amplification products are those generated from DNA or RNA within a cell embedded in the matrix, the amplification products can also be functionalized to form covalent attachment to the matrix preserving their spatial information within the cell thereby providing a subcellular localization distribution pattern. In some embodiments, the provided methods involve embedding the one or more polynucleotide probe sets and/or the amplification products in the presence of hydrogel subunits to form one or more hydrogel-embedded amplification products. In some embodiments, the hydrogel-tissue chemistry described comprises covalently attaching nucleic acids to in situ synthesized hydrogel for tissue clearing, enzyme diffusion, and multiple-cycle sequencing while an existing hydrogel-tissue chemistry method cannot. In some embodiments, to enable amplification product embedding in the tissue-hydrogel setting, amine-modified nucleotides are comprised in the amplification step (e.g., RCA), functionalized with an acrylamide moiety using acrylic acid N-hydroxysuccinimide esters, and copolymerized with acrylamide monomers to form a hydrogel.

In some embodiments, a method disclosed herein may also comprise one or more signal amplification components. In some embodiments, the present disclosure relates to the detection of nucleic acids sequences *in situ* using probe hybridization and generation of amplified signals associated with the probes, wherein background signal is reduced and sensitivity is increased. In some embodiments, an analyte disclosed herein can be detected in with a method that comprises signal amplification. Exemplary signal amplification methods include targeted deposition of detectable reactive molecules around the site of probe hybridization, targeted assembly of branched structures (e.g., bDNA or branched assay using locked nucleic acid (LNA)), programmed *in situ* growth of concatemers by enzymatic rolling circle amplification (RCA) (e.g., as described in US 2019/0055594 incorporated herein by reference), hybridization chain reaction, assembly of topologically catenated DNA structures using serial rounds of chemical ligation (clampFISH), signal amplification via hairpin-mediated concatemerization (e.g., as described in US 2020/0362398 incorporated herein by reference), e.g., primer exchange reactions such as signal amplification by exchange reaction (SABER) or SABER with DNA-Exchange (Exchange-SABER). In some embodiments, a non-enzymatic signal amplification method may be used.

The detectable reactive molecules may comprise tyramide, such as used in tyramide signal amplification (TSA) or multiplexed catalyzed reporter deposition (CARD)-FISH. In some embodiments, the detectable reactive molecule may be releasable and/or cleavable from a detectable label such as a fluorophore. In some embodiments, a method disclosed herein comprises multiplexed analysis of a biological sample comprising consecutive cycles of probe hybridization, fluorescence imaging, and signal removal, where the signal removal comprises removing the fluorophore from a fluorophore-labeled reactive molecule (e.g., tyramide). Exemplary detectable reactive reagents and methods are described in US 6,828,109, US 2019/0376956, US 2019/0376956, WO 2020/102094, WO 2020/163397, and WO 2021/067475, all of which are incorporated herein by reference in their entireties.

The detectable label can be directly detectable by itself (e.g., radioisotope labels or fluorescent labels) or can be indirectly detectable, e.g., by catalyzing chemical alterations of a substrate compound or composition, which substrate compound or composition is directly detectable. Detectable labels can be suitable for small scale detection and/or suitable for high-throughput screening. As such, suitable detectable labels include, but are not limited to, radioisotopes, fluorophores, chemiluminescent compounds, bioluminescent compounds, and dyes.

The detectable label can be qualitatively detected (e.g., optically or spectrally), or it can be quantified. Qualitative detection generally includes a detection method in which the existence or presence of the detectable label is confirmed, whereas quantifiable detection generally includes a detection method having a quantifiable (e.g., numerically reportable) value such as an intensity, duration, polarization, and/or other properties. In some embodiments, the detectable label is bound to a feature or to a capture probe associated with a feature. For example, detectably labelled features can include a fluorescent, a colorimetric, or a chemiluminescent label attached to a bead (see, for example, Rajeswari et al., J. Microbiol Methods 139:22-28, 2017, and Forcucci et al., J. Biomed Opt. 10:105010, 2015, the entire contents of each of which are incorporated herein by reference).

In some embodiments, a plurality of detectable labels can be attached to a feature, capture probe, or composition to be detected. For example, detectable labels can be incorporated during nucleic acid polymerization or amplification (e.g., Cy5^{®}-labelled nucleotides, such as Cy5^{®}-dCTP). Any suitable detectable label can be used. In some embodiments, the detectable label is a fluorophore. For example, the fluorophore can be from a group that includes: 7-AAD (7-Aminoactinomycin D), Acridine Orange (+DNA), Acridine Orange (+RNA), Alexa Fluor^{®} 350, Alexa Fluor^{®} 430, Alexa Fluor^{®} 488, Alexa Fluor^{®} 532, Alexa Fluor^{®} 546, Alexa Fluor^{®} 555, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, Allophycocyanin (APC), AMCA / AMCA-X, 7-Aminoactinomycin D (7-AAD), 7- Amino-4-methylcoumarin, 6-Aminoquinoline, Aniline Blue, ANS, APC-Cy7, ATTO-TAG^{™} CBQCA, ATTO-TAG^{™} FQ, Auramine O-Feulgen, BCECF (high pH), BFP (Blue Fluorescent Protein), BFP / GFP FRET, BOBO^{™}-1 / BO-PRO^{™}-1, BOBO^{™}-3 / BO-PRO^{™}-3, BODIPY^{®} FL, BODIPY^{®} TMR, BODIPY^{®} TR-X, BODIPY^{®} 530/550, BODIPY^{®} 558/568, BODIPY^{®} 564/570, BODIPY^{®} 581/591, BODIPY^{®} 630/650-X, BODIPY^{®} 650-665-X, BTC, Calcein, Calcein Blue, Calcium Crimson^{™}, Calcium Green-1^{™}, Calcium Orange^{™}, Calcofluor^{®} White, 5-Carboxyfluoroscein (5-FAM), 5-Carboxynaphthofluoroscein, 6-Carboxyrhodamine 6G, 5-Carboxytetramethylrhodamine (5-TAMRA), Carboxy-X-rhodamine (5-ROX), Cascade Blue^{®}, Cascade Yellow^{™}, CCF2 (GeneBLAzer^{™}), CFP (Cyan Fluorescent Protein), CFP / YFP FRET, Chromomycin A3, Cl-NERF (low pH), CPM, 6-CR 6G, CTC Formazan, Cy2^{®}, Cy3^{®}, Cy3.5^{®}, Cy5^{®}, Cy5.5^{®}, Cy7^{®}, Cychrome (PE-Cy5), Dansylamine, Dansyl cadaverine, Dansylchloride, DAPI, Dapoxyl, DCFH, DHR, DiA (4-Di-16-ASP), DiD (DilC18(5)), DIDS, Dil (DilC18(3)), DiO (DiOC18(3)), DiR (DilC18(7)), Di-4 ANEPPS, Di-8 ANEPPS, DM-NERF (4.5-6.5 pH), DsRed (Red Fluorescent Protein), EBFP, ECFP, EGFP, ELF^{®} -97 alcohol, Eosin, Erythrosin, Ethidium bromide, Ethidium homodimer-1 (EthD-1), Europium (III) Chloride, 5-FAM (5-Carboxyfluorescein), Fast Blue, Fluorescein-dT phosphoramidite, FITC, Fluo-3, Fluo-4, FluorX^{®}, Fluoro-Gold^{™} (high pH), Fluoro-Gold^{™} (low pH), Fluoro-Jade, FM^{®} 1-43, Fura-2 (high calcium), Fura-2 / BCECF, Fura Red^{™} (high calcium), Fura Red^{™} / Fluo-3, GeneBLAzer^{™} (CCF2), GFP Red Shifted (rsGFP), GFP Wild Type, GFP / BFP FRET, GFP / DsRed FRET, Hoechst 33342 & 33258, 7-Hydroxy-4-methylcoumarin (pH 9), 1,5 IAEDANS, Indo-1 (high calcium), Indo-1 (low calcium), Indodicarbocyanine, Indotricarbocyanine, JC-1, 6-JOE, JOJO^{™}-1 / JO-PRO^{™}-1, LDS 751 (+DNA), LDS 751 (+RNA), LOLO^{™}-1 / LO-PRO^{™}-1, Lucifer Yellow, LysoSensor^{™} Blue (pH 5), LysoSensor^{™} Green (pH 5), LysoSensor^{™} Yellow/Blue (pH 4.2), LysoTracker^{®} Green, LysoTracker^{®} Red, LysoTracker^{®} Yellow, Mag-Fura-2, Mag-Indo-1, Magnesium Green^{™}, Marina Blue^{®}, 4-Methylumbelliferone, Mithramycin, MitoTracker^{®} Green, MitoTracker^{®} Orange, MitoTracker^{®} Red, NBD (amine), Nile Red, Oregon Green^{®} 488, Oregon Green^{®} 500, Oregon Green^{®} 514, Pacific Blue, PBF1, PE (R-phycoerythrin), PE-Cy5, PE-Cy7, PE-Texas Red, PerCP (Peridinin chlorphyll protein), PerCP-Cy5.5 (TruRed), PharRed (APC-Cy7), C-phycocyanin, R-phycocyanin, R-phycoerythrin (PE), PI (Propidium Iodide), PKH26, PKH67, POPO^{™}-1 / PO-PRO^{™}-1, POPO^{™}-3 / PO-PRO^{™}-3, Propidium Iodide (PI), PyMPO, Pyrene, Pyronin Y, Quantam Red (PE-Cy5), Quinacrine Mustard, R670 (PE-Cy5), Red 613 (PE-Texas Red) , Red Fluorescent Protein (DsRed), Resorufin, RH 414, Rhod-2, Rhodamine B, Rhodamine Green^{™}, Rhodamine Red^{™}, Rhodamine Phalloidin, Rhodamine 110, Rhodamine 123, 5-ROX (carboxy-X-rhodamine), S65A, S65C, S65L, S65T, SBFI, SITS, SNAFL^{®}-1 (high pH), SNAFL^{®}-2, SNARF^{®}-1 (high pH), SNARF^{®}-1 (low pH), Sodium Green^{™}, SpectrumAqua^{®}, SpectrumGreen^{®} #1, SpectrumGreen^{®} #2, SpectrumOrange^{®}, SpectrumRed^{®}, SYTO^{®} 11, SYTO^{®} 13, SYTO^{®} 17, SYTO^{®} 45, SYTOX^{®} Blue, SYTOX^{®} Green, SYTOX^{®} Orange, 5-TAMRA (5-Carboxytetramethylrhodamine), Tetramethylrhodamine (TRITC), Texas Red^{®} / Texas Red^{®}-X, Texas Red^{®}-X (NHS Ester), Thiadicarbocyanine, Thiazole Orange, TOTOO-1 / TO-PRO^{®}-1, TOTO^{®}-3 / TO-PRO^{®}-3, TO-PRO^{®}-5, Tri-color (PE-Cy5), TRITC (Tetramethylrhodamine), TruRed (PerCP-Cy5.5), WW 781, X-Rhodamine (XRITC), Y66F, Y66H, Y66W, YFP (Yellow Fluorescent Protein), YOYO^{®}-1 / YO-PRO^{®}-1, YOYO^{®}-3 / YO-PRO^{®}-3, 6-FAM (Fluorescein), 6-FAM (NHS Ester), 6-FAM (Azide), HEX, TAMRA (NHS Ester), Yakima Yellow, MAX, TET, TEX615, ATTO 488, ATTO 532, ATTO 550, ATTO 565, ATTO Rho101, ATTO 590, ATTO 633, ATTO 647N, TYE 563, TYE 665, TYE 705, 5' IRDye^{®} 700, 5' IRDye^{®} 800, 5' IRDye^{®} 800CW (NHS Ester), WellRED D4 Dye, WellRED D3 Dye, WellRED D2 Dye, Lightcycler^{®} 640 (NHS Ester), and Dy 750 (NHS Ester).

As mentioned above, in some embodiments, a detectable label is or includes a luminescent or chemiluminescent moiety. Common luminescent/chemiluminescent moieties include, but are not limited to, peroxidases such as horseradish peroxidase (HRP), soybean peroxidase (SP), alkaline phosphatase, and luciferase. These protein moieties can catalyze chemiluminescent reactions given the appropriate substrates (e.g., an oxidizing reagent plus a chemiluminescent compound. A number of compound families provide chemiluminescence under a variety of conditions. Non-limiting examples of chemiluminescent compound families include 2,3-dihydro-1,4-phthalazinedione luminol, 5-amino-6,7,8-trimethoxy- and the dimethylamino[ca]benz analog. These compounds can luminesce in the presence of alkaline hydrogen peroxide or calcium hypochlorite and base. Other examples of chemiluminescent compound families include, e.g., 2,4,5-triphenylimidazoles, para-dimethylamino and - methoxy substituents, oxalates such as oxalyl active esters, p-nitrophenyl, N-alkyl acridinum esters, luciferins, lucigenins, or acridinium esters. In some embodiments, a detectable label is or includes a metal-based or mass-based label. For example, small cluster metal ions, metals, or semiconductors may act as a mass code. In some examples, the metals can be selected from Groups 3-15 of the periodic table, e.g., Y, La, Ag, Au, Pt, Ni, Pd, Rh, Ir, Co, Cu, Bi, or a combination thereof.

In some embodiments, hybridization chain reaction (HCR) can be used for *in situ* signal amplification and detection. HCR is an enzyme-free nucleic acid amplification based on a triggered chain of hybridization of nucleic acid molecules starting from HCR monomers, which hybridize to one another to form a nicked nucleic acid polymer. This polymer is the product of the HCR reaction which is ultimately detected in order to indicate the presence of the target reporter oligonucleotide. HCR is described in detail in Dirks and Pierce, 2004, PNAS, 101(43), 15275-15278 and in US 7,632,641 and US 7,721,721 (see also US 2006/00234261; Chemeris et al, 2008 Doklady Biochemistry and Biophysics, 419, 53-55; Niu et al, 2010, 46, 3089-3091; Choi et al, 2010, Nat. Biotechnol. 28(11), 1208-1212; and Song et al, 2012, Analyst, 137, 1396-1401). HCR monomers typically comprise a hairpin, or other metastable nucleic acid structure. In the simplest form of HCR, two different types of stable hairpin monomer, referred to here as first and second HCR monomers, undergo a chain reaction of hybridization events to form a long nicked double-stranded DNA molecule when an "initiator" nucleic acid molecule is introduced. The HCR monomers have a hairpin structure comprising a double stranded stem region, a loop region connecting the two strands of the stem region, and a single stranded region at one end of the double stranded stem region. The single stranded region which is exposed (and which is thus available for hybridization to another molecule, e.g. initiator or other HCR monomer) when the monomers are in the hairpin structure may be known as the "toehold region" (or "input domain"). The first HCR monomers each further comprise a sequence which is complementary to a sequence in the exposed toehold region of the second HCR monomers. This sequence of complementarity in the first HCR monomers may be known as the "interacting region" (or "output domain"). Similarly, the second HCR monomers each comprise an interacting region (output domain), e.g. a sequence which is complementary to the exposed toehold region (input domain) of the first HCR monomers. In the absence of the HCR initiator, these interacting regions are protected by the secondary structure (e.g. they are not exposed), and thus the hairpin monomers are stable or kinetically trapped (also referred to as "metastable"), and remain as monomers (e.g. preventing the system from rapidly equilibrating), because the first and second sets of HCR monomers cannot hybridize to each other. However, once the initiator is introduced, it is able to hybridize to the exposed toehold region of a first HCR monomer, and invade it, causing it to open up. This exposes the interacting region of the first HCR monomer (e.g. the sequence of complementarity to the toehold region of the second HCR monomers), allowing it to hybridize to and invade a second HCR monomer at the toehold region. This hybridization and invasion in turn opens up the second HCR monomer, exposing its interacting region (which is complementary to the toehold region of the first HCR monomers), and allowing it to hybridize to and invade another first HCR monomer. The reaction continues in this manner until all of the HCR monomers are exhausted (e.g. all of the HCR monomers are incorporated into a polymeric chain). Ultimately, this chain reaction leads to the formation of a nicked chain of alternating units of the first and second monomer species. The presence of the HCR initiator is thus required in order to trigger the HCR reaction by hybridization to and invasion of a first HCR monomer. The first and second HCR monomers are designed to hybridize to one another are thus may be defined as cognate to one another. They are also cognate to a given HCR initiator sequence. HCR monomers which interact with one another (hybridize) may be described as a set of HCR monomers or an HCR monomer, or hairpin, system.

An HCR reaction could be carried out with more than two species or types of HCR monomers. For example, a system involving three HCR monomers could be used. In such a system, each first HCR monomer may comprise an interacting region which binds to the toehold region of a second HCR monomer; each second HCR may comprise an interacting region which binds to the toehold region of a third HCR monomer; and each third HCR monomer may comprise an interacting region which binds to the toehold region of a first HCR monomer. The HCR polymerization reaction would then proceed as described above, except that the resulting product would be a polymer having a repeating unit of first, second and third monomers consecutively. Corresponding systems with larger numbers of sets of HCR monomers could readily be conceived.

### (b) Analysis

A target sequence for a probe or probe set disclosed herein may be comprised in any analyte disclose herein, including an endogenous analyte (e.g., a probe or probe set that hybridizes directly or indirectly to the RNA), a labelling agent, or a product or derivative of an endogenous analyte and/or a labelling agent.

In some aspects, one or more of the target sequences includes one or more barcode(s), e.g., at least two, three, four, five, six, seven, eight, nine, ten, or more barcodes. Barcodes can spatially-resolve molecular components found in biological samples, for example, within a cell or a tissue sample. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI"). In some aspects, a barcode comprises about 4, 5, 6, 7, 8, 9, 10, 11, 12, 13, 14, 15, 16, 17, 18, 19, 20, 21, 22, 23, 24, 25, 26, 27, 28, 29, 30, or more than 30 nucleotides.

In some embodiments, a barcode includes two or more sub-barcodes that together function as a single barcode. For example, a polynucleotide barcode can include two or more polynucleotide sequences (e.g., sub-barcodes) that are separated by one or more non-barcode sequences. In some embodiments, the teow or more sub-barcodes can be overlapping sequences. In some embodiments, the one or more barcode(s) can also provide a platform for targeting functionalities, such as oligonucleotides, oligonucleotide-antibody conjugates, oligonucleotide-streptavidin conjugates, modified oligonucleotides, affinity purification, detectable moieties, enzymes, enzymes for detection assays or other functionalities, and/or for detection and identification of the polynucleotide.

In any of the preceding embodiments, barcodes (e.g., primary and/or secondary barcode sequences) can be analyzed (e.g., detected or sequenced) using any suitable methods or techniques, including those described herein, such as RNA sequential probing of targets (RNA SPOTs), sequential fluorescent *in situ* hybridization (seqFISH), single-molecule fluorescent *in situ* hybridization (smFISH), multiplexed error-robust fluorescence *in situ* hybridization (MERFISH), *in situ* sequencing, hybridization-based *in situ* sequencing (HybISS), targeted *in situ* sequencing, fluorescent *in situ* sequencing (FISSEQ), sequencing by synthesis (SBS), sequencing by ligation (SBL), sequencing by hybridization (SBH), or spatially-resolved transcript amplicon readout mapping (STARmap). In any of the preceding embodiments, the methods provided herein can include analyzing the barcodes by sequential hybridization and detection with a plurality of labelled probes (e.g., detection oligos).

In some embodiments, in a barcode sequencing method, barcode sequences are detected for identification of other molecules including nucleic acid molecules (DNA or RNA) longer than the barcode sequences themselves, as opposed to direct sequencing of the longer nucleic acid molecules. In some embodiments, a N-mer barcode sequence comprises 4^{N} complexity given a sequencing read of N bases, and a much shorter sequencing read may be required for molecular identification compared to non-barcode sequencing methods such as direct sequencing. For example, 1024 molecular species may be identified using a 5-nucleotide barcode sequence (4⁵=1024), whereas 8 nucleotide barcodes can be used to identify up to 65,536 molecular species, a number greater than the total number of distinct genes in the human genome. In some embodiments, the barcode sequences rather than endogenous sequences, which can be an efficient read-out in terms of information per cycle of sequencing. Because the barcode sequences are pre-determined, they can also be designed to feature error detection and correction mechanisms, see, e.g., U.S. Pat. Pub. 20190055594 and US 2021/0164039, which are hereby incorporated by reference in their entirety.

In some embodiments, detection of the barcode sequences is performed by sequential hybridization of probes to the barcode sequences or complements thereof and detecting complexes formed by the probes and barcode sequences or complements thereof. In some cases, each barcode sequence or complement thereof is assigned a sequence of signal codes that identifies the barcode sequence or complement thereof (e.g., a temporal signal signature or code that identifies the analyte), and detecting the barcode sequences or complements thereof can comprise decoding the barcode sequences of complements thereof by detecting the corresponding sequences of signal codes detected from sequential hybridization, detection, and removal of sequential pools of intermediate probes and the universal pool of detectably labeled probes. In some cases, the sequences of signal codes can be fluorophore sequences assigned to the corresponding barcode sequences or complements thereof. In some embodiments, the detectably labeled probes are fluorescently labeled. In some embodiments, the barcode sequence or complement thereof is performed by sequential probe hybridization as described in US 2021/0340618, the content of which is herein incorporated by reference in its entirety.

In any of the embodiments herein, the detecting step can comprise contacting the biological sample with one or more detectably labeled probes that directly or indirectly hybridize to the barcode sequences or complements thereof (e.g., in amplification products generated using the probes or probe sets), and dehybridizing the one or more detectably labeled probes. In any of the embodiments herein, the contacting and dehybridizing steps can be repeated with the one or more detectably labeled probes and/or one or more other detectably labeled probes that directly or indirectly hybridize to the barcode sequences or complements thereof. In some aspects, the method comprises sequential hybridization of detectably labeled probes to create a spatiotemporal signal signature or code that identifies the analyte. In some aspects, the methods involving enzymatic and chemical reactions described herein (e.g., provided in Section II) can preserve the sample and maintain the spatial localization of the analytes through the repeated steps of probe hybridization and removal during the detecting step.

In any of the embodiments herein, the detecting step can comprise contacting the biological sample with one or more first detectably labeled probes that directly hybridize to the plurality of probes or probe sets. In some instances, the detecting step can comprise contacting the biological sample with one or more first detectably labeled probes that indirectly hybridize to the plurality of probes or probe sets. In any of the embodiments herein, the detecting step can comprise contacting the biological sample with one or more first detectably labeled probes that directly or indirectly hybridize to the plurality of probes or probe sets.

In any of the embodiments herein, the detecting step can comprise contacting the biological sample with one or more intermediate probes that directly or indirectly hybridize to the barcode sequences or complements thereof (e.g., of the plurality of probes or probe sets or rolling circle amplification product generated using the plurality of probes or probe sets), wherein the one or more intermediate probes are detectable using one or more detectably labeled probes.

In any of the embodiments herein, the detecting step can further comprise removing the signal(s) of the one or more hybridized detectably labeled probes (e.g., by quenching, cleaving the label, and/or performing one or more washes). In any of the embodiments herein, the detecting step can further comprise dehybridizing the one or more intermediate probes and/or the one or more detectably labeled probes from the barcode sequences or complements thereof (e.g., of the plurality of probes or probe sets or rolling circle amplification product generated using the plurality of probes or probe sets). In any of the embodiments herein, the contacting and dehybridizing steps can be repeated with the one or more intermediate probes, the one or more detectably labeled probes, one or more other intermediate probes, and/or one or more other detectably labeled probes.

In some embodiments, sequence detection can be performed using single molecule sequencing by ligation. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. Aspects and features involved in sequencing by ligation are described, for example, in Shendure et al. Science (2005), 309: 1728-1732, and in US 5,599,675; US 5,750,341; US 6,969,488; US 6,172,218; US and 6,306,597, all of which are herein incorporated by reference in their entireties.

In some embodiments, nucleic acid hybridization can be used for detecting the analytes. These methods utilize labeled nucleic acid probes that are complementary to at least a portion of a barcode sequence. Multiplex decoding can be performed with pools of many different probes with distinguishable labels. Non-limiting examples of nucleic acid hybridization sequencing are described for example in US 8,460,865, and in Gunderson et al., Genome Research 14:870-877 (2004), all of which are herein incorporated by reference in their entireties.

In some embodiments, a probe or probe set can be a probe comprising a 3' or 5' overhang upon hybridization to the target nucleic acid (e.g., an L-shaped intermediate probe). In some embodiments, the overhang comprises one or more barcode sequences corresponding to the target nucleic acid (e.g., the target RNA transcript). In some embodiments, a plurality of probes are designed to hybridize to the target nucleic acid (e.g., at least 20, 30, or 40 probes can hybridize to the target nucleic acid). In some embodiments, the probe or probe set is a probe comprising a 3' overhang and a 5' overhang upon hybridization to the target nucleic acid (a U-shaped probe). In some embodiments, the 3' overhang and the 5' overhang each independently comprises one or more detectable labels and/or barcode sequences. In some embodiments, the 3' and/or 5' overhang comprises one or more detectable labels and/or barcode sequences.

In some embodiments, analysis comprises using a codebook comprising signal code sequence that are sequences of color codes, arranged in the order of the corresponding signal color detected in sequential cycles of probe hybridization and imaging. In some aspects, the provided methods which immobilize and tether RNA in the biological sample can be advantageous when using detection methods that comprise a plurality of repeated cycles of hybridization and removal of probes (e.g., detectably labeled probes, or intermediate probes that bind to detectably labeled probes) to the primary probe or probe set hybridized to the RNA, or to a rolling circle amplification product generated from the probe or probe set hybridized to the RNA.

### IV. Kits

In some aspects, provided herein is a kit for analyzing fragmented ribonucleic acid (RNA) in a biological sample, comprising: (a) an attachment agent comprising a boronic acid moiety capable of covalently reacting with at least one 2',3' vicinal diol of the RNA and an attachment moiety capable of attaching covalently or noncovalently to an exogenous or endogenous molecule in the biological sample; (b) a matrix-forming agent; (c) a polymerization-inducing catalyst or functional cross linker; and (d) instructions for use. In some embodiments, any one or more of the attachment agent, the matrix-forming agent, and the polymerization-inducing catalyst or functional cross linker can be premixed, stored, and/or shipped in one or more containers such as vials. In some embodiments, any one or more of the attachment agent, the matrix-forming agent, and the polymerization-inducing catalyst or functional cross linker can be provided separately. For instance, each of the attachment agent, the matrix-forming agent, and the polymerization-inducing catalyst or functional cross linker can be provided, stored, and/or shipped in a separate container, although any two or more of the separate containers can be packaged together.

In other aspects, provided herein is a kit for analyzing fragmented ribonucleic acid (RNA) in a biological sample. In some embodiments, provided herein is a kit for analyzing RNA in a biological sample embedded in a three-dimensional polymerized matrix according to any of the methods described herein. In some embodiments, the kit comprises (a) a matrix-forming agent, wherein the matrix forming agent comprises a boronic acid moiety capable of covalently reacting with at least one 2',3' vicinal diol of the RNA; (b) a polymerization-inducing catalyst or functional cross linker; and (c) instructions for use. In some embodiments, any one or more of matrix-forming agent and the polymerization-inducing catalyst or functional cross linker can be premixed, stored, and/or shipped in one or more containers such as vials. In some embodiments, any one or more of the matrix-forming agent and the polymerization-inducing catalyst or functional cross linker can be provided separately. For instance, each of the matrix-forming agent and the polymerization-inducing catalyst or functional cross linker can be provided, stored, and/or shipped in a separate container, although any two or more of the separate containers can be packaged together.

In some embodiments, hydrogel forming monomers (e.g., bis acrylamide, acrylamide, and/or boronic acid acrylamide) can be provided in a single container that would be shipped and ready to polymerize. In some embodiments, some hydrogel forming monomers can be provided separately from the attachment agent comprising a boronic acid moiety, and the attachment agent can be provided (e.g., in a drop) and mixed with hydrogel forming monomers before forming the hydrogel.

In some embodiments, the kit further comprises a 3' phosphatase. In some embodiments, the 3' phosphatase is T4 polynucleotide kinase. In some embodiments, the kit further comprises a clearing agent. In some embodiments, the clearing agent is a detergent, a lipase, a protease, and/or a deglycosylase.

In some embodiments, the kit further comprises a probe or probe set designed to hybridize to a fragmented nucleic acid. In some aspects, the probe or probe set that binds directly or indirectly to the RNA is provided in a separate kit. In some cases, further provided is a kit comprising reagents for detecting the probe or probe set.

The various components of the kit may be present in separate containers or certain compatible components may be pre-combined into a single container. In some embodiments, the kits further contain instructions for using the components of the kit to practice the provided methods.

In some embodiments, the kits can contain reagents and/or consumables required for performing one or more steps of the provided methods. In some embodiments, the kits contain reagents for fixing, embedding, and/or permeabilizing the biological sample. In some embodiments, the kits contain reagents, such as enzymes and buffers for ligation and/or amplification, such as ligases and/or polymerases. In some aspects, provided is a kit that comprises any of the reagents described herein, e.g., wash buffer and ligation buffer. In some embodiments, provided is a kit that contain reagents for detection and/or sequencing, such as barcode detection probes or detectable labels. In some embodiments, the kits optionally contain other components, for example nucleic acid primers, enzymes and reagents, buffers, nucleotides, modified nucleotides, reagents for additional assays.

### V. OPTO-FLUIDIC INSTRUMENTS FOR ANALYSIS OF BIOLOGICAL SAMPLES

Provided herein is an instrument having integrated optics and fluidics modules (an "opto-fluidic instrument" or "opto-fluidic system") for detecting target molecules (*e.g.*, nucleic acids, proteins, antibodies, *etc.)* in biological samples *(e.g.,* one or more cells or a tissue sample) as described herein. In an opto-fluidic instrument, the fluidics module is configured to deliver one or more reagents (*e.g*., detectably labeled probes) to the biological sample and/or remove spent reagents therefrom. Additionally, the optics module is configured to illuminate the biological sample with light having one or more spectral emission curves (over a range of wavelengths) and subsequently capture one or more images of emitted light signals from the biological sample during one or more probing cycles (e.g., as described in Section III). In some embodiments, tethering RNAs using attachment agents described herein (e.g., as described in Section II) can help with cyclic assays where there are rounds of probe hybridization, unhybridization (e.g., by washing), and rehybridization, such that the assay (e.g., an *in situ* assay) can be performed using an automated instrument or system, e.g., an opto-fluidic instrument or system disclosed herein.

In various embodiments, the captured images may be processed in real time and/or at a later time to determine the presence of the one or more target molecules in the biological sample, as well as three-dimensional position information associated with each detected target molecule. Additionally, the opto-fluidics instrument includes a sample module configured to receive (and, optionally, secure) one or more biological samples. In some instances, the sample module includes an X-Y stage configured to move the biological sample along an X-Y plane (e.g., perpendicular to an objective lens of the optics module).

In various embodiments, the opto-fluidic instrument is configured to analyze one or more target molecules (e.g., any of the analytes described in Section III) in their naturally occurring place *(i.e., in situ)* within the biological sample. For example, an opto-fluidic instrument may be an *in-situ* analysis system used to analyze a biological sample and detect target molecules (e.g., analytes) including but not limited to DNA, RNA, proteins, antibodies, and/or the like.

It is to be noted that, although the above discussion relates to an opto-fluidic instrument that can be used for *in situ* target molecule detection via probe hybridization, the discussion herein equally applies to any opto-fluidic instrument that employs any imaging or target molecule detection technique. That is, for example, an opto-fluidic instrument may include a fluidics module that includes fluids needed for establishing the experimental conditions required for the probing of target molecules in the sample. Further, such an opto-fluidic instrument may also include a sample module configured to receive the sample, and an optics module including an imaging system for illuminating (*e.g*., exciting one or more fluorescent probes within the sample) and/or imaging light signals received from the probed sample. The *in-situ* analysis system may also include other ancillary modules configured to facilitate the operation of the opto-fluidic instrument, such as, but not limited to, cooling systems, motion calibration systems, etc.

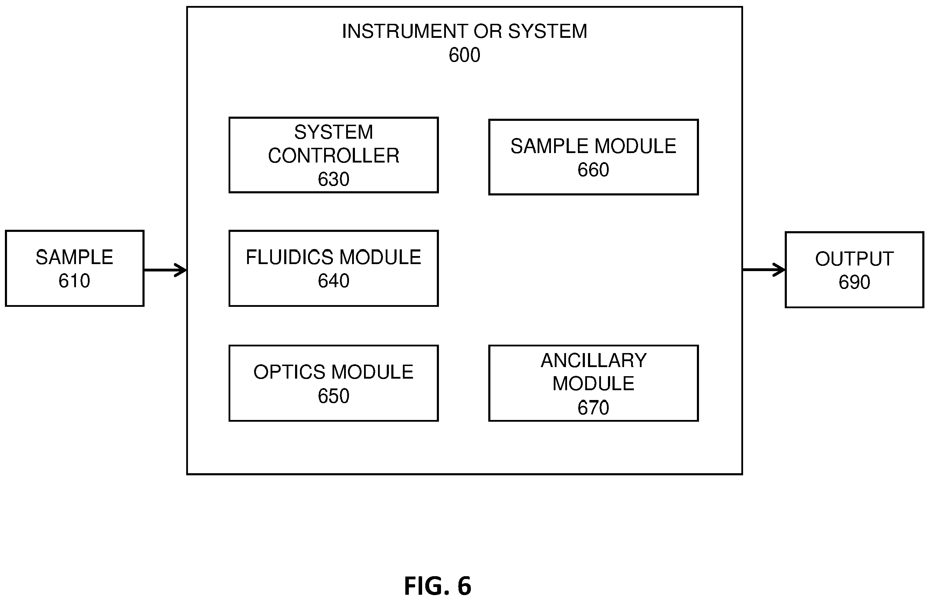

**FIG. 6** shows an example workflow of analysis of a biological sample 610 *(e.g.,* cell or tissue sample) using an opto-fluidic instrument or system 600, according to various embodiments. In various embodiments, the sample 610 can be a biological sample (e.g., a tissue) that includes molecules such as DNA, RNA, proteins, antibodies, etc. For example, the sample 610 can be a sectioned tissue that is treated to access the RNA thereof for tethering using attachment agents described herein (e.g., as described in Section II) and labeling with probes described herein (e.g., in Section III). Ligation of the probes may generate a circular probe which can be enzymatically amplified and bound with detectably labeled probes, which can create bright signal that is convenient to image and has a high signal-to-noise ratio.

In various embodiments, the sample 610 may be placed in the opto-fluidic instrument or system 600 for analysis and detection of the molecules in the sample 610. In various embodiments, the opto-fluidic instrument or system 600 can be a system configured to facilitate the experimental conditions conducive for the detection of the target molecules. For example, the opto-fluidic instrument or system 600 can include a fluidics module 640, an optics module 650, a sample module 660, and an ancillary module 670, and these modules may be operated by a system controller 630 to create the experimental conditions for the probing of the molecules in the sample 610 by selected probes (e.g., circularizable DNA probes), as well as to facilitate the imaging of the probed sample (e.g., by an imaging system of the optics module 650). In various embodiments, the various modules of the opto-fluidic instrument or system 600 may be separate components in communication with each other, or at least some of them may be integrated together.

In various embodiments, the sample module 660 may be configured to receive the sample 610 into the opto-fluidic instrument or system 600. For instance, the sample module 660 may include a sample interface module (SIM) that is configured to receive a sample device (e.g., cassette) onto which the sample 610 can be deposited. That is, the sample 610 may be placed in the opto-fluidic instrument or system 600 by depositing the sample 610 (e.g., the sectioned tissue) on a sample device that is then inserted into the SIM of the sample module 660. In some instances, the sample module 660 may also include an X-Y stage onto which the SIM is mounted. The X-Y stage may be configured to move the SIM mounted thereon (e.g., and as such the sample device containing the sample 610 inserted therein) in perpendicular directions along the two-dimensional (2D) plane of the opto-fluidic instrument or system 600.

The experimental conditions that are conducive for the detection of the molecules in the sample 610 may depend on the target molecule detection technique that is employed by the opto-fluidic instrument or system 600. For example, in various embodiments, the opto-fluidic instrument or system 600 can be a system that is configured to detect molecules in the sample 610 via hybridization of probes. In such cases, the experimental conditions can include molecule hybridization conditions that result in the intensity of hybridization of the target molecule (e.g., nucleic acid) to a probe (e.g., oligonucleotide) being significantly higher when the probe sequence is complementary to the target molecule than when there is a single-base mismatch. The hybridization conditions include the preparation of the sample 610 using reagents such as washing/stripping reagents, hybridizing reagents, etc., and such reagents may be provided by the fluidics module 640.

In various embodiments, the fluidics module 640 may include one or more components that may be used for storing the reagents, as well as for transporting said reagents to and from the sample device containing the sample 610. For example, the fluidics module 640 may include reservoirs configured to store the reagents, as well as a waste container configured for collecting the reagents (e.g., and other waste) after use by the opto-fluidic instrument or system 600 to analyze and detect the molecules of the sample 610. Further, the fluidics module 640 may also include pumps, tubes, pipettes, etc., that are configured to facilitate the transport of the reagent to the sample device (e.g., and as such the sample 610). For instance, the fluidics module 640 may include pumps ("reagent pumps") that are configured to pump washing/stripping reagents to the sample device for use in washing/stripping the sample 610 (e.g., as well as other washing functions such as washing an objective lens of the imaging system of the optics module 650).

In various embodiments, the ancillary module 670 can be a cooling system of the opto-fluidic instrument or system 600, and the cooling system may include a network of coolant-carrying tubes that are configured to transport coolants to various modules of the opto-fluidic instrument or system 600 for regulating the temperatures thereof. In such cases, the fluidics module 640 may include coolant reservoirs for storing the coolants and pumps (e.g., "coolant pumps") for generating a pressure differential, thereby forcing the coolants to flow from the reservoirs to the various modules of the opto-fluidic instrument or system 600 via the coolant-carrying tubes. In some instances, the fluidics module 640 may include returning coolant reservoirs that may be configured to receive and store returning coolants, i.e., heated coolants flowing back into the returning coolant reservoirs after absorbing heat discharged by the various modules of the opto-fluidic instrument or system 600. In such cases, the fluidics module 640 may also include cooling fans that are configured to force air (e.g., cool and/or ambient air) into the returning coolant reservoirs to cool the heated coolants stored therein. In some instance, the fluidics module 640 may also include cooling fans that are configured to force air directly into a component of the opto-fluidic instrument or system 600 so as to cool said component. For example, the fluidics module 640 may include cooling fans that are configured to direct cool or ambient air into the system controller 630 to cool the same.

As discussed above, the opto-fluidic instrument or system 600 may include an optics module 650 which include the various optical components of the opto-fluidic instrument or system 600, such as but not limited to a camera, an illumination module (e.g., LEDs), an objective lens, and/or the like. The optics module 650 may include a fluorescence imaging system that is configured to image the fluorescence emitted by the probes (e.g., oligonucleotides) in the sample 610 after the probes are excited by light from the illumination module of the optics module 650.

In some instances, the optics module 650 may also include an optical frame onto which the camera, the illumination module, and/or the X-Y stage of the sample module 660 may be mounted.

In various embodiments, the system controller 630 may be configured to control the operations of the opto-fluidic instrument or system 600 (e.g., and the operations of one or more modules thereof). In some instances, the system controller 630 may take various forms, including a processor, a single computer (or computer system), or multiple computers in communication with each other. In various embodiments, the system controller 630 may be communicatively coupled with data storage, set of input devices, display system, or a combination thereof. In some cases, some or all of these components may be considered to be part of or otherwise integrated with the system controller 630, may be separate components in communication with each other, or may be integrated together. In other examples, the system controller 630 can be, or may be in communication with, a cloud computing platform.

In various embodiments, the opto-fluidic instrument or system 600 may analyze the sample 610 and may generate the output 690 that includes indications of the presence of the target molecules in the sample 610. For instance, with respect to the example embodiment discussed above where the opto-fluidic instrument or system 600 employs a hybridization technique for detecting molecules, the opto-fluidic instrument or system 600 may cause the sample 610 to undergo successive rounds of detectably labeled probe hybridization (e.g., using two or more sets of fluorescent probes, where each set of fluorescent probes is excited by a different color channel) and be imaged to detect target molecules in the probed sample 610. In such cases, the output 690 may include optical signatures (*e.g*., a codeword) specific to each gene, which allow the identification of the target molecules.

### VI. Terminology

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polynucleotide," "polynucleotide," and "nucleic acid molecule", used interchangeably herein, refer to polymeric forms of nucleotides of any length, either ribonucleotides or deoxyribonucleotides. Thus, this term comprises, but is not limited to, single-, double-, or multi- stranded DNA or RNA, genomic DNA, cDNA, DNA-RNA hybrids, or a polymer comprising purine and pyrimidine bases or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups.

A "primer" used herein can be an oligonucleotide, either natural or synthetic, that is capable, upon forming a duplex with a polynucleotide template, of acting as a point of initiation of nucleic acid synthesis and being extended from its 3' end along the template so that an extended duplex is formed. The sequence of nucleotides added during the extension process is determined by the sequence of the template polynucleotide. Primers usually are extended by a DNA polymerase.

"Ligation" may refer to the formation of a covalent bond or linkage between the termini of two or more nucleic acids, e.g., oligonucleotides and/or polynucleotides, in a template-driven reaction. The nature of the bond or linkage may vary widely and the ligation may be carried out enzymatically or chemically. As used herein, ligations are usually carried out enzymatically to form a phosphodiester linkage between a 5' carbon terminal nucleotide of one oligonucleotide with a 3' carbon of another nucleotide.

The term "about" as used herein refers to the usual error range for the respective value readily known to the skilled person in this technical field. Reference to "about" a value or parameter herein comprises (and describes) embodiments that are directed to that value or parameter per se.

As used herein, the singular forms "a," "an," and "the" comprise plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

### EXAMPLES

The following examples are included for illustrative purposes only and are not intended to limit the scope of the present disclosure.

### Example 1: Immobilizing Fragmented RNA

This example describes the immobilization of the RNA contained in formalin-fixed paraffin-embedded (FFPE) mouse brain (mBrain) tissue samples and subsequent embedding of the FFPE mBrain samples in a hydrogel matrix for imaging. FFPE tissue samples are known to contain degraded RNA due to the extensive processing, including baking, deparaffinization, decrosslinking and permeabilization that these samples undergo in order to facilitate analysis and imaging. In some cases, a significant amount of fragmented RNA can be lost during sample processing such as during decrosslinking of the sample, thus limiting the amount of RNA material present for imaging.

**Sample Preparation:** First, FFPE mBrain samples are baked at 60°C for 2 hr and deparaffinized via (a) xylene treatment-incubation in 100% xylene twice for (2x) 10 min each, followed by (b) serial ethanol treatment-incubation with 100% ethanol (2x) 3 min each, then 96% ethanol 2x 3 min each, then 70% ethanol once (1x) 3 min, followed by brief immersion in nuclease-free water (1x 20 sec). The FFPE mBrain samples are not de-crosslinked or permeabilized. Next, the samples are treated with T4 polynucleotide kinase (T4 PNK) for 1 hr at 37 °C followed by a 30 min incubation at 4°C with 5 mM of the attachment agent having a boronic acid moiety provided herein at basic pH.

**Sample Embedding:** FFPE mBrain samples are then embedded by adding Acrylamide 4%, Bisacrylamide 0.2%, and SSC 2x to the samples. A control sample is incubated similarly with Acrylamide 4%, Bisacrylamide 0.2%, and SSC 2x, without prior treatment with the attachment agent. Samples are incubated with acrylamide, bisacrylamide and SSC 2x for 15 min followed by addition of a polymerization mixture comprising 0.2% ammonium persulphate and 0.2% tetramethylethylenediamine. Samples are allowed to polymerize for 30 min at RT. Following sample embedding, mBrain FFPE samples are cleared for 1 hr at 37°C with 0.2 mg/mL proteinase K (PK) resuspended in PBS.

The embedded samples are contacted with a panel of probes targeting 50 mouse brain target genes, and reagents for ligation and amplification. Eight cycles of detection are performed to the barcode sequences associated with the probes. For a cycle of detection, a pool of intermediate probes targeting RCPs of the different genes are added and incubated, and fluorescently detected oligonucleotides are added to detect the intermediate probes. Different pools of intermediate probes are cycled and a universal pool of fluorescently labeled oligonucleotides are used to detect the intermediate probes in each cycle.

Through the sequential hybridization and detection cycles, fluorescent signals from the RCPs are detected and recorded at locations in the biological sample. The order of signals (or absence thereof) at a given location through the multiple cycles provided a signal code sequence for the RCP at the location, and the signal code sequence is compared to those in the codebook to identify a corresponding barcode sequence in the RCP and the gene associated therewith.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### NUMBERED ASPECTS OF THE INVENTION

The following numbered aspects of the invention form part of the present disclosure.
1. A method, comprising:
   (a) contacting a biological sample comprising a ribonucleic acid (RNA) with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3' vicinal diol of the RNA;
   (b) contacting the biological sample with a matrix forming agent; and
   (c) forming a three-dimensional polymerized matrix from the matrix-forming agent, thereby embedding the biological sample and immobilizing the RNA in the three-dimensional polymerized matrix.
2. The method of aspect 1, wherein the attachment moiety is capable of attaching covalently to the matrix-forming agent in the biological sample.
3. The method of aspect 2, wherein the attachment moiety is or comprises an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety.
4. The method of aspect 2, wherein the attachment moiety is or comprises a click functional group.
5. The method of any one of aspects 2-4, wherein the step of contacting the biological sample and attachment agent further comprises contacting the biological sample with one or more reagents under suitable conditions to facilitate the formation of a covalent bond between the attachment moiety of the attachment agent and an exogenous or endogenous molecule in the biological sample.
6. The method of aspect 1, wherein the attachment moiety is capable of attaching non-covalently to the matrix-forming agent in the biological sample.
7. The method of aspect 6, wherein the attachment moiety is biotin.
8. The method of any one of aspects 1-7, wherein the attachment moiety is a nonaromatic compound.
9. The method of any one of aspects 1-8, wherein the attachment agent is a compound of formula (I) 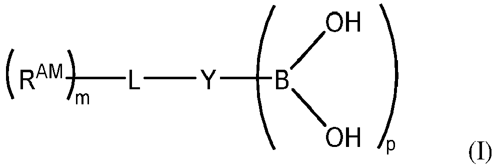 or a salt thereof, wherein
   - each R^{AM}: is independently an attachment moiety;
   - L: is a bond or linker moiety;
   - Y: is a bond, -CH₂CH₂- or -O-;
   - m: is an integer from 1 to 4; and
   - p: is an integer from 1 to 4.
10. The method of any one of aspects 1-9, wherein the attachment agent is multifunctional and comprises at least two boronic moieties or at least two attachment moieties.
11. The method of any one of aspects 1-10, wherein the attachment agent comprises at least two boronic acid moieties.
12. The method of any one of aspects 1-11, wherein the attachment agent comprises at least two attachment moieties.
13. The method of any one of aspects 1-9, wherein the attachment agent is bifunctional.
14. The method of any one of aspectaspects 1-9 and 13, wherein the attachment agent is a compound of formula (I-a) 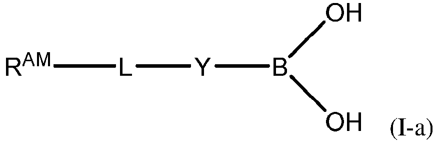 or a salt thereof, wherein
   - R^{AM}: is the attachment moiety;
   - L: is a bond or linker moiety; and
   - Y: is a bond, -CH₂CH₂-, or -O-.
15. The method of any one of aspects 9-14, wherein L is an unbranched or branched C₁-C₁₅₀ alkylene optionally interrupted by 1 to 50 heteroatoms independently selected from the group consisting of O, S and NH.
16. The method of any one of aspects 9-15, wherein L is the group 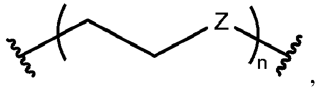 wherein
   Z is CH₂, O, S, or NH; and
   n is an integer from 0 to 50.
17. The method of any one of aspects 1 and 9-16, wherein each R^{AM} is independently an acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety.
18. The method of aspect 1 and 9-17, wherein the attachment agent is a compound of formula (II-a) 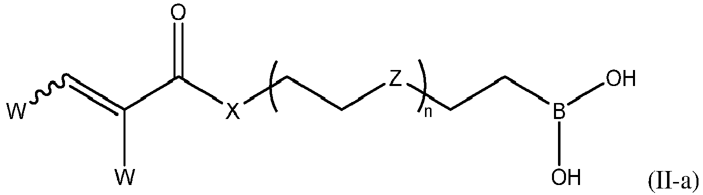 or a salt thereof, wherein
   W is H or CH₃;
   X is NH or O;
   Z is NH, O, or S; and
   n is an integer from 0 to 50.
19. The method of aspect 1 and 9-17, wherein the attachment agent is a compound of formula (I) 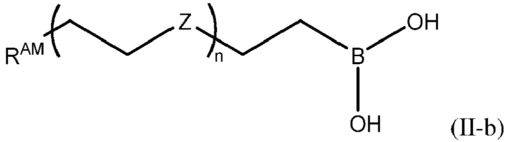 or a salt thereof, wherein
   R^{AM} is a biotinyl moiety, dextrin moiety, a click moiety, a thiol moiety, norbornenyl moiety, furanyl moiety, alkyl ester moiety, or maleimidyl moiety;
   Z is CH₂, O, S, or NH; and
   n is an integer from 0 to 50.
20. The method of any one of aspects 16-19, wherein n is 6.
21. A method, comprising:
   (a) contacting a biological sample comprising a ribonucleic acid (RNA) with a matrix-forming agent, wherein:
      (i) the RNA comprises a 2'3' vicinal diol, and
      (ii) the matrix-forming agent comprises a boronic acid moiety capable of covalently reacting with at least one 2',3' vicinal diol of the RNA, and
      wherein the biological sample and the matrix-forming agent are contacted under conditions suitable to form a covalent bond between the boronic acid moiety and the 2',3;-vicinal diol of the RNA; and
   (b) forming a three-dimensional polymerized matrix from the matrix-forming agent, thereby embedding the biological sample and immobilizing the RNA in the three-dimensional polymerized matrix.
22. The method of any one of aspects 1-21, wherein the conditions suitable to form a covalent bond between the boronic acid moiety and the 2',3'-vicinal diol of the RNA are acidic conditions, optionally wherein the acidic conditions are maintained at between pH 5 and pH 7.
23. The method of any one of aspects 1-22, wherein the RNA is a fragmented RNA.
24. The method of any one of aspects 1-23, wherein the 2',3'-vicinal diol is a fragmented 3' end of the RNA.
25. The method of any one of aspects 1-24, wherein the 2',3'-vicinal diol is generated from a fragmented RNA having a 2',3'cyclo-phosphate fragmentation at the 3'-terminal end or a 2' hydroxyl and a 3' phosphate fragmentation at the 3'-terminal end.
26. The method of any one of aspects 1-25, wherein the 2',3'-vicinal diol is provided by contacting the fragmented RNA with a 3' phosphatase.
27. The method of aspect 26, wherein the 3' phosphatase is T4 polynucleotide kinase.
28. The method of any one of aspects 1-27, wherein the biological sample is treated with a degradation agent to induce fragmentation of RNAs, optionally wherein the degradation agent is an RNase or restriction enzyme.
29. The method of any of aspects 1-28, wherein the RNA comprises a 5' cap and the 2',3'-vicinal diol is in the 5' cap.
30. The method of aspect 29, wherein the 5' cap is a 7-methylguanosine cap.
31. The method of any one of aspects 1-30, further comprising clearing the biological sample embedded in the three-dimensional polymerized matrix.
32. The method of aspect 31, wherein the biological sample is cleared with a detergent, a lipase, and/or a protease.
33. The method of aspect 32, wherein the detergent comprises a non-ionic surfactant or anionic surfactant, optionally wherein the detergent comprises SDS, tergitol, NP-40, saponin, polyethylene glycol *p*-(1,1,3,3-tetramethylbutyl)-phenyl ether, or polysorbate 20, or any combinations thereof.
34. The method of aspect 32, wherein the protease comprises proteinase K, pepsin, or collagenase, trypsin, dispase, thermolysin, or alpha-chymotrypsin, or any combinations thereof, optionally wherein the protease comprises Liberase^{™}.
35. The method of aspect 32, wherein the lipase comprises a pancreatic, hepatic and/or lysosomal lipase, or any combinations thereof, optionally wherein the lipase comprises sphingomyelinase or esterase, or a combination thereof.
36. The method of any one of aspects 31 to 35, further comprising contacting the biological sample with a probe or probe set that binds directly or indirectly to the RNA, optionally wherein the probe or probe set is a detectable probe.
37. The method of aspect 36, wherein the probe or probe set is a circular or circularizable probe or probe set, optionally wherein the method comprises circularizing the circularizable probe or probe set using the RNA or a product thereof as a template, optionally wherein the method comprises generating an RCA product using the circular or circularizable probe as a template.
38. The method of aspect 36 or 37, comprising imaging the biological sample to detect the probe or probe set or the RCA product.
39. The method of aspect 38, wherein imaging comprises detecting a signal associated with the probe or probe set or the RCA product, optionally wherein the signal is from a fluorescently labeled probe that directly or indirectly binds to the probe or probe set or the RCA product.
40. The method of any of aspects 36-39, wherein the probe or probe set comprises a barcode sequence.
41. The method of aspect 40, wherein the method comprises detecting the barcode sequence or a complement thereof in the probe or probe set or in a product of the probe or probe set.
42. A method of analyzing a fragmented ribonucleic acid (RNA) in a biological sample, the method comprising:
   (a) contacting the biological sample comprising the fragmented RNA with a 3' phosphatase to provide a fragmented RNA comprising a 2',3'-vicinal diol;
   (b) contacting the biological sample with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3;-vicinal diol of the RNA;
   (c) contacting the biological sample with a matrix-forming agent;
   (d) forming a three-dimensional polymerized matrix from the matrix-forming agent, thereby embedding the biological sample in the three-dimensional polymerized matrix and anchoring the fragmented RNA to the three-dimensional polymerized matrix;
   (e) clearing the biological sample;
   (f) contacting the biological sample with a probe or probe set that binds directly or indirectly to the RNA; and
   (g) detecting the probe or a product thereof at a location in the matrix.
43. The method of aspect 42, wherein the attachment moiety is capable of attaching covalently to the matrix-forming agent in the biological sample.
44. The method of aspect 43, wherein the attachment moiety is or comprises an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety.
45. The method of aspect 43, wherein the attachment moiety is or comprises a click functional group.
46. The method of any one of aspects 43-45, wherein the step of contacting the biological sample and attachment agent further comprises contacting the biological sample with one or more reagents or under suitable conditions to facilitate the formation of a covalent bond between the attachment moiety of the attachment agent and an exogenous or endogenous molecule in the biological sample.
47. The method of aspect 42, wherein the attachment moiety is capable of attaching non-covalently to the matrix-forming agent in the biological sample.
48. The method of aspect 47, wherein the attachment moiety is biotin.
49. The method of any one of aspects 42-48, wherein the attachment moiety is a nonaromatic compound.
50. The method of any one of aspects 42-49, wherein the attachment agent is a compound of formula (I) 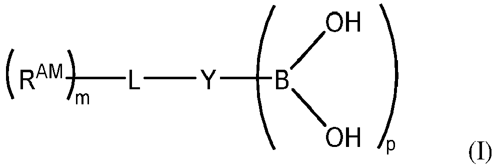 or a salt thereof, wherein
   - each R^{AM}: is independently the attachment moiety;
   - L: is a bond or linker moiety;
   - Y: is a bond, -CH₂CH₂- or -O-;
   - m: is an integer from 1 to 4; and
   - p: is an integer from 1 to 4.
51. The method of any one of aspects 42-50, wherein the attachment agent is multifunctional and comprises at least two boronic moieties or at least two attachment moieties.
52. The method of any one of aspects 42-51, wherein the attachment agent comprises at least two boronic acid moieties.
53. The method of any one of aspects 42-52, wherein the attachment agent comprises at least two attachment moieties.
54. The method of any one of aspects 42-50, wherein the attachment agent is bifunctional.
55. The method of any one of aspects 42-50 and 54, wherein the attachment agent is a compound of formula (I-a) 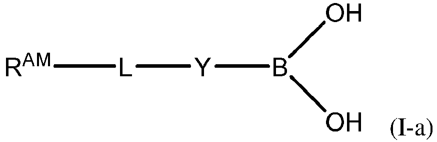 or a salt thereof, wherein
   - R^{AM}: is the attachment moiety;
   - L: is a bond or linker moiety; and
   - Y: is a bond, -CH₂CH₂- or -O-.
56. The method of any one of aspects 50-55, wherein L is an unbranched or branched C₁-C₁₅₀ alkylene optionally interrupted by 1 to 50 heteroatoms independently selected from the group consisting of O, S and NH.
57. The method of any one of aspects 50-56, wherein L is the group 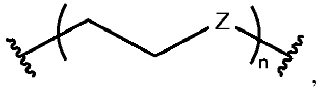 wherein
   Z is CH₂, O, S, or NH; and
   n is an integer from 0 to 50.
58. The method of any one of aspects 42 and 50-57, wherein each R^{AM} is independently acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety.
59. The method of aspect 42 and 50-58, wherein the attachment agent is a compound of formula (II-a) 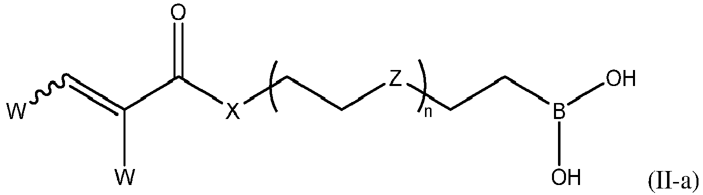 or a salt thereof, wherein
   W is H or CH₃;
   X is NH or O;
   Z is NH, O, or S; and
   n is an integer from 0 to 50.
60. The method of aspect 42 and 50-58, wherein the attachment agent is a compound of formula (I) 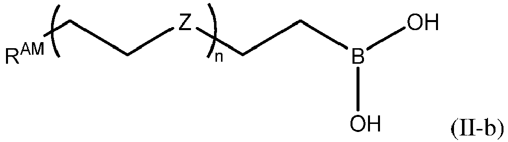 or a salt thereof, wherein
   R^{AM} is a biotinyl moiety, dextrin moiety, a click moiety, a thiol moiety, norbornenyl moiety, furanyl moiety, alkyl ester moiety, or maleimidyl moiety;
   Z is CH₂, O, S, or NH; and
   n is an integer from 0 to 50.
61. The method of any one of aspects 57-60, wherein n is 6.
62. A method of analyzing a biological sample comprising a fragmented ribonucleic acid (RNA), the method comprising:
   (a) contacting the biological sample comprising the fragmented RNA with T4 polynucleotide kinase, wherein the T4 polynucleotide kinase catalyzes formation of a 2',3'-vicinal diol moiety on the fragmented RNA;
   (b) contacting the biological sample with a deglycosylation agent;
   (c) contacting the biological sample with a matrix-forming agent, wherein the matrix-forming agent comprises a boronic acid moiety capable of covalently reacting with at least one 2',3' vicinal diol of the RNA, and wherein the biological sample and the matrix-forming agent are contacted under conditions suitable to form a covalent bond between the boronic acid moiety and the 2',3;-vicinal diol of the RNA;
   (d) forming a three-dimensional polymerized matrix from the matrix-forming agent, thereby embedding the biological sample in the three-dimensional polymerized matrix and anchoring the fragmented RNA to the three-dimensional polymerized matrix;
   (e) clearing the biological sample;
   (f) contacting the biological sample with a probe or probe set that binds directly or indirectly to the RNA; and
   (g) detecting the probe or a product thereof at a location in the matrix.
63. The method of any one of aspects 42-62 wherein the fragmented RNA in step (a) has a 2',3'cyclo-phosphate fragmentation at the 3'-terminal end or a 2' hydroxyl and a 3' phosphate fragmentation at the 3'-terminal end, and wherein the 3' phosphatase catalyzes the formation of the 2',3'-vicinal diol.
64. The method of any one of aspects 42-63, wherein the 3' phosphatase is T4 polynucleotide kinase.
65. The method of any one of aspects 42- 64, wherein the fragmented RNA of step (a) is generated by treating the biological sample with a degradation agent to induce fragmentation of RNAs, optionally wherein the degradation agent is an RNase or restriction enzyme.
66. The method of any one of aspects 42-65, wherein the fragmented RNA further comprises an additional vicinal diol moiety provided by a 5' cap, optionally wherein the 5' cap is a 7-methylguanosine cap.
67. The method of any one of aspects 42-66, wherein the biological sample is cleared with a detergent, a lipase, and/or a protease.
68. The method of aspect 67, wherein the detergent comprises a non-ionic surfactant or anionic surfactant, optionally wherein the detergent comprises SDS, tergitol, NP-40, saponin, polyethylene glycolp-(1,1,3,3-tetramethylbutyl)-phenyl ether, or polysorbate 20, or any combinations thereof.
69. The method of aspect 67, wherein the protease comprises proteinase K, pepsin, or collagenase, trypsin, dispase, thermolysin, or alpha-chymotrypsin, or any combinations thereof, optionally wherein the protease comprises Liberase^{™}.
70. The method of aspect 67, wherein the lipase comprises a pancreatic, hepatic and/or lysosomal lipase, or any combinations thereof, optionally wherein the lipase comprises sphingomyelinase or esterase, or a combination thereof.
71. The method of any one of aspects 42-70, wherein the probe or probe set is a circular or circularizable probe or probe set capable of binding the fragmented RNA.
72. The method of aspect 71, further comprising:
   generating a rolling circle amplification (RCA) product of the circular or circularizable probe or probe set; and
   detecting a signal associated with a fluorescently labeled probe that directly or indirectly binds to the rolling circle amplification product.
73. A method of analyzing a fragmented ribonucleic acid (RNA) in a biological sample, the method comprising:
   (a) contacting the biological sample comprising the fragmented RNA with a 3' phosphatase to provide a fragmented RNA comprising a 2',3'-vicinal diol;
   (b) contacting the biological sample with an attachment agent comprising a boronic acid moiety capable of covalently reacting with at least one 2',3' vicinal diol of the RNA and an attachment moiety capable of attaching covalently or noncovalently to a matrix-forming agent in the biological sample, wherein the biological sample and the attachment agent are contacted under conditions suitable to form a covalent bond between the boronic acid moiety and the 2',3;-vicinal diol of the RNA;
   (c) contacting the biological sample with a matrix-forming agent;
   (d) forming a three-dimensional polymerized matrix from the matrix-forming agent, thereby embedding the biological sample in the three-dimensional polymerized matrix and anchoring the fragmented RNA to the three-dimensional polymerized matrix;
   (e) clearing the biological sample;
   (f) contacting the biological sample with a circular or circularizable probe or probe set, wherein the circular or circularizable probe binds the RNA;
   (g) generating a rolling circle amplification (RCA) product of the circular or circularizable probe or probe set; and
   (h) detecting a signal associated with a fluorescently labeled probe that directly or indirectly binds to the rolling circle amplification product.
74. The method of aspect 73, wherein the attachment moiety is capable of attaching covalently to the matrix-forming agent in the biological sample.
75. The method of aspect 74, wherein the attachment moiety is or comprises an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety.
76. The method of aspect 74, wherein the attachment moiety is or comprises a click functional group.
77. The method of any one of aspects 74-76, wherein the step of contacting the biological sample and attachment agent further comprises contacting the biological sample with one or more reagents or under suitable conditions to facilitate the formation of a covalent bond between the attachment moiety of the attachment agent and an exogenous or endogenous molecule in the biological sample.
78. The method of aspect 73, wherein the attachment moiety is capable of attaching non-covalently to the matrix-forming agent in the biological sample.
79. The method of aspect 78, wherein the attachment moiety is biotin.
80. The method of any one of aspects 73-79, wherein the attachment moiety is a nonaromatic compound.
81. The method of any one of aspects 73-80, wherein the attachment agent is a compound of formula (I) 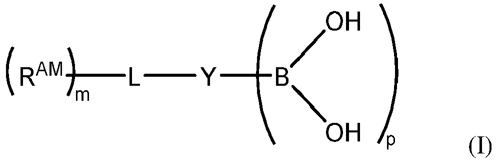 or a salt thereof, wherein
   - each R^{AM}: is independently the attachment moiety;
   - L: is a bond or linker moiety;
   - Y: is a bond, -CH₂CH₂- or -O-;
   - m: is an integer from 1 to 4; and
   - p: is an integer from 1 to 4.
82. The method of any one of aspects 73-81, wherein the attachment agent is multifunctional and comprises at least two boronic moieties or at least two attachment moieties.
83. The method of any one of aspects 73-82, wherein the attachment agent comprises at least two boronic acid moieties.
84. The method of any one of aspects 73-83, wherein the attachment agent comprises at least two attachment moieties.
85. The method of any one of aspects 73-81, wherein the attachment agent is bifunctional.
86. The method of any one of aspects 73-81 and 85, wherein the attachment agent is a compound of formula (I-a) 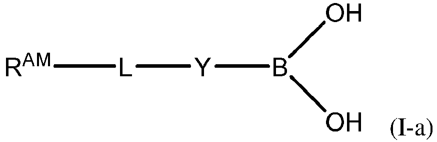 or a salt thereof, wherein
   - R^{AM}: is the attachment moiety;
   - L: is a bond or linker moiety; and
   - Y: is a bond, -CH₂CH₂- or -O-.
87. The method of any one of aspects 81-86, wherein L is an unbranched or branched C₁-C₁₅₀ alkylene optionally interrupted by 1 to 50 heteroatoms independently selected from the group consisting of O, S and NH.
88. The method of any one of aspects 81-87, wherein L is the group 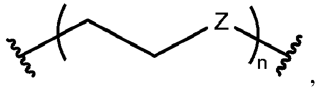 wherein
   Z is CH₂, O, S, or NH; and
   n is an integer from 0 to 50.
89. The method of any one of aspects 73 and 81-88, wherein each R^{AM} is independently acrylate moiety, methacrylate moiety, acrylamide moiety, methacrylamide moiety, biotinyl moiety, dextrin moiety, a click moiety, a thiol moiety, norbornenyl moiety, furanyl moiety, alkyl ester moiety, or maleimidyl moiety.
90. The method of aspect 73 and 81-89, wherein the attachment agent is a compound of formula (II-a) 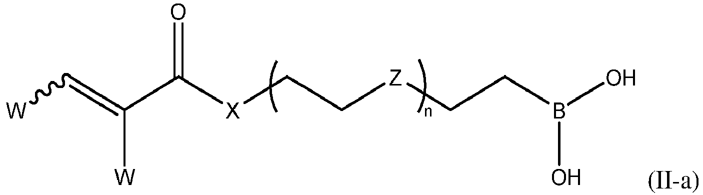 or a salt thereof, wherein
   each W is independently H or CH₃;
   X is NH or O;
   Z is CH₂, O, S, or NH; and
   n is an integer from 0 to 50.
91. The method of aspect 73 and 81-89, wherein the attachment agent is a compound of formula (I) 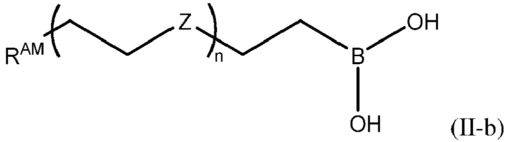 or a salt thereof, wherein
   R^{AM} is a biotinyl moiety, dextrin moiety, a click moiety, a thiol moiety, norbornenyl moiety, furanyl moiety, alkyl ester moiety, or maleimidyl moiety;
   Z is CH₂, O, S, or NH; and
   n is an integer from 0 to 50.
92. The method of any one of aspects 88-91, wherein n is 6.
93. A method of analyzing a biological sample comprising a fragmented ribonucleic acid (RNA), the method comprising:
   (a) contacting the biological sample comprising the fragmented RNA with T4 polynucleotide kinase, wherein the T4 polynucleotide kinase catalyzes formation of a 2',3'-vicinal diol moiety on the fragmented RNA;
   (b) contacting the biological sample with a deglycosylation agent;
   (c) contacting the biological sample with a matrix-forming agent, wherein the matrix-forming agent comprises a boronic acid moiety capable of covalently reacting with at least one 2',3' vicinal diol of the RNA;
   (d) forming a three-dimensional polymerized matrix from the matrix-forming agent, thereby embedding the biological sample in the three-dimensional polymerized matrix and anchoring the fragmented RNA to the three-dimensional polymerized matrix;
   (e) clearing the biological sample;
   (f) contacting the biological sample with a circular or circularizable probe or probe set, wherein the circular or circularizable probe binds the RNA;
   (g) generating a rolling circle amplification (RCA) product of the circular or circularizable probe or probe set; and
   (h) detecting a signal associated with a fluorescently labeled probe that directly or indirectly binds to the rolling circle amplification product.
94. The method of any one of aspects 73-93, wherein the biological sample is cleared with a detergent, a lipase, and/or a protease.
95. The method of aspect 94, wherein the detergent comprises a non-ionic surfactant or anionic surfactant, optionally wherein the detergent comprises SDS, tergitol, NP-40, saponin, polyethylene glycolp-(1,1,3,3-tetramethylbutyl)-phenyl ether, or polysorbate 20, or any combinations thereof.
96. The method of aspect 94, wherein the protease comprises proteinase K, pepsin, or collagenase, trypsin, dispase, thermolysin, or alpha-chymotrypsin, or any combinations thereof, optionally wherein the protease comprises Liberase^{™}.
97. The method of aspect 94, wherein the lipase comprises a pancreatic, hepatic and/or lysosomal lipase, or any combinations thereof, optionally wherein the lipase comprises sphingomyelinase or esterase, or a combination thereof.
98. The method of any one of aspects 1 to 97, wherein the biological sample comprises cells or cellular components.
99. The method of any one of aspects 1 to 98, wherein the biological sample is a tissue sample.
100. The method of aspect 99, wherein the tissue sample is a tissue slice between about 1 µm and about 50 µm in thickness.
101. The method of any one of aspects 1 to 100, wherein the biological sample is fixed.
102. The method of aspect 101, wherein the biological sample is a formalin-fixed, paraffin-embedded (FFPE) sample, a fresh tissue sample, or a frozen tissue sample.
103. The method of any one of aspects 1 to 102, further comprising staining, permeabilizing, cross-linking, expanding, and/or de-cross-linking the biological sample embedded in the three-dimensional polymerized matrix.
104. The method of any one of aspects 1 to 103, wherein the biological sample and fragmented RNA are treated with a ribonuclease inhibitor.
105. The method of any one of aspects 1 to 104, wherein the step of contacting the biological sample with the 3' phosphatase is not performed in the presence of ammonium ions, phosphate ions, or metal chelators.
106. The method of any one of aspects 1 to 105, wherein the step of contacting the biological sample with the 3' phosphatase is not performed in the presence of sodium chloride or potassium chloride buffer having a concentration of greater than 50 mM.
107. The method of any one of aspects 1 to 106, wherein the matrix-forming agent comprises polyacrylamide, cellulose, alginate, polyamide, cross-linked agarose, cross-linked dextran or cross-linked polyethylene glycol.
108. The method of any one of aspects 1 to 107, wherein the three-dimensional polymerized matrix is formed by subjecting the matrix-forming agent to polymerization.
109. The method of aspect 108, wherein the polymerization is initiated by adding a polymerization-inducing catalyst, UV light or functional cross-linkers.
110. The method of any one of aspects 1 to 109, wherein the fragmented RNA is fragmented mRNA.
111. The method of any one of aspects 1-110, comprising treating the biological sample with a detergent and a protease after forming the three-dimensional polymerized matrix
112. The method of aspect 111, wherein the detergent comprises SDS and the protease comprises proteinase K.
113. The method of aspect 111 or 112, wherein the detergent and the protease are provided in a buffer of at least pH 8.0.
114. The method of any one of aspects 111-113, the biological sample is treated with the detergent and the protease at least 45°C for no more than 4 minutes.
115. The method of any one of aspects 111-114, the biological sample is treated with the detergent and the protease at about 50°C for about 3 minutes.
116. The method of any one of aspects 111-115, the biological sample is treated with 1% SDS and 200 µg/mL proteinase K provided in a PBS buffer of at least pH 8.5 at about 50°C for about 3 minutes.
117. A kit for analyzing fragmented ribonucleic acid (RNA) in a biological sample, comprising:
   (a) an attachment agent comprising a boronic acid moiety capable of covalently reacting with at least one 2',3' vicinal diol of the RNA and an attachment moiety capable of attaching covalently or noncovalently to an exogenous or endogenous molecule in the biological sample;
   (b) a matrix-forming agent;
   (c) a polymerization-inducing catalyst or functional cross linker; and
   (d) instructions for use.
118. A kit for analyzing fragmented ribonucleic acid (RNA) in a biological sample, comprising:
   (a) a matrix-forming agent, wherein the matrix forming agent comprises a boronic acid moiety capable of covalently reacting with at least one 2',3' vicinal diol of the RNA; and
   (b) a polymerization-inducing catalyst or functional cross linker; and
   (c) instructions for use.
119. The kit of aspect 117 or 118, wherein the kit further comprises a deglycosylation agent.
120. The kit of any one of aspects 117-119, wherein the kit further comprises a 3' phosphatase.
121. The kit of aspect 120, wherein the 3' phosphatase is T4 polynucleotide kinase.
122. The kit of any one of aspects 117-121, wherein the kit further comprises a clearing agent.
123. The kit of any one of aspects 117-122, wherein the clearing agent is a detergent, a lipase, a protease, and/or a deglycosylase.
124. The method of any one of aspects 21-41, wherein (b) comprises contacting the biological sample with a radical generating compound to initiate formation of the three-dimensional polymerized matrix.

## Claims

1. A method, comprising:
(a) contacting a biological sample comprising a ribonucleic acid (RNA) with a matrix-forming agent, wherein:
(i) the RNA comprises a 2'3' vicinal diol, and
(ii) the matrix-forming agent comprises a boronic acid moiety capable of covalently reacting with at least one 2',3' vicinal diol of the RNA, and
wherein the biological sample and the matrix-forming agent are contacted under conditions suitable to form a covalent bond between the boronic acid moiety and the 2',3;-vicinal diol of the RNA; and
(b) forming a three-dimensional polymerized matrix from the matrix-forming agent, thereby embedding the biological sample and immobilizing the RNA in the three-dimensional polymerized matrix.

2. The method of claim 1, wherein the RNA is a fragmented RNA.

3. The method of claim 1 or claim 2, wherein the 2',3'-vicinal diol is a fragmented 3' end of the RNA.

4. The method of claim 3, wherein the 2',3'-vicinal diol is provided by contacting the fragmented RNA with a 3' phosphatase, wherein the 3' phosphatase is T4 polynucleotide kinase.

5. The method of any one of claims 1 to 4, wherein the biological sample is treated with a degradation agent to induce fragmentation of RNAs, optionally wherein the degradation agent is an RNase or restriction enzyme.

6. The method of any one of claims 1 to 5, further comprising clearing the biological sample embedded in the three-dimensional polymerized matrix.

7. The method of claim 6, further comprising contacting the biological sample with a probe or probe set that binds directly or indirectly to the RNA, optionally wherein the probe or probe set is a detectable probe.

8. The method of claim 7, wherein the probe or probe set is a circular or circularizable probe or probe set, optionally wherein the method comprises circularizing the circularizable probe or probe set using the RNA or a product thereof as a template, optionally wherein the method comprises generating an RCA product using the circular or circularizable probe as a template.

9. The method of claim 8, wherein the probe or probe set comprises a barcode sequence.

10. The method of claim 9, wherein the method comprises detecting the barcode sequence or a complement thereof in the probe or probe set or in a product of the probe or probe set.

11. The method of any one of claims 1 to 10, wherein the matrix-forming agent is prepared by contacting a precursor matrix-forming agent with an attachment agent comprising a boronic acid moiety and an attachment moiety capable of attaching covalently to the precursor matrix-forming agent, wherein the precursor matrix forming agent does not comprise a boronic acid moiety.

12. The method of claim 11, wherein the attachment moiety is or comprises an alkenyl, allyl or vinyl moiety, an amide moiety, an alcohol moiety, a polyol moiety, a furan moiety, a maleimide moiety, a norbornene moiety, a thiol moiety, a phenol moiety, a urethane moiety, a cyano moiety, an isocyanate moiety, an isothiocyanate moiety, an ether moiety, a dextran moiety, or an alginate moiety.

13. The method of claim 11 or 12, wherein the attachment agent is a compound of formula (I) 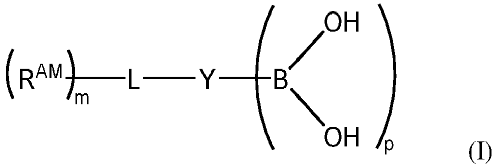 or a salt thereof, wherein
each R^{AM} is independently the attachment moiety;
L is a bond or linker moiety;
Y is a bond, -CH₂CH₂- or -O-;
m is an integer from 1 to 4; and
p is an integer from 1 to 4.

14. The method of any one of claims 11 to 13, wherein the attachment agent is multifunctional and comprises at least two boronic moieties or at least two attachment moieties.

15. The method of any one of claims 11 to 13, wherein the attachment agent is a compound of formula (I-a) 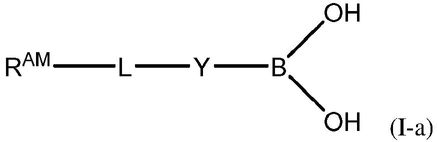 or a salt thereof, wherein
R^{AM} is the attachment moiety;
L is a bond or linker moiety, wherein L is an unbranched or branched C₁-C₁₅₀ alkylene optionally interrupted by 1 to 50 heteroatoms independently selected from the group consisting of O, S and NH; and
Y is a bond, -CH₂CH₂- or -O-.
